(19) **Europäisches Patentamt / European Patent Office / Office européen des brevets**

(11) **EP 1 815 245 B1**

(12) **EUROPEAN PATENT SPECIFICATION**

(45) Date of publication and mention
of the grant of the patent:
**21.04.2010 Bulletin 2010/16**

(51) Int Cl.:
*G01N 33/50* (2006.01)

(21) Application number: **05809080.4**

(22) Date of filing: **15.11.2005**

(86) International application number:
**PCT/NL2005/050043**

(87) International publication number:
**WO 2006/052136 (18.05.2006 Gazette 2006/20)**

(54) **PREMATURELY AGEING MOUSE MODELS FOR THE ROLE OF DNA DAMAGE IN AGEING AND INTERVENTION IN AGEING-RELATED PATHOLOGY**

VORZEITIG ALTERNDE MÄUSE ZUR DEMONSTRATION DER ROLLE DER DNA-SCHÄDIGUNG BEI DER ALTERUNG UND EINGRIFF IN DIE ALTERSBEDINGTE PATHOLOGIE

MODELES DE SOURIS VIEILLISSANT DE MANIERE PREMATUREE DESTINES A LA DEFINITION DU ROLE DES DOMMAGES DE L'ADN DANS LE VIEILLISSEMENT ET INTERVENTION DANS UNE PATHOLOGIE RELATIVE AU VIEILLISSEMENT

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.11.2004 EP 04078128**

(43) Date of publication of application:
**08.08.2007 Bulletin 2007/32**

(60) Divisional application:
**10154025.0**
**10154034.2**

(73) Proprietor: **Erasmus MC**
**3015 GD Rotterdam (NL)**

(72) Inventors:
• **HOEIJMAKERS, Jan, Hendrik, Jozef**
**NL-2761 XW Zevenhuizen (NL)**
• **VAN DER HORST, Gijsbertus, Theodorus, Johannes**
**NL-3161 LK Rhoon (NL)**
• **VERMEULEN, Wim**
**NL-3333 VL Zwijndrecht (NL)**
• **KANAAR, Roland**
**NL-3062 GA Rotterdam (NL)**
• **VAN DER PLUIJM, Ingrid**
**NL-3356 EK Papendrecht (NL)**
• **GARINIS, George, Aris**
**NL-3054 NL Rotterdam (NL)**
• **VAN STEEG, Harmen**
**NL-2161 PG Blaricum (NL)**

• **MITCHELL, James, Robert**
**NL-3054 NM Rotterdam (NL)**
• **JASPERS, Nicolaas, Gerardus, Joseph**
**NL-3023 EJ Rotterdam (NL)**
• **NIEDERNHOFER, Laura**
**Pittsburgh, PA 15232 (US)**
• **DE BOER, Jan**
**NL-3708 CR Zeist (NL)**
• **ANDRESSOO, Jaan Olle**
**EE12612 Tallinn (EE)**

(74) Representative: **Schut, Frederik**
**Vereenigde**
**Johan de Wittlaan 7**
**2517 JR The Hague (NL)**

(56) References cited:
**US-A1- 2003 167 556     US-A1- 2004 067 228**

• **DE WAARD HARM ET AL: "Different effects of CSA and CSB deficiency on sensitivity to oxidative DNA damage" MOLECULAR AND CELLULAR BIOLOGY, vol. 24, no. 18, September 2004 (2004-09), pages 7941-7948, XP009044218 ISSN: 0270-7306 cited in the application**
• **DE BOER JAN ET AL: "Premature aging in mice deficient in DNA repair and transcription" SCIENCE (WASHINGTON D C), vol. 296, no. 5571, 17 May 2002 (2002-05-17), pages 1276-1279, XP002318350 ISSN: 0036-8075 cited in the application**

• HASTY PAUL ET AL: "Aging and genome maintenance: Lessons from the mouse?" SCIENCE (WASHINGTON D C), vol. 299, no. 5611, 28 February 2003 (2003-02-28), pages 1355-1359, XP002318351 ISSN: 0036-8075 cited in the application

• HASTY PAUL ET AL: "Accelerating aging by mouse reverse genetics: a rational approach to understanding longevity." AGING CELL. APR 2004, vol. 3, no. 2, April 2004 (2004-04), pages 55-65, XP002318352 ISSN: 1474-9718 cited in the application

**Description**

Field of the invention

The present invention relates to the field of ageing, in particular the relation between ageing and genome maintenance (GM); induction and response to DNA damage. More specifically the invention relates to ageing and DNA damage repair/response systems, having major effects on cell survival and cellular resistance to genotoxins.

The invention pertains to a method for screening and discovery of compounds capable of inhibiting, preventing, delaying or reducing genome maintenance disorders and consequences thereof. In particular it provides a method for screening for compounds that inhibit, reduce or prevent ageing-related symptoms and conditions in mammals, such as those caused by genome maintenance disorders or those caused by normal, natural ageing processes during the normal life span of a mammal. The invention provides strategies of intervention for GM disorders and provides methods for screening, aimed at the discovery of new treatments for ageing-related symptoms. These ageing-related symptoms to be treated with these compounds may be ageing-related symptoms brought about by genetic defects and disorders, in particular genetic defects in NER/TCR/XLR/DSBR, but may also be ageing-related symptoms and diseases observed in normal ageing. In particular the invention provides a method for the development and use of mouse models deficient in genome maintenance and displaying premature ageing phenotypes, which are particularly suited for testing of compounds, substances and compositions that will prevent, inhibit, reduce or delay an ageing-related parameter or several ageing-related parameters and/or phenotypes in mammals.

Background of the invention

Ageing can be defined as the progressive deterioration of cells, tissues, organs and a mammalian body, associated with increased age of an organism. Evolutionary theories of ageing are based on the observation that the efficacy of natural selection decreases with age. This is because, even without ageing, individuals will die of environmental causes, such as predation, disease and accidents. The process of ageing would function to weed out worn out and older individuals in order to prevent them from competing with their progeny for resources. Ageing is thereby thought to have evolved as the result of optimising fitness early in life.

Progressive accumulation of damage with effects later in life is widely believed to be a prime cause of ageing-related symptoms, although also many other theories have been put forward, such as hormonal-induction of ageing. The fitness of an ageing organism and the longevity of a species seems at least partially determined by the balance of intrinsically and environmentally caused damage to cellular biomolecules on one side and the activity of maintenance and stress resistance systems on the other. The nature of which biomolecules are the main target(s): lipids, membranes, organelles (such as the mitochondrion), proteins, RNA or DNA or a combination is still a matter of debate.

There are 4 major model systems for studying the genetics of ageing; the budding yeast Saccharomyces cerevisiae, the nematode Caenorhabditis elegans, the fruitfly Drosophila melanogaster, and most importantly the mouse Mus musculus as a mammalian model. These models have been widely used to test theories about the mechanisms of ageing,

Testing of common gene variants or environmental factors, such as for instance food intake, for their influence on human mortality and disease, have contributed to the understanding of ageing at the cellular level. The search for genetic pathways and development of animal models, that influence ageing and ageing-related diseases or phenotypes, and that allow the ageing process to be studied in detail, is progressing rapidly due to the latest developments in genetics and genomics.

Research into rare inherited human diseases, such as segmental progeroid syndromes that display some features of premature and/or accelerated ageing, have led to the discovery of some of the underlying genetic mechanisms of (accelerated segmental) ageing. This has allowed the development of specific animal models, such as genetically modified mice, to study ageing-related phenomena. More in particular, this has led to the development of animal models, such as genetically modified mouse models deficient in genome maintenance systems, that display accelerated or enhanced segmental ageing phenotypes (Boer J, et al., Science. 2002 May 17;296(5571):1276-9, de Waard H, et al., Mol Cell Biol. 2004 Sep; 24(18):7941-8., reviewed in Hasty P, Campisi J, Hoeijmakers J, van Steeg H, Vijg J., Science. 2003 Feb 28;299(5611):1355-9.)

Animal models deficient in genome maintenance and displaying accelerated and/or enhanced ageing or segmental ageing phenotypes, and the use of such animal models to study ageing have met with wide scepticism from the scientific community. There is an ongoing debate (Hasty P, Vijg J., Ageing Cell 2004 vol 3, pp55-65 and Hasty P., Vijg J., Ageing Cell 2004 vol 3 pp. 67-69) whether or not, and to what extent, animal models exhibiting features of accelerated ageing provide a useful model for the process of normal ageing. Many scientists and experts in the field claim that such animal models merely display the effects of a specific genetic alteration, in particular mutations affecting genome maintenance systems. In their view, most of these phenotypic effects merely resemble symptoms of natural ageing at best and developmental impairment at the worst and bear little relevance to normal ageing (Miller RA., Ageing Cell 2004 vol 3, pp 47-51, Miller R.A. Ageing Cell 2004 vol 3, pp 52-53, Miller RA. Science. 2005 Oct; 310(5747):441-3).

Although many potential uses of these animal models have been discussed in the literature mentioned above, the great difficulty concerning the validity of these animal models remains a widely recognized problem in the art, the art being the field of ageing and ageing research.

The current invention provides a method for screening and discovery of compounds that are capable of inhibiting, preventing, delaying or reducing genome maintenance disorders and consequences thereof. The invention exploits animal models that comprise deficiencies in their genome maintenance systems and display premature, enhanced, accelerated or segmental ageing phenotypes. The current invention shows for the first time that the use of these animal models exhibiting features of dramatically accelerated, premature and/or enhanced ageing phenotypes is in fact valid and can be advantageously applied to screen compounds and thereby develop schemes of intervention to treat, delay, inhibit, prevent or cure ageing-related symptoms. The provided examples herein illustrate the method and provide clear evidence that such compounds can be positively identified using the method of screening according to the invention. The current invention thus provides a new and powerful tool to screen and discover compounds capable of counteracting ageing related symptoms comprising prophylactic and/or therapeutically active compounds.

The method of screening compounds according to the current invention has several advantages over similar methods of screening known in the art, which comprise the use of animals that are not genetically altered and do not display a phenotype of enhanced, accelerated and/or premature ageing.

Firstly, the current invention provides methods of screening which are more efficient, as much less time is required before the animal displays ageing symptoms or characteristics which can be influenced by the compounds to be screened. Some animals models display even ageing-related symptoms in utero, as illustrated in the examples of the current invention, whereas normal mice exhibit such ageing-related symptoms only after one and a half, two or even more years.

Secondly, the method according to the current invention allows compounds to be screened for having an effect on specific phenotypes that are much more pronounced in a genetically modified animal as compared to wild type animals, where only a small fraction of animals will display an ageing-related symptom, and only after two years or more. Particularly, the method can be used to screen the influence of specific compounds on the specific phenotype at the level of individual organs and tissues. Hence the method according to the current invention can be advantageously applied to screen compounds and develop strategies of interventions for particular ageing-related symptoms or diseases.

Detailed description of the invention

A. General definitions

"Gene" or "coding sequence" refers to a DNA or RNA region (the transcribed region) which "encodes" a particular protein. A coding sequence is transcribed (DNA) and translated (RNA) into a polypeptide when placed under the control of an appropriate regulatory region, such as a promoter. A gene may be a genomic sequence comprising non-coding introns and coding exons, or may be a complementary DNA (cDNA) sequence. A gene may comprise several operably linked fragments, such as a promoter, transcription regulatory sequences, a 5' leader sequence, a coding sequence and a 3'nontranslated sequence, comprising a polyadenylation site. A chimeric or recombinant gene is a gene not normally found in nature, such as a gene in which for example the promoter is not associated in nature with part or all of the transcribed DNA region. "Expression of a gene" refers to the process wherein a gene is transcribed into an RNA and/or translated into an active protein.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated. A "tissue specific" promoter is only active in specific types of tissues or cells.

As used herein, the term "operably linked" refers to two or more nucleic acid or amino acid sequence elements that are physically linked in such a way that they are in a functional relationship with each other. For instance, a promoter is operably linked to a coding sequence if the promoter is able to initiate or otherwise control/regulate the transcription and/or expression of a coding sequence, in which case the coding sequence should be understood as being "under the control of" the promoter. Generally, when two nucleic acid sequences are operably linked, they will be in the same orientation and usually also in the same reading frame. They will usually also be essentially contiguous, although this may not be required.

"Gene delivery" or "gene transfer" refers to methods for reliable introduction of recombinant or foreign DNA into host cells. The transferred DNA can remain non-integrated or preferably integrates into the genome of the host cell. Gene delivery can take place for example by transduction, using viral vectors, or by transformation of cells, using known methods, such as electroporation, cell bombardment and the like. In addition, genes can be directly (and in a tissue-specific manner) delivered to the living mouse, for example by viral vectors or by the use of liposomal vehicles (Current protocols in molecular biology, Ausubel et al. Wiley Interscience, 2004).

"Vector" refers generally to nucleic acid constructs suitable for cloning and expression of nucleotide sequences. The term vector may also sometimes refer to transport vehicles comprising the vector, such as viruses, virions or liposomes, which are able to transfer the vector into and between host cells.

A "transgene" is herein defined as a gene that has been newly introduced into a cell, i.e. reintroduction of an endogenous gene, a mutated gene, an inactivated gene or a gene that does not normally occur in the cell. The transgene may comprise sequences that are native to the cell, sequences that in nature do not occur in the cell and it may comprise combinations of both. A transgene may contain sequences coding for one or more proteins that may be operably linked to appropriate regulatory sequences for expression of the coding sequences in the cell. Preferably, the transgene is integrated into the host cell's genome, either in a random fashion or integrated in a specific locus by homologous recombination. Delivery can occur *in vitro* (oocyte/ES cells) or *in vivo* (living mouse) via methods known in the art.

"Subjects" means any member of the class mammalia, including without limitation humans, non-human primates, farm animals, domestic animals and laboratory animals.

The term "substantial identity" means that two peptide or two nucleotide sequences, when optimally aligned, such as by the programs GAP or BESTFIT using default parameters, share at least 80 percent sequence identity, preferably at least 90 percent sequence identity, more preferably at least 95 percent sequence identity or more (e.g., 99 percent sequence identity). GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992).

The term "comprising" is to be interpreted as specifying the presence of the stated parts, steps or components, but does not exclude the presence of one or more additional parts, steps or components. A nucleic acid sequence comprising region X, may thus comprise additional regions, i.e. region X may be embedded in a larger nucleic acid region.

The term 'substance' comprises compounds and compositions comprising two or more compounds.

B. Detailed description of the invention.

Genome maintenance systems encompass nucleotide excision repair (NER; including global genome NER (GG-NER) and transcription-coupled NER (TC-NER)), transcription-coupled repair (TCR), differentiation associated repair (DAR), base excision repair (BER), as well as double strand break repair (DSBR) and DNA crosslink repair (XLR) pathways and associated DNA damage tolerance and signalling (DT&S) systems and proteins involved therein. For brevity this area will be designated here as GM (genome maintenance).

The invention provides a new use of genetically modified animal models (as well as tissues, cultured cells and cell-free systems derived thereof) for assessing ageing-related phenotypes. The animal models comprise mutations in the above specified GM systems. Several of these 'GM animals' closely mimic human GM syndromes and the animals exhibit a multitude of symptoms resulting from defective DNA maintenance and involving multiple signs of premature ageing including osteoporosis, kyphosis, cachexia, early onset of infertility, accelerated neuro- hemato- and muscular degeneration, liver and kidney failure, thymic involution, age-related hormonal changes, as indicated below and demonstrated in the examples in this specification. The parallels with normal aging are also apparent from the striking resemblance of genome wide expression profiles of various progeroid mouse mutants and normally aged animals (see example 9). The invention provides a method for the selection of compounds or mixtures capable of inhibiting, delaying, preventing or curing premature ageing phenotypes in mammals. At the same time the method allows -besides the characterization of the ageing process itself- also identification of compounds or mixtures (such as drugs or known/ unknown chemical agents) that enhance the ageing process. In an additional aspect of the invention the use of these GM animals (and the cells derived thereof) is aimed the identification of compounds that improve the condition of organs and tissues for transplantation purposes in order to prevent or reduce oxygen reperfusion damage. In another aspect of the invention the method is applied for the optimization of use of chemotherapeutic agents that induce DNA damage and in this manner enhance ageing. In another aspect the method of the invention encompasses the use of the GM animal models (and cells thereof) for testing cosmetic compounds and treatments in the context of ageing. In yet another aspect of the invention the method comprises the use of the GM animal models with accelerated ageing for stem cell transplantation for use of organ renewal. In a final aspect of the invention, the methods and GM animal models described here are used for the derivation of ageing-related signatures at the level of gene- or protein expression, for instance on micro-arrays, and/or at the level of SNP's, and /or at the level of metabolites (metabolomics), which are indicative for the ageing-status of the specific organ or tissue and the effect of (mixtures of) compounds and applications on the ageing status. Some of the above meant compounds/applications will provide novel treatments and therapies for ageing-related conditions, more in particular premature ageing-related conditions caused by defective GM systems, as well as natural ageing symptoms in animals and in humans. The invention particularly encompasses the use of the ability of offspring of specific (combinations of) GM mutants to overcome birth stress, their pre- and early postnatal development (weight/ size/behaviour), onset of osteoporosis, kyphosis and lifespan beyond a period of~3 weeks as rapid reliable read-out for ageing in general, including osteoporosis, ageing of the neuronal, muscular and hematopoietic systems, liver-, kidney and other organ dysfunction, age-related hormonal changes, cachexia, onset of infertility. Thus this rapid, valid model allows efficient, reliable and rapid screening of compounds/treatments that influence specific and general ageing, ageing-related pathology, chemotherapy and organ/tissue and stem cell transplantation.

DNA is continuously exposed to a myriad of environmental and endogenously produced damaging agents, including (but not limited to) oxidative metabolites, ionizing and ultraviolet (UV) radiation and numerous natural or man-made chemical toxins. The resulting DNA damage may compromise essential cellular processes such as transcription and replication, or can cause mutations that can trigger carcinogenesis or (in the case of germ cells) inborn disorders. In addition, DNA damage can cause transient or permanent cell cycle arrest, cellular (replicative) senescence or cell death (either directly or by triggering apoptosis) and thereby contribute to ageing.

To prevent these deleterious consequences, all organisms are equipped with a sophisticated network of complementary and partly overlapping DNA repair mechanisms each dealing with a specific class of DNA lesions. This network of highly interwoven genome maintenance systems is essential to maintain their genomes intact. For a review on the DNA damage repair and response systems described below see Hoeijmakers, Genome maintenance mechanisms for preventing cancer, Nature 2001, May 17;411(6835):366-74.

Base excision repair (BER) removes more subtle types of damage such as a number of oxidative lesions in DNA. A number of DNA glycosylases, each with a more narrow spectrum of lesions that are recognized initiate a multi-step incision, lesion excision reaction involving short or long patch repair synthesis.

DNA damage can also comprise single or even double strand breaks (e.g. induced by X- or $\gamma$ rays or ionising radiation), which are repaired by homologous recombination or by non-homologous endjoining, the two main types of Double Strand Break Repair (DSBR). Another repair system that is closely related to homologous recombination but is poorly understood eliminates the very toxic interstrand crosslinks induced naturally as byproduct of lipid peroxidation (malondialdehyde) or intentionally by chemotherapeutic agents such as cis-Platin. This process is called crosslink repair, here referred to as XLR.

A well known and well studied genome maintenance system is the highly conserved nucleotide excision repair (NER) pathway that requires the concerted action of at least 25 proteins to recognize and eliminate the damage in a complex "cut and patch" reaction. NER is one of the most versatile DNA repair pathways since it removes a wide variety of helix-distorting DNA lesions, hereafter referred to as "classical NER lesions", including major UV- induced injuries and bulky chemical adducts, in addition to some forms of oxidative damage. NER consists of two subpathways, global genome NER (GG-NER) and transcription-coupled NER (TC-NER); the latter functions specifically to remove damage from the transcribed strand of active genes and in this manner permit recovery of RNA synthesis and cellular survival. The TC-NER reaction is triggered upon stalling of an elongating RNA polymerase at a DNA lesion, with recruitment of the core NER machinery following removal or displacement of the blocked polymerase. Moreover, evidence exists that a number of lesions that are typical substrates of the base excision repair (BER) pathway, and that cause a block of the transcription machinery, are also repaired in a transcription-coupled manner. We will refer to TC-NER for transcription-coupled repair of NER-type of lesions and to TCR when transcription-coupled repair of all kinds of transcription-blocking DNA damage is meant. Evidence is increasing that non-replicating cells (i.e. terminally differentiated cells such as neurons) attenuate global genome repair, but maintain a mechanism to keep the non-transcribed strand (NTS) of active genes, which serves as the template for TCR), free of lesions via a mechanism designated differentiation-associated repair (DAR) (Nouspikel T, Hanawalt PC (2002) DNA repair in terminally differentiated cells. DNA Repair Jan 22;1(1): 59-75).

It is important to stress that a large number of the GG-NER and TC-NER components such as TFIIH (composed of 10 protein subunits), XPG, CSB and CSA are at the same time key factors for TCR. Additionally, the multi-subunit TFIIH complex is an essential player in transcription initiation of all structural genes transcribed by RNA polymerase II as well as the rRNA genes, transcribed by RNA polymerase I. Moreover, at least one of the GG-NER and TC-NER protein complexes, ERCC1/XPF is simultaneously implicated in the repair of the very cytotoxic interstrand cross-links (XLR) and in some forms of recombination repair (DSBR). Additionally, evidence has been reported for the involvement of the NER/TCR factor XPG to be also engaged in BER. This extensive multi-functionality implies that the corresponding GM mechanisms are strongly intertwined and should be considered in tight relationship with each other. Consequently, mutations in the different NER factors described above either in patients, transgenic mice or acquired somatic mutations in individual cells have not only major effects in the strict NER context but also important implications for many other GM systems extending into BER, DSBR, XLR, transcription initiation and elongation and -with that- in major DT&S (DNA damage tolerance & signaling) pathways. Thus affecting NER components with mutations such as XPB, XPD, XPG, CSB, CSA and ERCC1/XPF as is the subject in this patent application, at the same time has major effects on many GM mechanisms.

When repair fails, cells may abort their proliferative capacity by executing a permanent cell cycle block called senescence, (Campisi, J. (2001) Trends Cell Biol 11:S27-31) or apoptosis (Bernstein, C., H. et al.,2002, Mutat. Res. 511:145-78).Cells lost via apoptosis or other forms of cell death need to be replaced by progenitors in order to avoid loss of organ functioning. Moreover, even when a high apoptotic rate is sufficiently compensated by new cells, the organism can still suffer from the effect of apoptosis, as elevated levels of apoptosis and tissue regeneration can lead to depletion of the specific stem cell compartment. As such, both apoptosis and senescence are expected in the end to disrupt tissue homeostasis, and thus tissue function. Deterioration of function finally reaches a threshold at which symptoms appear (e.g. joint pain, loss of sensory functions, osteoporosis, organ failure, mental degeneration). Most theories of ageing agree that such changes are due to the accumulation of a variety of damaged cellular biomolecules (lipids, proteins, nucleic acids) and organelles (e.g. mitochondria). Some theories include the accrual of unrepaired DNA damage amongst others in cells of tissues and organs. The scenario for the principal mechanism of ageing that is strongly supported by the findings of the Institute of Genetics points specifically to the accumulation of DNA injury as the main source of ageing. This scenario involves DNA damage which leads to blocking transcription and replication, loss of metabolic and replicative potential of individual cells, induction of senescence and cell death or induction of mutations and chromosomal aberrations. The latter may trigger onset of cancer. The former in the end will culminate in ageing-related diseases, primarily by organ/tissue failures and overall functional decline including reduced resistance to stress (reviewed by Hasty, et al., 2003, Science 299:1355-9 and Mitchell, J. R., J. H. Hoeijmakers, and L. J. Niedernhofer, 2003, Curr Opin Cell Biol 15:232-40).

In this scenario two key factors are relevant for the process of ageing: firstly factors that influence the induction of DNA damage, mainly -but not exclusively-from endogenous origin (including free radicals, chemical decay of DNA, but also scavenger systems that prevent induction of lesions) and secondly the genome maintenance (GM) machinery that attempts to counteract the effects of DNA injury.

An important source of DNA damage are free radicals or reactive oxygen species (ROS), which are chemically highly reactive molecules produced as byproducts of cellular metabolism and thus especially affect body tissues, which are metabolically active. The level of radical formation not only depends on the degree of metabolic activity but also on parameters of mitochondrial functioning and the respiratory chain. The magnitude of the problem is evident from the fact that more than 100 different types of oxidative DNA lesions have already been described, ranging from base modifications to various kinds of single- and double-strand DNA breaks and interstrand cross-links (J.H. Hoeijmakers, Nature, 2001 *supra).* In addition, certain chemical bonds in DNA can undergo spontaneous hydrolysis, leading to abasic sites. E.g. exposure to toxins, infections, smoking and high saturated fat intake in the diet increase production and damage by free-radicals and this accelerates the ageing process. In contrast, restriction of caloric intake will decrease free radical production and is associated with an increase in life span in a wide range of organisms, including mammals.

A final important component in the defence against induction of DNA damage is the elaborate scavenging system, including enzymatic scavengers such as superoxide dismutase (SOD), glutathion-S-transferase (GST) and glutathion synthethase (GSS), low MW scavengers as well as natural or man-made scavengers in e.g. food. In terms of the scenario of aging depicted above all above mentioned factors including the spectrum of GM systems constitute relevant targets for intervention in particular for ameliorating ageing-associated illnesses and handicaps. The link of ageing with the genome maintenance machinery has been highlighted by a still extending series of human syndromes and in particular the generation of animal models with compromised genome maintenance pathways (GM models, subject of this application).

Studies of human nucleotide excision repair syndromes have provided the first indications that GM systems, more specifically DNA repair systems are not only critical for preventing mutations and chromosomal rearrangements thereby thwarting cancer, but may also be involved in preventing at least some ageing-related phenotypes and conditions by counteracting accumulation of DNA damage, ensuring unhampered and unaffected transcription and replication. Several human progeroid syndromes are known that display an accelerated onset of multiple ageing phenotypes and features. Many of these are caused by mutations affecting DNA repair systems and DNA metabolism (i.e. RNA transcription from the DNA template and correct replication of DNA); together referred to as genome maintenance. As patients display early onset of a subset, but not all features of normal ageing, these disorders are considered "segmental" progeroid syndromes. The existence of a possible correlation between genome maintenance and ageing and age-related disease is further emphasized by the fmding that many (if not most) of the other known progeroid syndromes are caused by mutations in genes involved in DNA metabolism. Examples are among others syndromes such as Werner syndrome (WS), Ataxia telangiectasia (AT) and Hutchinson-Gilford progeria syndrome (HGPS). WS is caused by a defect in the WRN RecQ helicase gene. AT is caused by a defect in DNA damage recognition/signalling process by mutations in the ATM gene, while HGPS is due to specific point mutations in a nuclear lamin that plays a role in chromatin organization.

Three human UV sensitivity syndromes are long known but more recently have been explicitly associated with some distinct features of premature ageing, xeroderma pigmentosum (XP), Cockayne syndrome (CS) and trichothiodystrophy (TTD). These three syndromes are NER-disorders (for a review, see Bootsma *et al,,* 2001). The identification of these genetic deficiencies led to the discovery of genes and gene products involved in NER.

Xeroderma pigmentosum (XP) is a multigenic, multiallelic autosomal recessive disease that occurs at a frequency of about 1: 250,000 (USA), but with higher frequency in Japan and the Mediterranean areas. Individuals with XP can be classified into at least seven excision-deficient complementation groups (XP-A to XP-G) in addition to one group called XP-variant in which a defect occurs in the replicational bypass of specific UV-lesions by a special translesion polymerase (DNA damage tolerance). The hallmarks of the disease are, UV(sun)-hypersensitivity, an up to 1000-fold increase in UV-B induced skin cancer (basal and squamous cell carcinomas and melanomas), as well as accelerated photo-ageing of the skin and in some patients neurodegeneration. Heterozygotes appear generally unaffected.

Genes affected in XP are designated XPA, *APB, XPC, XPD, APE, XPF* and *XPG.* The *XPC* and *XPE* genes encode lesion recognition proteins that operate genome wide, whereas the XPA gene product is thought to verify the lesion in a later stage of the NER reaction. XPB and XPD proteins are helicase components of the basal transcription factor complex TFIIH, which is involved in opening the DNA double helix for both basal and activated transcription initiation of RNA polymerase I and II and for the purpose of DNA repair processes prior to incision of the damaged strand by the ERCC1-XPF complex, a structure-specific 5' endonuclease that functions in multiple DNA repair pathways (Niedernhofer et al, EMBO Journal, 2001) and XPG a complementary structure-specific endonuclease which makes an incision 3' to DNA photoproducts (Tian et al, Mol Cell Biol. 2004 Mar;24(6):2237-42).

Cockayne syndrome (CS) is an autosomal, recessive disease characterized by cachectic dwarfism, retinopathy, microcephaly, deafness, neural defects, and retardation of growth and development after birth. The average lifespan of CS patients reported in the literature is limited to 12 years indicating the severity of the disorder. Cause of death is frequently opportunistic infections related to overall physical decline, due to feeding problems and immunological deficits. Patients have a typical facial appearance with sunken eyes, a beaked nose and projecting jaw. CS patients are sun sensitive but remarkably have not been reported to develop cancers, setting this disease apart from XP. Classical CS comprises two complementation groups, CS-A and CS-B, the latter the most common, and is caused by mutations in the *CSA* or *CSB* gene. CSA-and CSB-deficient cells are specifically defective in the TC-NER (transcription-coupled NER) pathway, while the global genome-NER (GG-NER) pathway remains functional. Although in CS only one subpathway of NER is affected, CS patients have a more complex phenotype than XP-A patients, which completely lack both subpathways of NER The CSA and CSB proteins affected in CS are both components of complexes that are associated with RNA polymerase II or indirectly triggered by RNA polymerase II, and their role is thought to be in assisting the polymerase in dealing with DNA damage induced transcription blocks. Thus, the defect in these patients is not limited to TC-NER but extends to TCR in general. Interestingly, mutations in *XPB, XPD* or *XPG* can cause a combination of XP and CS (Bootsma, 2001). Patients with combined XPCS present with CS symptoms, but on top of that suffer from UV skin cancer predisposition. Also these proteins appear to be not only involved in GG-NER and TC-NER, but at the same time in TCR and the TFIIH helicases XPB and XPD are additionally implicated in basal and activated transcription of virtual all genes.

Trichothiodystrophy (TTD) is a rare autosomal recessive disorder characterized by sulfur-deficient brittle hair and ichthyosis. Hair shafts split longitudinally into small fibers, and this brittleness is associated with levels of cysteine/cystine in hair proteins that are 15 to 50% of those in normal individuals. The hair has characteristic "tiger-tail" banding visible under polarized light. The patients often have an unusual facial appearance, with protruding ears and a receding chin. Mental abilities range from low normal to severe retardation. Several categories of the disease can be recognized on the basis of cellular responses to UV damage and the affected gene. Severe cases have low NER activity and mutations in XPB, XPD or TTDA genes. The latter gene has been cloned recently and encodes a very small 76 kD polypeptide that is important for the repair functions of TFIIH and that stabilises the 10 subunit complex (Giglia-Mari et al., Nature Genetics, 2004). TTD patients do not exhibit increased incidence of skin cancer. Corresponding knock-in mice with a human TTD point mutation in the *Xpd* gene display moderately increased skin cancer upon UV exposure, however spontaneous cancer may be reduced, consistent with the human syndrome (de Boer et al., Cancer Res. 1998). XPB is part of the core of TFIIH and has a central role in transcription, whereas XPD connects the core to the CAK subcomplex, and can tolerate many different mutations. Subtle differences in the effects of these individual mutations on the many activities of TFIIH (GG-NER, TC-NER, TCR and transcription initiation) and on its stability determine the clinical outcomes, which can be XP, TTD, XP with CS and XP with TTD.

An additional very rare novel progeroid syndrome involving a NER complex was recently discovered by the team of the applicant. This autosomal recessive condition, which is provisionally designated XPF/ERCC1 (XFE) syndrome, has been observed in 2 cases, which exhibited striking parallels with the mouse models previously established. One case is due to a severe mutation in the XPF gene, causing multi-system accelerated ageing from the age of approximately 10 years with involvement of developmental, dermatological, hematological, hepatic, renal, and severe neurological symptoms leading to early death at the age of 16. The other case was due to a severe mutation in the *ERCC1* gene, causing multi-system failure and death around the first year of life. This syndrome and the corresponding *Ercc1* mouse mutant have several features distinctive from the above mentioned other NER/TCR syndromes. These stem most likely from the additional engagement of the ERCC1/XPF endonuclease in XLR and parts of the DSBR pathways. This again emphasizes the strong interwoven nature of the various GM mechanisms and their link with (accelerated) ageing.

An overview of genes involved in NER and which are mutated in humans are shown in table I below. A comprehensive and frequently updated list of more than 360 XP, TTD and CS mutations in humans can be found on www.xpmutations.org. The mouse with its relatively short lifespan, easy genetic accessibility and close genetic and physiological relatedness to humans, can provide a suitable tool to model premature and accelerated ageing phenotypes. A number of mouse models with engineered defects in genome maintenance (GM mice) by knocking out NER genes (Weeda et al, van der Horst et al., DNA Repair 2002) or by introduction of mutations (closely) mimicking human XP, CS, XPCS or TTD mutations in NER-related genes have been generated and partially characterized, including those by the authors of the current invention. For instance van der Horst et al, Cell, 1997, de Boer et al, Cancer Research 1999, Niedernhofer et al., EMBO Journal 2001, de Boer et al., Science 2002, all provide mouse models with NER defects, some of which display a phenotype comprising hallmarks of accelerated or premature ageing. For a review see Hoeijmakers, Nature 2001, Hasty et al, Science 2003, Hasty and Vijg, Aging Cell, 2004.

The wide variety of mutations in XP, CS and TTD patients give rise to different phenotypes, with specific characteristic features and a varying severity of the disorder. Recent research in NER/TCR/XLR-deficient humans and NER/TCR/XLR/DSBR mouse-models by the current inventors have led to the observation that mutations in GM genes affecting mainly global genome repair systems (such as GG-NER, BER) lead grosso modo to a cancer-prone phenotype. On the other hand, mutations in GM genes specifically affecting TCR or other (repair) systems that promote cellular survival from DNA damage, such as repair and damage processing of the very cytotoxic interstrand crosslinks and double strand breaks give mainly rise to a premature and enhanced ageing phenotype. The latter XLR, DSBR, DT&S systems are particularly relevant for proliferating cells.

The current invention is based on this concept and thus links ageing with any pathway relevant for DNA damage induced cell death or cellular senescence. In addition it seeks to exploit the striking difference in the biological effect of GG-NER/BER and the other GM deficiencies such as TCR/XLR/DSBR and DT&S for exploring the nature of the ageing process and means to influence this by interfering with DNA damage induction or processing.

Table I

| Gene | Patient | Mutation Type | Mutation Detail (nt) | Mutation Detail (aa) |
|------|---------|---------------|----------------------|----------------------|
| Gene | Patient | Mutation Type | Mutation Detail (nt) | Mutation Detail (aa) |
| CSA | CS5BR | deletion | 81&279del in cDNA | |
| CSA | GM2964 | nucleotide substitution, nonsense | | Y322ter |
| CSB | 25627 | nucleotide substitution, nonsense | C2282T | R735ter |
| CSB | 25627 | nucleotide substitution, nonsense | C1436T | R453ter |
| CSB | CS10BR | deletion, frameshift | 3686ins26 | fs1203-1235ter |
| CSB | CS10LO | deletion, frameshift | 1359ins1 | fs427-435ter |
| CSB | CS1ABR | deletion, frameshift | _del_ | fs715-738ter |
| CSB | CS1BE | nucleotide substitution, missense | C2087T | R670W |
| CSB | CS1BE | deletion, frameshift | _del_ | fs1179-1200ter |
| CSB | CS1BO | deletion | _del_ | fs506-542ter |
| CSB | CS1BO | nucleotide substitution, nonsense | C2918T | R947ter |
| CSB | CS1IAF | nucleotide substitution, missense | T2949G | V957G |
| CSB | CS1MA | deletion | exon10del | 665-723del |
| CSB | CS1TAN | nucleotide substitution, nonsense | C2282T | R735ter |
| CSB | CS2BE | deletion, frameshift | 3614del1 | fs1179-1200ter |
| CSB | CS2BE | deletion | exon10del | 665-723del |

(continued)

| CSB | CS2BI | nucleotide substitution, missense | C2087T | R670W |
|---|---|---|---|---|
| CSB | CS2BI | nucleotide substitution, missense | C3204T | P1042L |
| CSB | CS2TAN | nucleotide substitution, nonsense | G1630A | W517ter |
| CSB | CS3TAN | nucleotide substitution, missense | T2630A | W851R |
| CSB | CS4BR | nucleotide substitution, nonsense | C629T | Q184ter |
| CSB | CS4BR | nucleotide substitution, missense | C2087T | R670W |
| CSB | CS7TAN | nucleotide substitution, missense | A3716G | R1213G |
| CSB | CS8BR | nucleotide substitution, nonsense | C2639T | Q854ter |
| CSB | synthetic | nucleotide substitution, missense | | K>R |
| CSB | | nucleotide substitution, missense | G3363C | P1095R |
| CSB | | deletion, frameshift | __del_ | fs1179-1200ter |
| XPA | A.S. | nucleotide substitution, splice site | G>C | missplice/fs |
| XPA | AG6971 | deletion, frameshift | del(C349-T353) | frames hift |
| XPA | AG6971 | nucleotide substitution, splice site | A>G | new splice |
| XPA | FTMR | nucleotide substitution, splice site | G>C | missplice |
| XPA | FTMR | nucleotide substitution, nonsense | C682T | R228ter |
| XPA | J.K | nucleotide substitution, splice site | G>C | missplice/fs |
| XPA | J.K | nucleotide substitution, nonsense | C682T | R228ter |
| XPA | K.F. | nucleotide substitution, splice site | G>C | missplice/fs |
| XPA | K.I. | nucleotide substitution, splice site | G>C | missplice/fs |
| XPA | K.I. | | ? | ? |
| XPA | K.K. | nucleotide substitution, splice site | G>C | missplice/fs |
| XPA | K.U. | nucleotide substitution, splice site | G>C | missplice/fs |
| XPA | M.T. | nucleotide substitution, splice site | G>C | missplice/fs |
| XPA | M.T. | | ? | ? |
| XPA | M.Y. | nucleotide substitution, splice site | G>C | missplice/fs |
| XPA | M.Y. | nucleotide substitution, nonsense | T348A | Y116ter |
| XPA | Ma.Y. | nucleotide substitution, nonsense | C682T | R228ter |
| XPA | Ma.Y. | | ? | ? |

(continued)

| | | | | |
|------|--------|---------------------------------------|--------|---------------|
| XPA | Mi.Y. | nucleotide substitution, nonsense | C682T | R228ter |
| XPA | Mi.Y. | | ? | ? |
| XPA | S.K | nucleotide substitution, splice site | G>C | missplice/fs |
| XPA | S.K. | nucleotide substitution, nonsense | C682T | R228ter |
| XPA | T.M. | nucleotide substitution, splice site | G>C | missplice/fs |
| XPA | XP104TO | nucleotide substitution, splice site | G>C | missplice/fs |
| XPA | XP10OS | nucleotide substitution, splice site | G>C | missplice/fs |
| XPA | XP11KY | nucleotide substitution, splice site | G>C | missplice/fs |
| XPA | XP11OS | nucleotide substitution, splice site | G>C | missplice/fs |
| XPA | XP11TU | nucleotide substitution, nonsense | C682T | R228ter |
| XPA | XP12BE | nucleotide substitution, splice site | G>T | missplice/fs |
| XPA | XP12BE | nucleotide substitution, splice site | G555C | missplice/ins |
| XPA | XP12RO | nucleotide substitution, nonsense | C619T | R207ter |
| XPA | XP12TU | nucleotide substitution, nonsense | C682T | R228ter |
| XPA | XP13KY | nucleotide substitution, splice site | G>C | missplice/fs |
| XPA | XP13LO | nucleotide substitution, splice site | G555C | missplice |
| XPA | XP13TU | nucleotide substitution, nonsense | C682T | R228ter |
| XPA | XP15KY | nucleotide substitution, splice site | G>C | missplice/fs |
| XPA | XP15OS | nucleotide substitution, splice site | G>C | missplice/fs |
| XPA | XP16NA | nucleotide substitution, splice site | G>C | missplice/fs |
| XPA | XP18KY | nucleotide substitution, splice site | G>C | missplice/fs |
| XPA | XP18OS | nucleotide substitution, nonsense | T348A | Y116ter |
| XPA | XP19KY | nucleotide substitution, splice site | G>C | missplice/fs |
| XPA | XP1BP | nucleotide substitution, splice site | G>C | missplice |
| XPA | XP1CA | deletion, frameshift | delC374 | frameshift |
| XPA | XP1EH | nucleotide substitution, splice site | G>C | missplice/fs |
| XPA | UP1FI | nucleotide substitution, splice site | G>C | missplice/fs |

(continued)

| | | | | | |
|---|---|---|---|---|---|
| XPA | XP1HM | nucleotide substitution, splice site | G>C | | missplice/fs |
| XPA | XP1KG | nucleotide substitution, splice site | G>C | | missplice/fs |
| XPA | XP1KN | nucleotide substitution, splice site | G>C | | missplice/fs |
| XPA | XP1KR | nucleotide substitution, nonsense | C682T | | R228ter |
| XPA | XP1MG | nucleotide substitution, splice site | G>C | | missplice/fs |
| XPA | XP1NI | nucleotide substitution, splice site | G>C | | missplice |
| XPA | XP1OS | nucleotide substitution, splice site | G>C | | missplice/fs |
| XPA | XP1OS | nucleotide substitution, nonsense | C682T | | R228ter |
| XPA | XP1PD | deletion, frameshift | del(C349-T353) | | frameshift |
| XPA | XP1PD | nucleotide substitution, missense | G323T | | C108F |
| XPA | XP1WI | nucleotide substitution, splice site | G555C | | missplice |
| XPA | XP22KY | nucleotide substitution, splice site | G>C | | missplice/fs |
| XPA | XP22OS | nucleotide substitution, splice site | G>C | | missplice/fs |
| XPA | XP22SF | deletion, frameshift | del(A468-A487) | | frameshift |
| XPA | XP22SF | insertion, frameshift | A insertion (663) | | frameshift |
| XPA | XP23CA | nucleotide substitution, nonsense | C683T | | R228ter |
| XPA | XP25RO | nucleotide substitution, nonsense | C619T | | R207ter |
| XPA | XP26KY | nucleotide substitution, splice site | G>C | | missplice/fs |
| XPA | XP26SF | deletion, frameshift | del(A468-A487) | | frameshift |
| XPA | XP26SF | insertion, frameshift | A insertion (663) | | frameshift |
| XPA | XP27OS | nucleotide substitution, splice site | G>C | | missplice/fs |
| XPA | XP27OS | nucleotide substitution, nonsense | C682T | | R228ter |
| XPA | XP27TU | nucleotide substitution, nonsense | C682T | | R228ter |
| XPA | XP2CA | deletion, frameshift | delC374 | | frameshift |
| XPA | XP2HM | nucleotide substitution, splice site | G>C | | missplice/fs |
| XPA | XP2KY | nucleotide substitution, splice site | G>C | | missplice/fs |
| XPA | XP2NI | nucleotide substitution, splice site | G>C | | missplice/fs |
| XPA | XP2NI | nucleotide substitution, splice site | G673C | | missplice |
| XPA | XP2OS | nucleotide substitution, splice site | G>C | | missplice |
| XPA | XP2PD | deletion, frameshift | del(C349-T353) | | frameshift |

(continued)

| | | | | |
|------|--------|-----------------------------------------|--------------|-------------|
| XPA | XP2PD | nucleotide substitution, missense | G323T | C108F |
| XPA | XP31TO | nucleotide substitution, splice site | G>C | missplice/fs |
| XPA | XP32TO | nucleotide substitution, splice site | G>C | missplice/fs |
| XPA | XP33TU | nucleotide substitution, nonsense | C682T | R228ter |
| XPA | XP34OS | nucleotide substitution, splice site | G>C | missplice/fs |
| XPA | XP35OS | nucleotide substitution, splice site | G>C | missplice/fs |
| XPA | XP35TO | nucleotide substitution, splice site | G>C | missplice/fs |
| XPA | XP39OS | nucleotide substitution, nonsense | C682T | R228ter |
| XPA | XP3HM | nucleotide substitution, splice site | G>C | missplice/fs |
| XPA | XP3JO | nucleotide substitution, splice site | G389A | no splice |
| XPA | XP3KG | nucleotide substitution, splice site | G>C | missplice/fs |
| XPA | XP3KR | nucleotide substitution, splice site | G>C | missplice |
| XPA | XP3KR | nucleotide substitution, nonsense | C682T | R228ter |
| XPA | XP3OS | nucleotide substitution, splice site | G>C | missplice/fs |
| XPA | XP42OS | nucleotide substitution, splice site | G>C | missplice/fs |
| XPA | XP45OS | nucleotide substitution, splice site | G>C | missplice |
| XPA | XP45OS | nucleotide substitution, nonsense | C682T | R228ter |
| XPA | XP46OS | nucleotide substitution, splice site | G>C | missplice |
| XPA | XP46OS | nucleotide substitution, nonsense | C682T | R228ter |
| XPA | XP4JO | nucleotide substitution, splice site | G389A | no splice |
| XPA | XP4KG | nucleotide substitution, splice site | G>C | missplice/fs |
| XPA | XP4KR | nucleotide substitution, splice site | G>C | missplice |
| XPA | XP4KR | nucleotide substitution, nonsense | C682T | R228ter |
| XPA | XP4LO | deletion, frameshift | del(A468-A469) | frameshift |
| XPA | XP54TO | nucleotide substitution, splice site | G>C | missplice/fs |
| XPA | XPSCA | nucleotide substitution, splice site | A>G | no splice |
| XPA | XP5JO | nucleotide substitution, splice site | G389A | no splice |

(continued)

| XPA | XP5PD | nucleotide substitution, splice site | G555C | missplice |
|---|---|---|---|---|
| XPA | XP5PD | nucleotide substitution, nonsense | C631T | R211ter |
| XPA | XP67TO | nucleotide substitution, splice site | G>C | missplice/fs |
| XPA | XP67TO | nucleotide substitution, nonsense | T348A | Y116ter |
| XPA | XP6EH | nucleotide substitution, splice site | G>C | missplice/fs |
| XPA | XP6TO | nucleotide substitution, splice site | G>C | missplice/fs |
| XPA | XP75TO | nucleotide substitution, splice site | G>C | missplice/fs |
| XPA | XP75TO | nucleotide substitution, nonsense | C682T | R228ter |
| XPA | XP78TO | nucleotide substitution, splice site | G>C | missplice/fs |
| XPA | XP7TO | nucleotide substitution, splice site | G>C | missplice/fs |
| XPA | XP84TO | nucleotide substitution, splice site | G>C | missplice/fs |
| XPA | XP87TO | nucleotide substitution, splice site | G>C | missplice/fs |
| XPA | XP8LO | nucleotide substitution, splice site | G555C | missplice |
| XPA | XP8LO | nucleotide substitution, missense | A731G | H244R |
| XPA | XP8MY | nucleotide substitution, splice site | G>C | missplice/fs |
| XPA | XP8OS | nucleotide substitution, splice site | G>C | missplice/fs |
| XPA | XP8OS | | 2nd allele na | |
| XPA | XP8TU | nucleotide substitution, nonsense | C682T | R228ter |
| XPA | XP96TO | nucleotide substitution, splice site | G>C | missplice/fs |
| XPA | XP9KY | nucleotide substitution, splice site | G>C | missplice/fs |
| XPA | XPEMB-1 | nucleotide substitution, splice site | G>C | missplice/fs |
| XPA | XRITS | nucleotide substitution, splice site | G>C | missplice/fs |
| XPA | Y.H. | nucleotide substitution, splice site | G>C | missplice/fs |
| XPA | Y.K. | nucleotide substitution, splice site | G>C | missplice/fs |
| XPA | Y.N | nucleotide substitution, splice site | G>C | missplice/fs |
| XPA | Y.N | | ? | ? |
| XPB | TTD4VI | nucleotide substitution, missense | A>C | T119P |

(continued)

| | | | | |
|------|----------|----------------------------------------|----------------------|---------------------------------------|
| XPB | TTD6VI | nucleotide substitution, missense | A>C | T119P |
| XPB | XP11BE | nucleotide substitution, splice site | C>A | missplice/fs |
| XPB | XP11BE | | ? | not expressed |
| XPB | XPCS1BA | nucleotide substitution, missense | T>C | F99S |
| XPB | XPCS1BA | | ? | not expressed |
| XPB | XPCS2BA | nucleotide substitution, missense | T>C a | F99S |
| XPB | XPCS2BA | | ? | not expressed |
| XPC | XP1BE | deletion, frameshift | DEL (A1396-A1397) | frameshift |
| XPC | XP1BE | deletion, frameshift | DEL (A1396-A1397) | frameshift |
| XPC | XP1MI | nucleotide substitution, missense | C1106A | P343H |
| XPC | XP1MI | nucleotide substitution, missense | C1106A | P343H |
| XPC | XP22BE | nucleotide substitution, splice site | +2 IV 9,T to G | see comments |
| XPC | XP3BE | insertion, frameshift | ins83bp @nt462 | frameshift |
| XPC | XP4PA | deletion, frameshift | DEL (T1744-G1745) | frameshift |
| XPC | XP4PA | deletion, frameshift | DEL (T1744-G1745) | frameshift |
| XPC | XP8BE | insertion | insertion of GTG | insV697 |
| XPC | XP8BE | nucleotide substitution, missense | A>C | K822Q |
| XPD | TTD10PV | nucleotide substitution, missense | G335A | R112H |
| XPD | TTD10VI | nucleotide substitution, missense | G1973A | R658H |
| XPD | TTD11PV | nucleotide substitution, missense | G335A | R112H |
| XPD | TTD11PV | deletion | del363-477 | del121>159 (exon 6) (likely null) |
| XPD | TTD11VI | nucleotide substitution, missense | G1973A | R658H |
| XPD | TTD12PV | nucleotide substitution, missense | G776A | C259Y |
| XPD | TTD12PV | nucleotide substitution, missense | C2164T | R722W |
| XPD | TTD15PV | nucleotide substitution, missense | G776A | C259Y |
| XPD | TTD15PV | nucleotide substitution, missense | C2164T | R722W |
| XPD | TTD183ME | complex | {C1381G, del2146>2190} | L461V, del7l6>730 (likely null) |
| XPD | TTD183ME | nucleotide substitution, missense | G2173C | A725P |
| XPD | TTD1BEL | nucleotide substitution, missense | C2164T | R722W |
| XPD | TTD1BEL | nucleotide substitution, missense | G1847C | R616P (likely null) |
| XPD | TTD1BI | deletion, frameshift | G2189del | 730fs>744ter |
| XPD | TTD1BI | | not expressed | null |

(continued)

| XPD | TTD1PV | nucleotide substitution, missense | G335A | R112H |
|-----|--------|-----------------------------------|-------|-------|
| XPD | TTD1RO | nucleotide substitution, missense | {C1972T,A2251C} | R658C,K751Q |
| XPD | TTD1RO | nucleotide substitution, missense | G2137C | G713R |
| XPD | TTD1VI | complex | {C1381G. del2l46>2190} | L461V, del716>730 (likely null) |
| XPD | TTD1VI | | C2164T | R722W |
| XPD | TTD2BR | deletion, frameshift | G2189del | 730fs>744ter |
| XPD | TTD2BR | deletion, complex | {deN462>1479, de11378>1479} | {del488>493, del460>493} (likely null) |
| XPD | TTD2GL | nucleotide substitution, missense | G335A | R112H |
| XPD | TTD2GL | deletion, complex | {del1462>1479, del1378>1479} | {del488>493, del460>493} (likely nulla) |
| XPD | TTD2PV | nucleotide substitution, missense | G335A | R112H |
| XPD | TTD3PV | nucleotide substitution, missense | G335A | R112H |
| XPD | TTD3VI | complex | {C1381G, del2146>2190} | L461V, del716>730 (likely null) |
| XPD | TTD3VI | | G1973A | R658H |
| XPD | TTD4PV | nucleotide substitution, missense | G335A | R112H |
| XPD | TTD4PV | | del1445>1447 | del482 (likely null) |
| XPD | TTD6PV | nucleotide substitution, missense | A2018G | D673G |
| XPD | TTD6PV | | not expressed | null |
| XPD | TTD7PV | nucleotide substitution, missense | C2164T | R722W |
| XPD | TTD7PV | | {C1381G, del2146>2190} | L461V, de1716>730 (likely null) |
| XPD | TTD8PV | nucleotide substitution, missense | G335A | R112H |
| XPD | TTD9VI | nucleotide substitution, missense | G335A | R112H |
| XPD | XP-CS2 | nucleotide substitution, missense | G1805A | G602D |
| XPD | XP-CS2 | | not expressed | ? |
| XPD | XP102LO | nucleotide substitution, missense | C2047T | R683W |
| XPD | XP102LO | complex | {C1381G, del2146>2190} | L461V, de1716>730 (likely null) |
| XPD | XP107LO | nucleotide substitution, missense | C2047T | R683W |
| XPD | XP111LO | nucleotide substitution, missense | C2047T | R683W |
| XPD | XP135LO | nucleotide substitution, missense | C2047T | R683W |
| XPD | XP15PV | nucleotide substitution, missense | G2048A | R683Q |

(continued)

| XPD | XP16BR | nucleotide substitution, missense | C2047T | R683W |
|---|---|---|---|---|
| XPD | XP16BR | nucleotide substitution, missense | G1847C | R616P (likely null) |
| XPD | XP16OS | deletion, frameshift | del590>593 | 197fs/ter (null) |
| XPD | XP16OS | nucleotide substitution, missense | A1621C | S541R |
| XPD | XP16PV | nucleotide substitution, missense | G2048A | R683Q |
| XPD | XP17PV | nucleotide substitution, missense | C2047T | R683W |
| XPD | XP17PV | nucleotide substitution, missense | G1847A | R616P |
| XPD | XP1BR | nucleotide substitution, missense | C2047T | R683W |
| XPD | XP1BR | nucleotide substitution, missense | C2047T | R683W |
| XPD | XP1DU | nucleotide substitution, missense | C2047T | R683W |
| XPD | XP1DU | nucleotide substitution, missense | not reported | R616W (likely null) |
| XPD | XP1NE | complex | {C1381G, del2146>2190} | L461V,del716>730 (likely nullc) |
| XPD | XP1NE | nucleotide substitution, missense | G139A | G47R |
| XPD | XP22VI | nucleotide substitution, missense | C2047T | R683W |
| XPD | XP23VI | nucleotide substitution, missense | C2047T | R683W |
| XPD | XP26VI | nucleotide substitution, missense | C2047T | R683W |
| XPD | XP26VI | nucleotide substitution, missense | G1847C | R616P (likely null) |
| XPD | XP2NE | nucleotide substitution, missense | C2047T | R683W |
| XPD | XP2NE | nucleotide substitution, missense | C2047T | R683W |
| XPD | XP43KO | nucleotide substitution, missense | not reported | R601L |
| XPD | XP43KO | nucleotide substitution, missense | not reported | D234N |
| XPD | XP67MA | nucleotide substitution, nonsense | C2176T | Q726ter |
| XPD | XP67MA | | not reported | not reported |
| XPD | XP6BE | nucleotide substitution, missense | C2047T | R683W |
| XPD | XP6BE | deletion | del106>183 | de136>61 (likely null) |
| XPD | XP8BR | nucleotide substitution, missense | G2023C | G675R |
| XPD | XP8BR | deletion, frameshift | A2005del | 669fs>708ter |
| XPD | XP9MA | nucleotide substitution, missense | G2048A | R683Q |

(continued)

| | | | | |
|---|---|---|---|---|
| XPD | XP9MA | nucleotide substitution, missense | G2048A | R683Q |
| XPD | XPJCLO | nucleotide substitution, missense | C2047T | R683W |
| XPD | XPLABE | nucleotide substitution, missense | not reported | Y542C |
| XPD | XPLABE | nucleotide substitution, missense | G1847C | R616P (likely null) |
| XPE | XP2RO | nucleotide substitution, missense | G818A | R273H |
| XPE | XP3RO | nucleotide substitution, missense | G818A | R273H |
| XPE | XPE82TO | nucleotide substitution, missense | A730G | K244E |
| XPF | Up101 ops | nucleotide substitution, missense | A642G | 1214M |
| XPF | XP1010S | nucleotide substitution, missense | G1504A | G502R |
| XPF | XP126LO | nucleotide substitution, missense | C2377T | R788W |
| XPF | XP126LO | deletion | ?del? | 803ter |
| XPF | XP1TS | deletion | del(1779-1799) | V594-G600 |
| XPF | XP23OS | insertion | 1330ins | K444>ter482 |
| XPF | XP24KY | deletion | del(1575-1584) | V525ter |
| XPF | XP24KY | nucleotide substitution, missense | A1327T | R443W |
| XPF | XP2YO | deletion | T1937del | E646ter |
| XPF | XP2YO | nucleotide substitution, missense | A1666G | T556A |
| XPF | XP3YO | nucleotide substitution, missense | G1436A | R479Q |
| XPF | XP3YO | nucleotide substitution, missense | T1790C | L599P |
| XPF | XP42RO | nucleotide substitution, missense | C2365T | R788W |
| XPF | XP7KA | nucleotide substitution, missense | G1471A | E491K |
| XPF | XP7KA | nucleotide substitution, missense | T1553C | I518T |
| XPG | 94RD27 | deletion, frameshift | del(T2972) | frameshift,Tyf322ter |
| XPG | XP124LO | nucleotide substitution, nonsense | G(3075)T | 960ter |
| XPG | XP124LO | nucleotide substitution | C(2572)T | ? |
| XPG | XP125LO | nucleotide substitution, missense | G(3075)T | A792V |
| XPG | XP125LO | nucleotide substitution | C(2572)T | ? |
| XPG | XP20BE/CS | nucleotide substitution | G>C | |
| XPG | XP20BE/CS | nucleotide substitution | A>T | |
| XPG | XPCS1LV | deletion | del(A2170-A2172) | 659ter |
| XPG | XPCS2LV | deletion | def(A2170-A2172) | nonsense |
| XPG | XPCS2LV | nucleotide substitution, missense | C984T | R263ter |
| XPV | XP51VI | deletion | del(661-764) | Fs163 |

(continued)

| | | | | |
|---|---|---|---|---|
| XPV | XP56RO | nucleotide substitution, nonsense | G890A | W297stop |
| XPV | XP56RO | deletion | del(661-764) | Fs163 |
| XPV | XP53RO | nucleotide substitution, nonsense | C1066T | Arg355stop |
| XPV | XP53RO | deletion | del(661-764) | Fs163 |
| XPV | XP53RO | insertion | insT882 | Fs294 |
| XPV | XP52RO | nucleotide substitution, nonsense | C1066T | Arg355stop |
| XPV | XP62VI | deletion | del(1075-1244) | Fs358 |
| XPV | XP75VI | deletion | del(1075-1244) | Fs358 |
| XPV | XP28VI | deletion | del(1075-1244) | Fs358 |
| XPV | XP28VI | insertion | insC1091 | Fs364 |
| XPV | XP127VI | insertion | insC1091 | Fs364 |
| XPV | XP7DU | deletion | del(1222-1225) | Fs407 |
| XPV | XP7DU | | ? | ? |
| XPV | XP58RO | deletion | del(224-226) | delLeu75 |
| XPV | XP2DU | deletion | del(224-226) | delLeu75 |
| XPV | XP2DU | deletion | delG207 | Fs69 |
| XPV | XP3DU | deletion | del(224-226) | delLeu75 |
| XPV | XP3DU | deletion | delG207 | Fs69 |
| XPV | XP6DU | deletion | del(224-226) | delLeu75 |
| XPV | XP6DU | deletion | delG207 | Fs69 |
| XPV | XP7BR | deletion | del(224-226) | delLeu75 |
| XPV | XP7BR | insertion | ins764 | Fs255 |
| XPV | XP36BR | nucleotide substitution, missense | G332A | Arg111His |
| XPV | XP36BR | depletion | del(1222-1225) | Fs407 |
| XPV | XPSBI | nucleotide substitution, missense | A364C | Thr122Pro |
| XPV | XP5BI | deletion | del(1222-1225) | Fs407 |
| XPV | XP11BR | nucleotide substitution, missense | G788T | Gly263Val |
| XPV | XP57RO | nucleotide substitution, missense | G1083T | Arg361Ser |
| XPV | XP86VI | nucleotide substitution, nonsense | C1561T | Glu521Stop |
| XPV | XP1AB | nucleotide substitution, nonsense | C1543A | Thr548Stop |
| XPV | XP1AB | deletion | del(224-226) | delLeu75 |
| XPV | XP37BR | insertion | ins1688 | Fs556 |
| XPF | XP80TO | | | |
| XPF | XP81TO | | | |
| XPV or UVS | XP93TO | | | |
| XPV or UVS | XP95TO | | | |
| XPE DDB2 | XP23PV | splice donor defect | G>T Intron VII | del 235-107aa |
| XPE DDB2 | XP27PV | deletion -> frameshift | del905-908; | Lys2440pa; |
| XPE DDB2 | XP27PV | deletion -> frameshift | del878-1055 | Fs235-> stop 10 codons 3' |
| XPE DDB2 | XP27PV | deletion | del 878-1198 | del235-341 aa |
| XPE DDB2 | XP25PV | transition | G1093A | silent |
| XPE DDB2 | XP25PV | transversion | G1094T | Asp307Tyr |
| XPE DDB2 | GM01389 | transversion | T1224C | Leu350Pro |

(continued)

| | | | | |
|---|---|---|---|---|
| XPE DDB2 | GM01389 | deletion | | del Asn349 |
| WS | XP24KO | | | |
| XPV | XP43TO | | | |
| XPF | XP89TO | | | |
| XPC | XP12PV | deletion -> frameshift | delC128 | fs43 -> 78stop |
| XPC | XP18PV | deletion -> frameshift | del C128 | fs43 -> 78stop |
| XPC | XP19PV | deletion -> frameshift | del C128 | fs43 -> 78stop |
| XPC | XP19PV | deletion -> frameshift | del AA1103-1104 | fs368 -> 373stop |
| XPC | XP5PV | insertion -> frameshift | ins AA321 | fs108 -> 113stop |
| XPC | XP13PV | insertion-> frameshift | ins AA321 | fs108 -> 113stop |
| XPC | XP13PV | insertion -> frameshift | ins T671 | fs257 -> 268stop |
| XPC | XP13PV | missense | G2069C | Trp690Ser |
| XPC | XP13PV | polymorphism | G1475A | Arg492His |
| XPC | XP13PV | polymorphism | T1496C | Val499Ala |
| XPC | XP13PV | polymorphism | G2061A | Arg687 |
| XPC | XP4BR | insertion -> frameshift | ins T671 | fs257 -> 268stop |
| XPC | XP26PV | deletion -> frameshift | del TG1643-1644 | fs548 -> 572stop |
| XPC | XP26PV | deletion | del 1627-1872 | del 543-624 |
| XPC | XP10PV | deletion | del 1627-1872 | del 543-624 |
| XPC | XP10PV | missense | C1735T | Arg579opal |
| XPC | XP9PV | deletion -> frameshift | del C2257 | fs753 -> 766stop |
| XPC | XP9PV | deletion -> frameshift | del 2251-2420 | del 751-806 -> 808stop |
| XPC | XP14BR | nonsense | C2152T | Arg718opal |
| XPC | XP4BR | polymorphism | G1475A | Arg492His |
| XPC | XP6BR | deletion -> frameshift | del 2421-2604 | fs807 -> 856stop |
| XPC | XP6BR | polymorphism | G1475A | Arg492His |
| XPC | XP10PV | polymorphism | G1475A | Arg492His |
| XPC | XP4RO | polymorphism | G1475A | Arg492His |
| XPC | XP9PV | polymorphism | G1475A | Arg492His |
| XPC | XP26PV | polymorphism | G1475A | Arg492His |
| XPC | XP4BR | polymorphism | G1475A | Arg492His |
| XPC | XP5PV | polymorphism | G1475A | Arg492His |
| XPC | XP19PV | polymorphism | G1475A | Arg492His |
| XPC | XP19PV | polymorphism | C303T | Asp101 |
| XPC | XP5PV | polymorphism | G2061A | Arg687 |
| XPC | XP4BR | polymorphism | G2061A | Arg687 |
| XPC | XP4RO | polymorphism | G2061A | Arg687 |
| XPC | XP6BR | polymorphism | G2061A | Arg687 |
| XPC | XP19PV | polymorphism | A2815C | Lys939Gln |
| XPC | XP9PV | polymorphism | A2815C | Lys939Gln |
| XPC | XP10PV | polymorphism | A2815C | Lys939Gln |

Embodiments of the invention

Mutations in genes affecting global genome NER primarily lead to cancer-prone phenotypes, whereas mutations in genes specifically affecting transcription-coupled repair (TCR) and -as part of this invention- all other mechanisms relevant for genome protection to prevent DNA damage-induced cell death or cell cycle arrest (GM mechanisms as defined above) give primarily rise to premature and enhanced ageing phenotypes. Moreover, such TCR-related premature and enhanced ageing phenotypes can be further boosted by an additional defect in GG-NER, as is evident from the phenotype of double mutant mouse models in which the TCR defect is combined with an *Xpa* or *Xpc* defect. The current invention seeks to develop and exploit new animal models with TCR/XLR/DSBR/DT&S deficiencies, with or without additional mutations in the *Xpa* or other GM genes, that result in impaired genome maintenance, and increased cell death or replicative senescence and that give rise to a premature, accelerated and enhanced segmental ageing phenotype.

The current invention pertains to a method for screening and discovery of compounds or mixtures of compounds capable of preventing, delaying, inhibiting or curing GM disorders, more specifically the interlinked NER/TCR/XLR/DSBR disorders. In particular it provides a method for screening for compounds or mixtures of compounds capable of inhibiting, preventing, delaying or reducing to some extent symptoms of NER/TCR/XLR/DSBR and other GM disorders, in particular ageing-related symptoms and conditions in mammals brought about by said disorders. In addition, by virtue of findings presented in this application it provides a method for screening for (mixtures of) compounds that inhibit, prevent, delay or reduce to some extent ageing-related symptoms and pathology in normally ageing mammals. By the application or administration of thus selected compounds, the invention also provides strategies of therapeutic intervention for ageing symptoms or ageing-related conditions, in GM disorders or diseases, as well as natural ageing. The therapeutic intervention may comprise the administration of the selected compound or compounds as a pharmaceutical, nutraceutical, or a cosmetic composition.

The method of screening for compounds according to the invention is aimed at the discovery and use of a) new compounds or compositions or b) new uses of known compounds and compositions, as new treatments for alleviating GM defects or mild aberrations in GM (such as polymorphic variants, with subtle deficiencies) and ageing-related symptoms. Treatment comprises prevention, reduction, slowing down of progression and/or onset of ageing related symptoms. The ageing-related symptoms to be treated with these selected or newly identified compounds may be ageing-related symptoms brought about by rare genetic defects and disorders or by more frequently occurring natural variants in GM systems, such as preferably but not limited to NER/TCR/XLR/DSBR/DT&S, but may also be ageing-related symptoms and diseases observed during normal ageing in a subject. Hence the identified and/or selected compounds or compositions by the screening method of the current invention will provide new treatments and therapies for both premature and normal ageing-related conditions in animals and in humans.

In another aspect the invention provides methods for developing animal models, preferably mouse models, carrying one or more mutations in genes affecting the GM capacity and, in particular TCR combined with other GM systems, as well as cells derived thereof Preferably, GG-NER and TCR mutants, or double or even triple mutants, may be used that display a premature ageing phenotype, which are particularly well suited for the method of screening compounds and/or substances or compositions according to the invention, that will prevent, inhibit, delay or reduce an ageing-related parameter in the animal model. More preferably, such animal models display tissue-specific aging pathology through inactivation of GM systems in a single or limited number of tissues or organs, including, but not limited to skin, bone, brain or retina. The GM mutant mammals may be heterozygous and preferably are homozygous for the mutations in respective systems.

In a first embodiment the current invention provides a method for determining the effect of a substance, which may be a single compound and/or compositions comprising two or more compounds, on DNA damage levels and genome maintenance in a mammal, the method comprising the steps of exposing a non-human mammal (or cells isolated there from) to the compound(s), whereby the mammal exhibits at least one mutation causing a deficiency in the mammal's interlinked NER and/or TCR/XLR/DSBR systems, or said mutation affecting genome maintenance and causing an accelerated accumulation of DNA damage and/or increased steady state levels of DNA damage, and determining the effect of substance(s), compound(s) or compositions on genome maintenance and DNA damage levels.

Preferably the effect of the (mixture of) compound(s) on the level of DNA damage and genome maintenance is determined or measured by its qualitative or quantitative effect on ageing-related parameters in the mammal. The ageing-related parameters may be studied in a mammal exhibiting normal ageing. Preferably, the ageing-related parameter is studied in an NER and/or TCR/XLR/DSBR-deficient mammal exhibiting premature, enhanced or accelerated (segmental) ageing phenotype.

The mammal exhibiting a premature and enhanced ageing phenotype will contain at least one, but may preferably contain two or more mutations or alterations in GM genes, and may be heterozygous but preferably homozygous for the mutation, or alternatively may be compound heterozygous for one, two or more GM related genes. The mutations in NER-related genes may cause mild or severe deficiencies in GM systems preferably global genome NER and/or transcription-coupled repair or a combination of the two.

The method for screening of (mixtures of) compound(s) that prevent, inhibit, delay or reduce ageing in a mammal may be studied on the living mammal *in vivo* or post mortem or utilizing explanted (parts of) organs/tissues, or cell systems derived thereof. Thus the effects of the (mixture of) compounds on DNA damage levels, genome maintenance or ageing may also be studied on parts derived from the animal tested. The parts may be collected organs, tissue biopsies, body fluids such as blood, serum or urine, faeces, isolated cells, tissue explants or cells cultured *in vitro,* or on biological material, such as isolated protein, metabolites, RNA or DNA samples from cultured cells or biopsies or body fluids and metabolites therein.

The mammal exhibiting a mutation causing a deficiency in the mammal's GM systems (as specified above) to be used for the screening method according to the current invention, preferably contains a mutation affecting the nucleotide excision repair capacity of the mammal, preferably global genome NER. More preferably the mutation causes a deficiency in the transcription-coupled repair (TCR) capacity of the mammal. Most preferably the mutation causes a deficiency in transcription-coupled repair or cross-link/double strand break repair and causes the animal to exhibit a phenotype with features of accelerated, enhanced and/or premature ageing. It is also an aspect of the invention to use mammals (and parts or cells thereof) having combinations of mutations in GM systems or mutations causing simultaneous inhibitions of two DNA repair systems, for instance mutations affecting both GG-NER and TCR capacity. Moreover, mammals may be used that comprise combinations of 2, 3 or 4 mutations, which may be homozygous, heterozygous or compound heterozygous mutant alleles of GM related genes, that affect the same or different GM systems. Most preferably, the combination of mutations causes an enhanced, premature or accelerated ageing phenotype in the mammal.

The mutations affecting the GM maintenance system and more specifically the DNA damage repair capacity in the mammal used for screening compounds that inhibit, delay or prevent accumulation of DNA damage and/or ageing symptoms, are preferably selected from the group of genes encoding structural proteins or enzymes involved in the NER process as well as ICL and DSBR and other relevant GM pathways. More preferably the mutation is in at least one or more genes of the following group of genes: *Xpa, Xpb, Xpc, Xpd, Xpf, Xpg, Csa, Csb, Ttda, HR23A, HR23B, Ercc1, Ku70, Ku80* and *DNA-PKcs.*

Mutations in genes involved in NER, TCR, XLR, DSBR or DT&S, combinations thereof or GM may comprise substitutions, deletions, inversions, insertions, temperature-sensitive alleles, splicing alleles, dominant negative alleles, over- or underexpressing alleles or insertion of stop codons (truncating alleles). The mutations may be null alleles, or subtle mutations that only partly affect the function of the gene-product or they may be dominant negative alleles that interfere or block the function of the wild-type protein also present in a cell. RNA interference (RNAi) strategies, including use of naturally occurring micro-RNA's, may also be used to inactivate systemically, locally or partially genes involved in GM systems. In yet other embodiments, combinations of mutations and genetic backgrounds may be used, for instance the use of conditional mutants, compound heterozygous animals or chimaeric animals consisting of different cell lineages wherein at least one cell lineage is deficient or altered and/or mutated in a GM system, may be advantageously used in the method for screening according to the current invention.

Preferred combinations of NER and or TCR mutations for use in the current invention are mutations inactivating *Xpa* and *Xpd,* wherein *Xpd* alleles can be homozygous for XP, XPCS, TTD, TTD-XP or COFS (cerebro-ocolo-facio-skeletal syndrome) causing alleles, or compound heterozygotes for these alleles as well as different mutants in the *Ercc1*/*Xpf* NER/XLR/DSBR genes.

Other preferred combinations are inactivating mutations *Xpa* and *Xpb, Xpa* and *Csb*, *Xpc* and *Csb*, *Xpa* and *Csa*, *Xpc* and *Csa*, *Xpb* and *Xpd*. Each of these preferred combinations of mutations in NER and/or TCR genes displays a different phenotype, comprising different aspects of ageing and characteristic for segmental ageing, or ageing-related pathologies with a different time of onset and/or severity, and may be used to screen for compounds affecting these conditions or disorders. Particularly preferred are those mutations and combinations of mutations that yield dramatically accelerated premature ageing phenotypes are present and may be scored *in utero,* at or around birth, or 1, 2 or 3 months after birth of the animal. In view of the multifunctional nature of the proteins involved, their simultaneous engagement in multiple pathways and the tight links with other GM systems it is important to emphasize that the scope of the invention is not limited to the above combinations.

The use of mammalian (preferably mouse) mutants with one and preferably two or more defects in genome maintenance systems, frequently (in most cases where a combination of two mutations was studied by the inventors so far) exhibit at least some premature ageing features in less than 3 months after birth. These models are most suited for the screening of compounds that influence the rate of ageing, for stem cell and organ/tissue transplantation purposes and for delineating RNA, protein and metabolite biomarkers of aging.

The inactivating mutations may be any mutation interfering with expression of a functional protein, such as, but not limited to introduction of partial or full deletions, insertions, frame-shifts and stop-codons. The introduction of mutation inhibiting correct expression or translation of a functional protein are well known in the art, for instance in (Ausubel et al., Current Protocols in Molecular Biology, Wiley Interscience, 2004). Preferably the mutations are introduced by homologous recombination techniques known in the art.

It is also an aspect of the invention to use conditional mutant animals for the method of screening, wherein (one or more of) the genetic alteration(s) is (are) limited to a specific tissue or organ or in which the defect may be introduced in a later developmental stage of the mammal, either systemically or in a tissue restricted fashion. Conditional mutants may for instance be generated with the Cre/Lox or FLP/FRT systems known in the art (Example 5), and may comprise introduction of mutations of NER-, TCR- or GM-involved genes in a tissue-specific manner, depending on where the recombinase is expressed, locally (Example 6) or systemically for instance using the Estrogen Receptor fusion / tamoxifen system, in which Cre (or other) recombinase is constitutively expressed, but only can be imported in the nucleus (and thus only can excise or otherwise inactivate the conditional allele) by treatment with this estrogen analog. Alternatively, cDNA expressed from a TetOn or TetOff promoter (in a knockout background for that same gene) that allows transcription in the presence/absence of doxycycline can be used. Tissue-specific transgenic animals may be used that overexpress mutated GM alleles restricted to specific tissues, or express for instance dominant negative alleles or inactivating RNAi molecules (knock down technology), restricted to for example a specific organ or tissue, preferably but not limited to the liver, skin, brain, retina or the lymphoid compartment.

Particularly preferred is the use of mammals (and parts or cells thereof) exhibiting a mutation in the *Xpb, Xpd* or *Ttda* genes, wherein the mutation is identical or closely mimicking a Trichothiodystrophy causing allele in humans. A non-limiting list of TTD-causing mutations in humans is provided in this application, table 1.Of the TTD-causing mutations, particularly preferred are those mutations or combinations of mutations causing a premature, enhanced and/or accelerated ageing phenotype, such as, but not limited to $Xpd^{R722W/R722W}$. Examples of other preferred TTD-associated mutations in the human *XPD* gene and causing enhanced/accelerated ageing phenotype are: G47R, R112H, D234N, C259Y, S541R, Y542C, R601L, R658C, R658H, D673G, R683W, R683Q, G713R, A725P, Q726 ter, K751Q (Cleaver et al., (1999) Human Mutation 14:9-22; Itin et al., 2001, J Am Acad Dermatol 44:891-920). Examples of TTD-associated mutations in the human XPB gene include T119P (Itin et al., 2001, J Am Acad Dermatol 44:891-920). Those in the human *TTDA* gene, M1T, L21P, R57ter, have been determined by the team of the applicant (Giglia-Mari G et al., Nat Genet. 2004 Jul;36(7):714-9.).

In another preferred embodiment of the invention, mammals exhibiting a mutation in the *Csa, Csb, Xpb, Xpd* and *Xpg* gene are used, wherein the mutation is identical or closely mimicking a human allele causing Cockayne Syndrome (CS) or combined Cockayne Syndrome and Xeroderma Pigmentosum (XPCS) or a recently discovered related, very severe condition called Cerebro-oculo-facio-skeletal syndrome (COFS). A non-limiting list of XP, CS, XPCS and COFS causing mutations in humans is provided in this application, table I. Of the CS-causing NER mutations, particularly preferred are those mutations or combination of mutations causing a premature, enhanced and/or accelerated ageing phenotype, such as, but not limited to: $Csa^{null/null}$, $Csb^{G744ter/G744ter}$ (mimicking CS1AN allele a). Other preferred Cockayne Syndrome associated mutations in the human *Csb* gene are: Q184ter (termination of translation-mutations; introduction of a stopcodon and/or frameshift mutation), R453ter, W517ter, R670W, R735ter, W851R, Q854ter, R947ter, P1042L, P1095R, R1213G. A preferred mutation in the human *Csa* gene for embodiments of the current invention is: Y322ter. Of the XPCS causing mutations, particularly preferred are those mutations causing a premature, enhanced and/or accelerated ageing phenotype, such as, but not limited to: $Xpd^{G602D/G602D}$, $Xpb^{FS740/FS740}$. Other preferred XPCS associated mutations for embodiments of the current invention are (i) for the human *Xpb* gene: F99S; (ii) for the human *Xpd* gene: G675R, 669fs708ter (iii) for the human *Xpg* gene: R263ter, 659ter (Cleaver et al., (1999) Human Mutation 14:9-22, and www.xpmutations.org).

In a most preferred embodiment of the invention, mammals exhibiting a TTD, CS, COFS and/or XPCS causing mutation in aforementioned genes also contain a mutation in the *Xpa* gene or other gene(s) affecting global genome NER. Particularly preferred are those combinations of mutations causing an increase in the severity and/or decrease in the age of onset of the premature, enhanced and/or accelerated ageing phenotype as observed in CS, XPCS, COFS or TTD mouse models, such as, but not limited to: $Csa^{null/null}/Xpa^{null/null}$, $Csa^{null/null}/Xpc^{null/null}$, $Csb^{G744ter/G744ter}/Xpa^{null/null}$, $Csb^{G744ter/G744ter}/Xpc^{null/null}$, $Xpd^{G602D/G602D}/Xpa^{null/null}$, $Xpd^{R722w/R722W}/Xpa^{null/null}$, $Xpd^{G602D/R722W}/Xpa^{null/null}$, or any other mutation in these GM genes that yield the phenotype specified above. This includes also single and multiple mutants in which one of the genes is made conditional as described above for tissue or developmental stage specific induction of ageing phenotypes (example 7 demonstrates a brain specific $Csb^{G744ter/G744ter}/Xpa^{conditional/null}$ mutant).

The mammal exhibiting a mutation affecting NER or other DNA repair pathways and genome maintenance systems which is preferably also affecting ageing of the mammal, and that is used in the method of screening for compounds according to the current invention, is preferably a rodent, more preferably selected from the group consisting of mice, rats, rabbits, guinea pigs, and is most preferably a mouse.

The method for screening of compounds according to the current invention, using mammals exhibiting a defect in the NER, TCR and other DNA repair and GM systems causing an accelerated accumulation of DNA damage and preferably a premature, enhanced and accelerated ageing phenotype, may be used to identify compounds and compositions that are capable of inhibiting, preventing or delaying said phenotype. The effect of compound(s) to be screened may be determined qualitatively and quantitatively by a number of phenotypic readouts in the mammal *in vivo*, or *in vitro*. Phenotypic readouts as herein defined may be any ageing-related quantitative or qualitative parameter identifying an ageing-related condition or disorder. Phenotypic readouts of the method for screening according to the invention may be performed on the animal itself, such as its behaviour and/or performance in tests. Other phenotypic readouts may be performed in or on its organs, tissue biopsies, cells, or on protein, DNA or RNA samples derived from the mammal or its *in vitro* cultured tissue explants, cells or cell-free extracts, and subsequently used for testing or analysis. Preferred readouts on the mammals exposed to compounds or compositions to be screened for their effect on DNA damage levels and/or ageing-related symptoms are parameters such as, but not limited to, life span, survival of perinatal stress (as illustrated in example 4), juvenile death, kyphosis, body weight, fat percentage (as determined by the fatty tissue vs. total body weight ratio), cachexia, hair loss, greying, neuronal and sensory dysfunction (loss of sight, hearing, smell, learning and memory capabilities), tremors, seizures, ataxia, sexual behaviour, fertility, muscle function, (limb-) coordination, heart function, hormonal-, immunological- or haematological-parameters, telomere shortening, osteosclerosis, retinal degeneration, photoreceptor cell loss, liver function, kidney function, thymic involution, Purkinje-cell loss, anemia, immune dysfunction (including autoimmune disease), cardiovascular dysfunction, diabetes, gene expression patterns, RNA expression levels, protein expression levels, metabolite levels and hormone levels. The phenotypic readouts in the method of screening according to the invention may be scored as statistically significant differences (at p values < 0.05, 0.02, 0.01 or 0.001) between individual or groups of mutant and comparable wild type mice that do not exhibit the mutation or mutations in a GM maintenance system. For the scoring of parameters in the above mentioned phenotypic readouts, methods known in the art, which will be obvious to the skilled person, may be used.

On the organ or tissue level, preferred ageing-related parameters to be used are osteoporosis (as illustrated in example 8), retinal degeneration and photoreceptor loss (as demonstrated in the example 3), lymphoid depletion (in spleen or thymus), thymic involution, loss of hypodermal fat, renal tubular dilation, lipofuscin deposition in the liver, kidney hyaline glomerulopathy, hepatic intranuclear inclusions, skin atrophy, anemia, neoplasia's and tumors. These parameters are merely provided to illustrate and are not limiting the potential read-outs for genome maintenance and ageing in the screening method according to the current invention.

In another embodiment of the current invention, preferred readouts in the method for screening of compounds are at the cellular and molecular level, in more preferred embodiments are gene expression analysis on RNA samples (transcriptomics), protein expression analysis on protein samples (proteomics) and accumulation of DNA damage and lesion analysis on genomic DNA samples. Other preferred embodiment comprises ageing-related parameters to be determined via metabolomics, i.e. measuring the effects of compounds in the method according to the current invention on metabolites and metabolic pathways in the tested mammal. RNA, DNA and proteins samples from mammals or cultured cells, treated and untreated with compounds or compositions in the method for screening according to the current invention may be compared, inter *alia,* with mammals or cells not exhibiting the mutation causing deficiencies in NER, reference samples / standards, or with mammals or cells of relatively younger or older age, in order to assess the effect on DNA damage levels and ageing processes that the compounds have in the mammal or cells derived from it. Differences in gene expression patterns may be determined on custom made or commercially available DNA microarrays, hybridised with RNA or cDNA samples obtained from mammals used for testing (transcriptomics, further illustrated in examples 1 and 2). Differences in protein expression levels may be determined using antibodies; in immune precipitation experiments, 1D or 2D immunoblotting techniques, protein (micro-)arrays and other proteomics techniques or metabolomics techniques. The effect of compounds on genome maintenance in the method of screening according to the current invention may also be determined directly on the accumulation of DNA damage in the genomic DNA directly. Analysis DNA may comprise DNA sequencing, mutation analysis, especially detection of mutation hot-spots. DNA damage may for instance be determined by the methods of H. Poulsen to measure oxidative DNA damage (Riis, B., L. Risom, S. Loft, and H. E. Poulsen. 2002, DNA Repair 1:419-24).

More than 100 different free radical mediated modifications in DNA have been described. However, the most preferred parameter for DNA damage and genome maintenance is a single modification, the 8-hydroxylation of guanine (8oxoG), which is one of the most abundant types of oxidative DNA base damage. A methodology that utilises a sodiumiodide based DNA extraction, enzymatic digestion of DNA and analysis with liquid chromatography tandem mass spectrometry (LC-MSMS) is a preferred readout to be applied in the method for screening compounds according to the current invention. It provides high sensitivity and indications of true values of 8-oxodG in genomic DNA in response to treatment of mammals with compounds according to the method of the current invention.

Another preferred technique for rapid and efficient mutation screening and accumulation of DNA damage in the method of the current invention is the lacZ reporter mouse model as described in, Vijg J. et al., Mech Ageing Dev. 1997 Dec; 98(3):189-202 or other variants of this method. This method allows studying the mutation accumulation in the DNA of somatic cells and tissues during aging in vivo in animal models used for the method of screening of compounds according to the current invention. The model lacZ reporter mouse model harbors plasmid vectors, containing the lacZ reporter gene, preferably integrated head to tail at various chromosomal locations. Procedures have been worked out to efficiently recover the plasmids into *E. coli* host cells. A positive selection system, permitting only *E. coli* cells with a lacZ mutated plasmid to grow, allows for the accurate determination of mutation frequencies as the ratio of mutant colonies versus the total number of transformants, i.e., the total number of plasmid copies recovered. Results obtained from a life span study of plasmid carrying mice with vector clusters on chromosome 3 and 4 indicated age-related mutation accumulation in cells of the animal, for instance liver cells. The effect of compounds to be screened according to the method of the current invention can be efficiently determined using this assay on liver (or any other type of cells) of mice treated (and not treated for comparison) with compounds assayed in the method of screening according to the invention.

An even more preferred method is the use reporter genes in aforementioned premature ageing mouse models. Such reporter genes are composed of a promoter, the expression level of which has been shown to increase upon ageing and to correlate with onset and severity of ageing-related pathology, has been coupled to bioluminescent (e.g.

luciferase) or fluorescent (e.g. green fluorescent protein) reporter genes, allowing longitudinal non-invasive screening of ageing and ageing-related pathology in the living mouse as well as screening of the interfering effect of compounds on the onset and severity of ageing and ageing-related pathology. Alternatively, reporter genes may be composed of a gene encoding a protein of which the expression level is enhanced upon ageing (e.g. via enhanced expression, increased stabilization or reduced degradation), in frame fused to a bioluminescent or fluorescent protein.

The method for screening of compounds according to the current invention may be enhanced by additionally exposing the mammals to DNA damaging treatments, in order to enhance the discriminating power of the method. DNA damaging treatments may be applied by physical treatments, such as exposure to UV, X-ray or gamma radiation, or by chemical means, such as exposure to reactive oxygen species (ROS), oxidative stress and exposure to DNA damaging compounds. DNA damaging compounds that may be favourably used comprise, but are not limited to; paraquat, $H_2O_2$, DMBA, AAF, aflatoxin, Benz(o)pyrene, EMS, ENU, MMS, MNNG, $H_2O_2$, bleomycin, illudinS, Nitrogen mustard, PUVA, mitomycin C, cisplatinum and taxol.

Alternatively, the method for screening of compounds having an effect on DNA damage levels and ageing symptoms according to the current invention and as described above, may be further enhanced and made more sensitive and/or more versatile by introducing the mutations compromising NER, TCR, XLR, DSBR or other GM-related pathways, in mammals exhibiting a specific genetic background. For instance a genetic background could be used that preferably is prone to, or with increased sensitivity for, the accumulation DNA damage and/or ageing symptoms. For instance, mammals may be used that carry activated oncogenes or inactivated tumour suppressor genes, either by mutations, deletions or insertions into their genome, as for example in, but not limited to transgenic or knock out animals, naturally occurring mutants, RNAi transgenic animals with RNAi silenced (tumorsuppressor-) genes. A wide range of tumor suppressor genes and oncogenes are well known and studied in the art. Examples of genes that may be favourably used as a genetic background for the mammals to be used in the method for screening of compounds according to the current invention are, but not limited to: p53, p16, pl9arf, Ras, c-Myc, Rb, cyclinD, telomerase, viral oncogenes such as adenovirus E1A, E1B, HPV E6 or E7, SV40 large T. Alternatively, additional genetic defects in cellular detoxification or anti-oxidant defence pathways may be used to sensitize the prematurely ageing mouse models.

The method for screening compounds having an effect on genome maintenance and ageing according to the current invention, as determined by the effect of the compounds in mammals with compromised NER, TCR, XLR, DSBR or GM capability, may be carried out to identify effects of novel compounds or compositions, for instance crude extracts from natural / biological sources, such as, but not limited to microorganisms, plants or animals. The method may also be favourably used on compounds which are known to have specific properties and may therefore have an effect on DNA damage levels and ageing, such as compounds with anti-oxidant properties which may eliminate or detoxify free radicals, reactive oxygen species or N-radicals. Also compounds having an effect on cell cycle progression, metabolism, cell death or apoptosis, DNA repair, detoxification and/or liver function, cardiovascular function and/or circulation, immunological performance may be tested for their effect on genome maintenance and ageing symptoms according to the current invention. Particularly preferred compounds to be used according to the current invention to inhibit, delay or reduce ageing-related symptoms and to improve or restore genome maintenance are antioxidants and radical scavengers selected from the group comprising: β-catechin, N-acetyl-cysteine, cystein, α-tocopherol, retinol, D-mannitol, proline, N-tert-butyl-a-phenylnitrone (PBN), vitamin-C/ascorbate, uric acid/urate, albumin, bilirubin, vitamin E, ubiquinol, cartenoids (such as lycopene, carotene, astaxanthin, canthaxanthin), flavonoids, catechines, 4-nitrophenol, 4-hydroxybenzoate, phenol, tyrosine, 4-methylphenol, 4-methoxyphenol, serotonin, α-, β-, γ-, δ-tocopherol, hydroquinone, DOPA, 4-aminophenol,4-demethylaminophenol, allopurinol, deferrithiacine, phenantroline, ergothioneine.

Enzymes involved in anti-oxidant / radical scavenging activities that may be used in the methods according to the current invention are enzymatic radical scavengers (such as peroxidase, superoxide dismutase (SOD), glutathione peroxidase (GPX)), enzymes involved in reduction of antioxidants (such as: GSH reductase, glutathione-S-transferase, dehydroascorbate reductase), and cellular enzymes that aid in maintaining a reducing environment (such as for instance glucose-6-phosphate dehydrogenase) and proteins involved in sequestration of metal ions such as for instance apoferritin, transferrin, lactoferrin, ceruloplasmin and other small radical scavengers. Based on the concepts outlined in this work inborn or acquired deficiencies in any of these components may also trigger an accelerated phenotype and as such are part of this application. The genes for these proteins are relevant in terms of transgenesis (overexpression as well as reduced expression) preferably in combination with the GM mutants described above.

In another embodiment the method for screening of compounds according to the current invention may also be employed as a method for screening for ageing promoting (side-) effects of compounds, and in particular of pharmaceuticals and food products.

In addition to the screening of compounds influencing ageing and ageing-related features, the GM mouse models and cell lines, (parts of) organs and tissues derived thereof are also relevant for a number of other ageing-related processes and phenomena. This includes the use of the GM mice for analysis and influencing reperfusion damage to the genome in the context of organ transplantation, particularly for the screening of compounds that reduce the reperfusion damage, which shortens the life span of the transplanted organ or tissue. In a similar setting the GM mouse models and cell lines, (parts of) organs and tissues derived thereof are also relevant for analysis of and influencing ischemia.

The use of DNA repair and genome instability mutants exhibiting accelerated ageing phenotypes according to this invention may also be used for various other interventions, such as transplantations and in particular stem cell transplantations.

Within the same conceptual framework the invention pertains to the use of GM mouse mutants and cell lines derived thereof for the purpose to reduce the negative effect of chemo- and radiotherapy to normal tissues, or enhance the effect on the tumor in order to enlarge the therapeutic window in the treatment for cancer. For this purpose the same or different compounds and substances might be utilized as the ones listed above for assessing the effect on ageing.

Similarly, the method can also be employed for the screening of agents and compounds that influence the ageing of the skin for cosmetic purposes and cosmoceuticals.

As another related application the above described GM mouse models are also relevant for the analysis and utilization of stem cell transplantation for specific organs and tissues that exhibit accelerated ageing.

A final application in the same domain is the use of the GM mouse models and cell lines derived thereof for defining ageing-related fingerprints for gene expression, protein expression and modification and links with genetic polymorphisms (SNPs) and their use in diagnosis, prognosis and treatment of ageing-related disease.

The invention and its embodiments are further illustrated and explained by the following non-limiting examples.

Description of the Figures

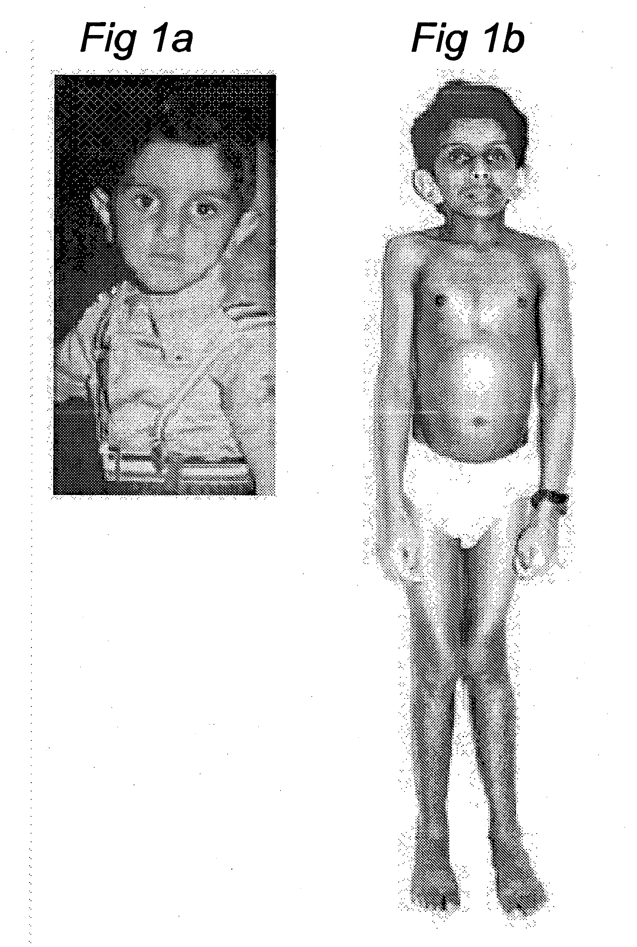

**Figure 1. The patient with a novel progeria**. (A) Photograph at three years of age. He was largely asymptomatic until the age of 10, with the exception of photosensitivity and a mild learning disability. (B) Photograph of the patient at 16 years of age, at which point he was markedly dwarfed, cachectic and had an aged, wizened appearance.

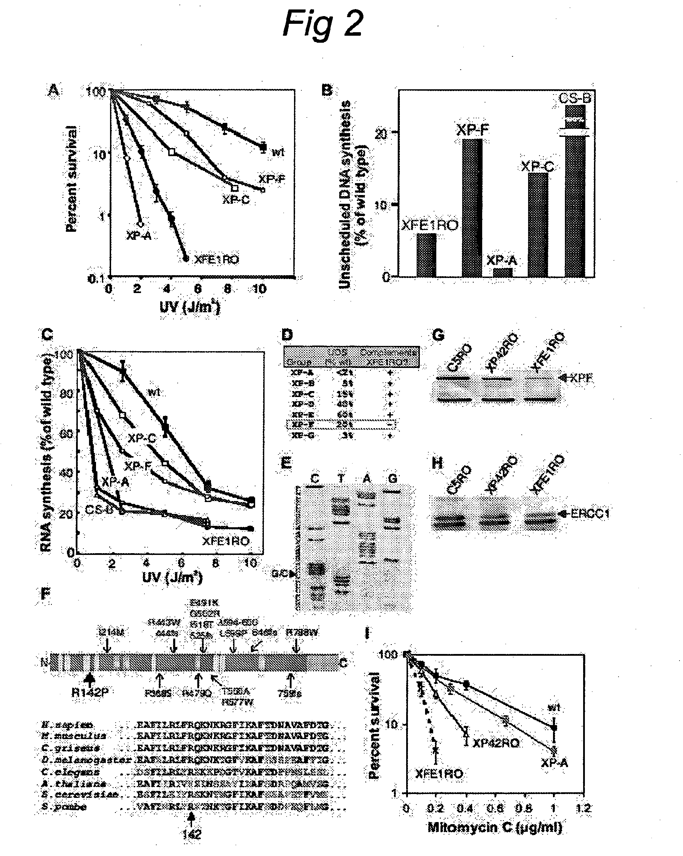

**Figure 2. Characterization of patient primary fibroblasts (XFE1RO).** (A) UV sensitivity of wt (■), XFE1RO (•) and XP patient primary fibroblasts (XP-F, O; XP-C, €; XP-A, 0). Cells were seeded sparsely on 6 cm dishes and after 48 hr exposed to increasing doses of UV-C (254 nm). One week after irradiation, the cells were fixed, stained and colonies counted by microscopy. (B) Quantitation of UDS to measure UV-induced DNA repair. Cultures of XP patient primary fibroblasts were exposed to 10 J/m$^2$ UV-C in the presence of $^3$H-thymidine. UV-induced DNA synthesis was measured as the number of radioactive grains per nucleus counted after autoradiography and plotted as a percentage of the number of grains detected in wt cells. (C) RNA synthesis recovery after UV irradiation of patient cell lines. RNA synthesis was measured by liquid scintillation counting of the amount of $^3$H-deoxyuridine incorporated into the cells and plotted as the percent of the label incorporated in unirradiated cells. (D) Complementation analysis. XFE1RO fibroblasts are fused with a genetically defined panel of XP patient cell lines (complementation group A-G) using inactivated Sendai virus. After fusion, the cells are irradiated with UV (20 J/m$^2$) and incubated in the presence of $^3$H-deoxythymidine and UDS was measured as described in Fig. 2B. Positive complementation was scored when the level of UDS was restored from the percent indicated in the chart to 100% or wt levels. (E) Sequence analysis of XFE1RO *XPF* cDNA demonstrating a point mutation in the coding sequence of *XPF*. (F) Schematic diagram of the XPF protein. Conserved domains are highlighted in color: yellow indicates non-functional helicase motifs, grey represents leucine rich domains; orange indicates a putative nuclear localization sequence; light blue indicates the domain required for ERCC1 protein interaction. The central dark blue region is poorly conserved. Coding changes identified in XP patients are indicated with arrows. Δ indicates deletion; fs indicates a frameshift resulting in protein truncation. Mutations identified in blue were not confirmed to be linked to human disease. The position of the R$^{142}$ change in XFE1RO is indicated in bold type. The sequence of the segment of *hXPF* protein containing residue 142 is aligned with the mouse and hamster (Ercc4) homologs, as well as homologs from *Drosophila melanogaster* (mei-9), *Arabidopsis thaliana, Saccharomyces cerevisiae* (RAD1) and *Schizosaccharomyces pombe* (RAD16). Conserved amino acids are indicated in black, conservative changes in green and non-conserved residues in red. The vertical arrow indicates the site of the patient's mutation at a highly conserved arginine residue. (G) Immunodetection of XPF in whole cell extracts (WCEs) derived from patient fibroblasts (normal C5RO, mild XP patient XP42RO and patient XFE1RO).Cross-reacting bands demonstrate equal protein loading (H) Immunodetection of ERCC1 in the same WCEs. (I) Clonogenic survival assay after treatment of immortalized fibroblasts (wt •; XP-A ■; mild XP-F Δ and XPFE1RO X) with the crosslinking agent mitomycin C. Fibroblasts immortalized with human telomerase were used for this assay, with the exception of the XP-A patient cell line which were early passage primary fibroblasts.

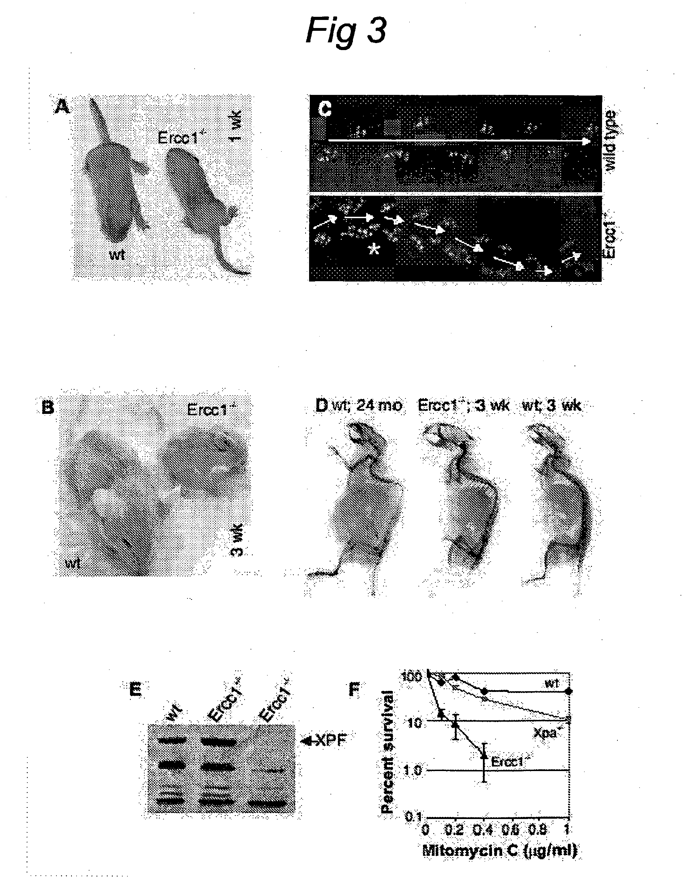

**Figure 3. Progeroid characteristics of *Ercc1$^{-/-}$* mice**. (A) *Ercc1$^{-/-}$* mouse compared to a wt littermate at one week of age. The knockout is well-developed and only fractionally smaller than its sibling. (B) *Ercc1$^{-/-}$* mouse compared to a wt littermate at three weeks of age. The knock-out mouse is dwarfed and cachectic. (C) Footprint analysis of 3 week old mice. Forepaws were painted with pink, hind paws with green. The animals were released into a narrow tunnel with a dark refuge at the end after three practice trials. The white arrows are placed equidistance between the left and right paw prints and indicate the trajectory of the gait. The top panel illustrates the pattern of a wt mouse. The bottom panel illustrates the gait of an *Ercc1*[-/-] mouse. A yellow asterisk indicates steps in which the hind paw prints are not superimposed upon the forepaw prints in the *Ercc1*[-/-] mouse, a diagnostic criteria of ataxia (53). (D) Radiographs of mice. On the left is an X-ray of a naturally aged wt mouse demonstrating severe kyphosis. In the center is a 3 wk old *Ercc1*[-/-] mouse also with kyphosis. On the right is an age-matched wt mouse demonstrating the normal curvature of the spine at 3 wk of age. (E) Immunoblot of protein extracts derived from liver of wt, *Ercc1*[-/-] and heterozygote mice. The blot was probed with an antibody that recognizes mouse XPF protein. (F) Clonogenic survival assay comparing response of wt ♦, *Ercc1*[-/-] ▲ and NER-deficient *Xpa*[-/-] ■ primary MEFs to the crosslinking agent MMC.

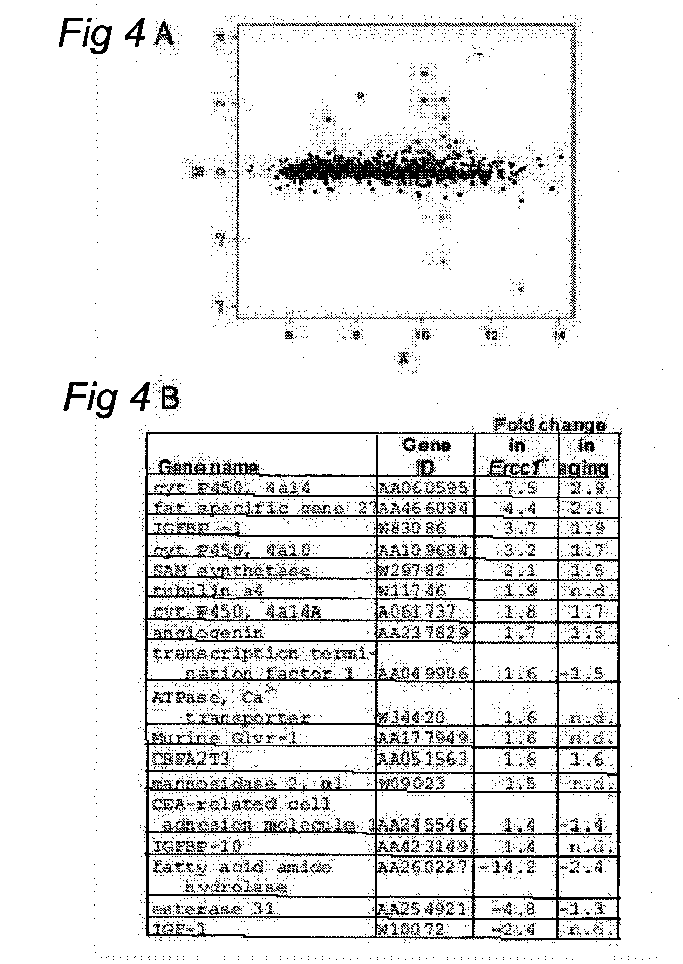

**Figure 4. Overlapping differential gene expression profiles between ERCC1-deficient mouse liver and liver from normally aged mice.** (A) Scatter plot representing each of the cDNA included in the microarray chip with an individual circle. Plotted is the ratio between *Ercc1*[-/-] mouse liver mRNA and that isolated from wt littermate controls. "M" represents the log2(red/green) ratio and "A" is the total intensity value calculated as log2(red x green). Differentially expressed genes were calculated using Significance Analysis of Microarrays (SAM) and are represented as over-expressed (red spots) and under-expressed (green spots). (B) Summary of the most significantly over- or under-expressed genes in *Ercc1*[-/-] mouse liver as identified by SAM, listed in rank order of expression change, derived from either the experiment using liver RNA pooled from multiple animals in an FVB/n;C57B1/6 genetic background. Stringency was set such that the list includes at most one false positive data point. The data are compared to the fold difference in expression caused by natural aging (last column), n.d. indicates gene for which no significant difference in expression levels was detected in the comparison between old and young wt mouse liver.

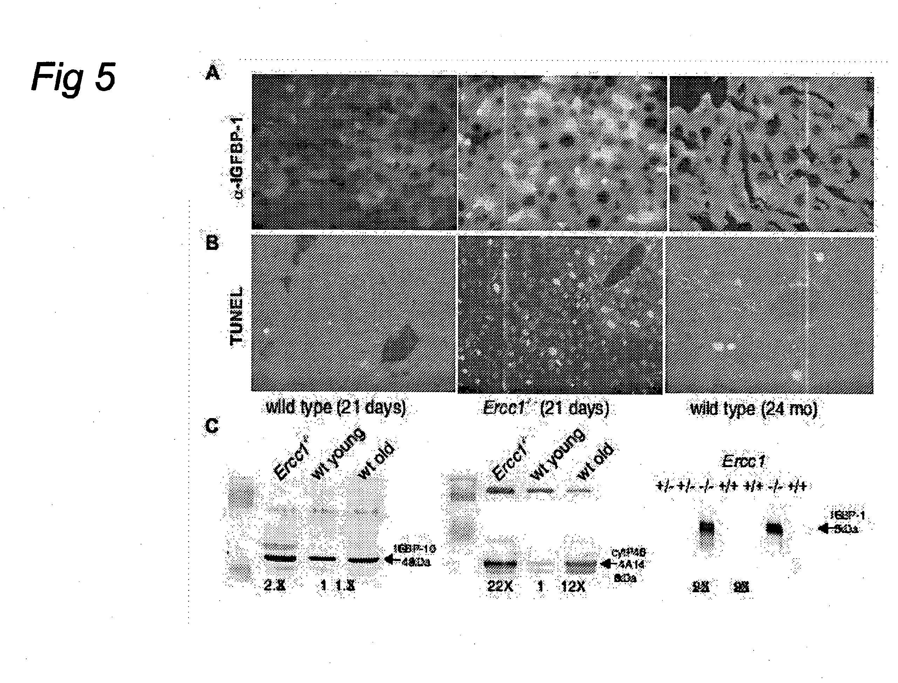

**Figure 5. Confirmation of microarray data by immunodetection.** (A) Detection of IGFBP-1 in paraffin embedded mouse liver from a prematurely aged but not moribund *Ercc1*[-/-] mouse and a wt littermate compared to the liver of a 2 yr old mouse liver. (B) TUNEL assay in fixed liver sections of the same mice. Apoptotic nuclei containing fragmented DNA were detected by fluorescence microscopy. (C) Immunoblot of protein extracts derived from liver isolated from an ERCC1-deficient mouse, a wt littermate or an aged wt mouse. Genotypes are indicated above each lane, and the identity and molecular mass of the detected protein to the right. The fold induction compared to protein levels in young wt mouse liver is indicated below each lane

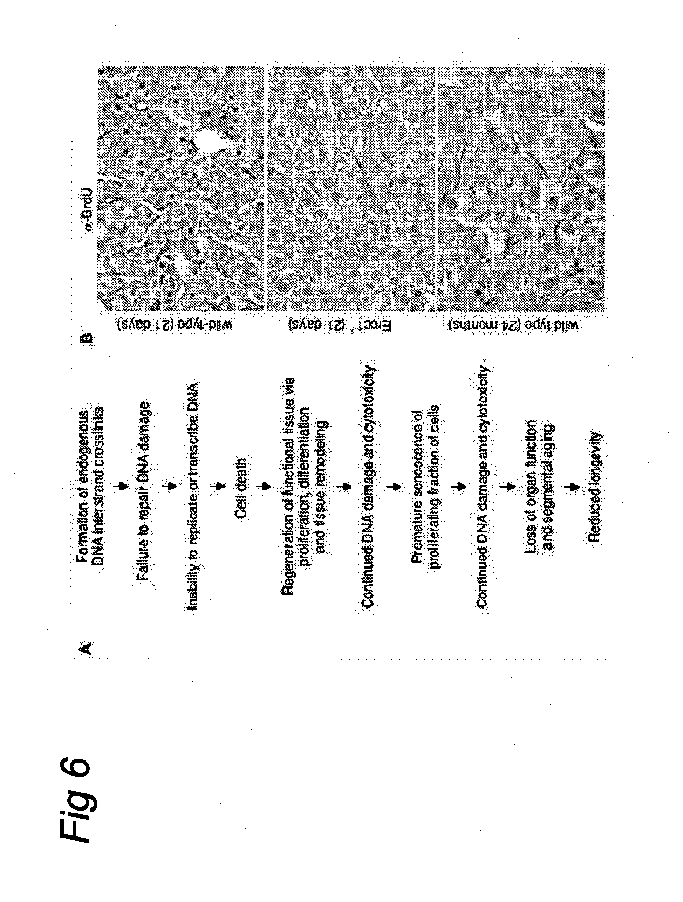

**Figure 6. (A) Model for the mechanism of premature aging as the consequence of a DNA ICL** repair defect. DNA ICLs are formed endogenously as a result of normal metabolism, likely secondary to lipid peroxidation. In the absence of crosslink repair, these damages accumulate. Due to their chemistry, these lesions are an absolute block to DNA metabolism such as replication and transcription. Failure to replicate DNA or transcribe essential genes results in cell death. If the tissue has regenerative capacity, such as the liver, then cell death is compensated for by a subsequent increase proliferation of mitotically active cells. If the cycle of damage, cytotoxicity and proliferation persist, or if proliferative cells are a direct target of DNA damage, then the total proliferative capacity of a tissue becomes reduced over time. This results in reduced function of the tissue and can contribute to segmental aging features and ultimately premature death. (B) Detection of proliferating cells using α-BrdU. The synthetic nucleotide was injected into mice 30 min prior to sacrificing and incorporated into the DNA of replicating cells. Nuclei containing BrdU were detected by immunohistochemistry and horse radish peroxidase activity as described.

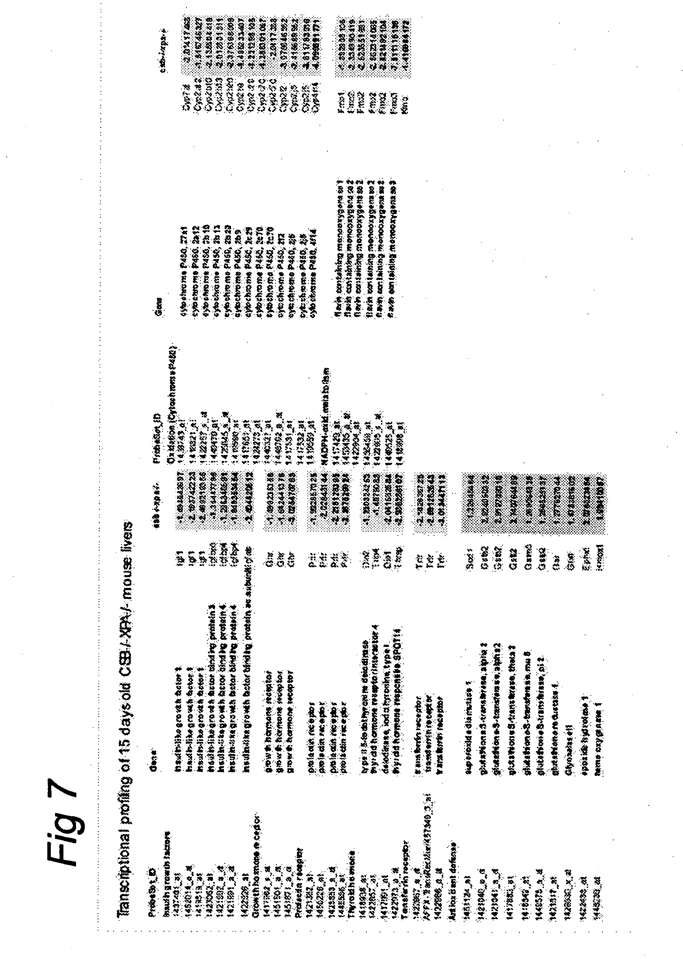

**Figure 7.** Transcriptional profiling of 15 day old *Csb*[G744ter/G744ter]/*Xpd*[null/null] mouse livers.

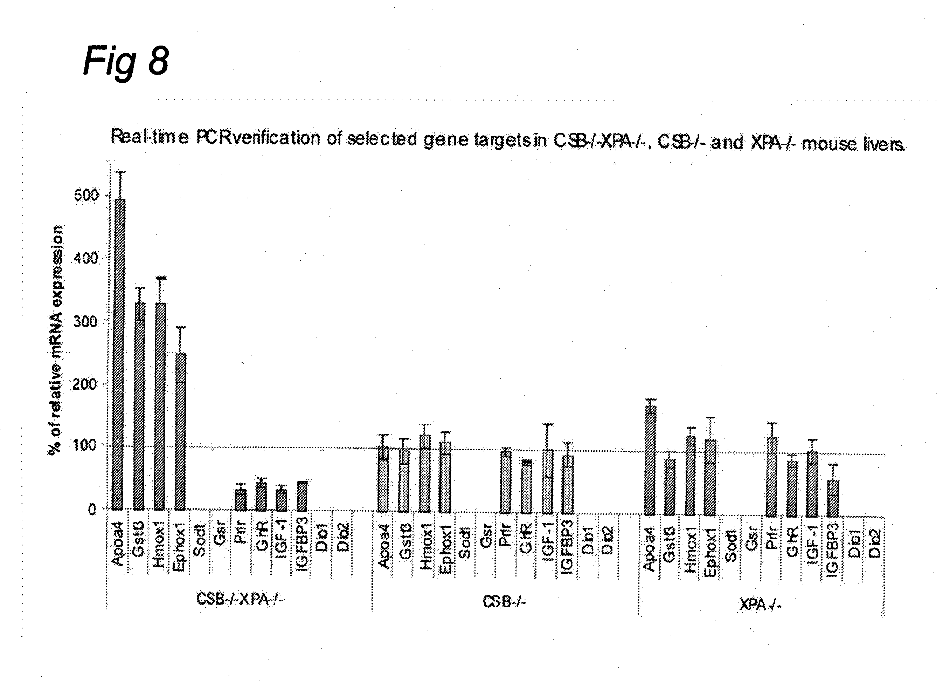

**Figure 8**. Real-time PCR verification of selected gene targets in *Csb*[G744ter/G744ter]/*Xpa*[null/null], *Csb*[G744ter/G744ter], and *Xpa*[null/null] mouse livers.

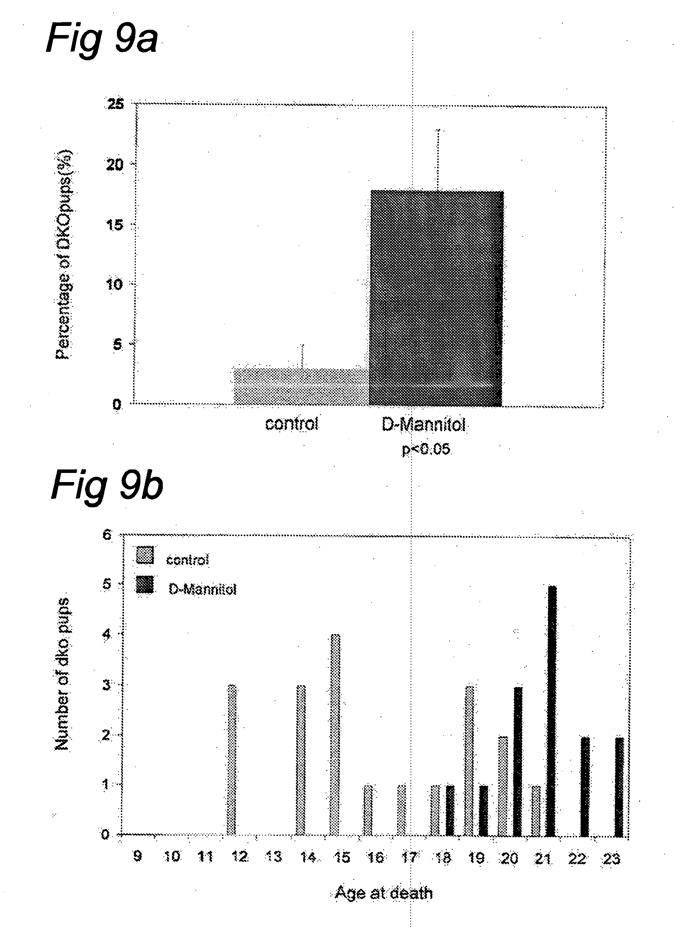

**Figure 9A.** Effect of mannitol on percentage *of Csb*[G744ter/G744ter]/*Xpa*[null/null] pups born. **B**. Effect of mannitol on survival in weeks after birth for *Csb*[G744ter/G744ter]/*Xpa*[null/null] pups.

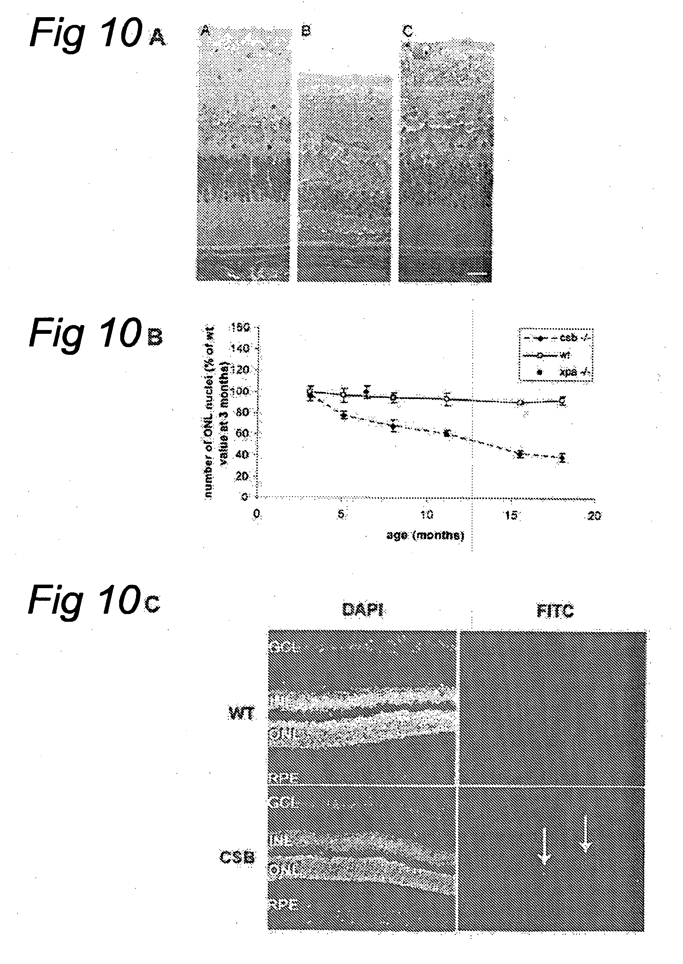

**Figure 10. Spontaneous changes in the retina of *Csb*[-/-] mice with age.** A) Micrographs were taken in the central part of the retina of 3-month-old *Csb* -/- (panel a), 18-month-old *Csb* -/- (panel b) and 18-month- old *Csb* +/+ mice (panel c). Note the loss of ONL nuclei and distortion of the outer segment layer in 18-month-old *Csb* -/mice. Bar 25 μm. (B) Counts of ONL nuclei (± standard deviation) demonstrate a loss of photoreceptor cells in *Csb* -/- mice with age. (ANOVA, followed by T-test; P<0.05). *Xpa* -/- mice at 6.5 months do not differ from wild type in photoreceptor number. (C) Paraffin sections of 3 month-old wild type and Csb-deficient mice stained with a nuclear stain (DAPI) on the left and stained with FITC for TUNEL-positive cells on the right. Arrows point at TUNEL-positive nuclei in the ONL of the *Csb* -/- mouse retina.

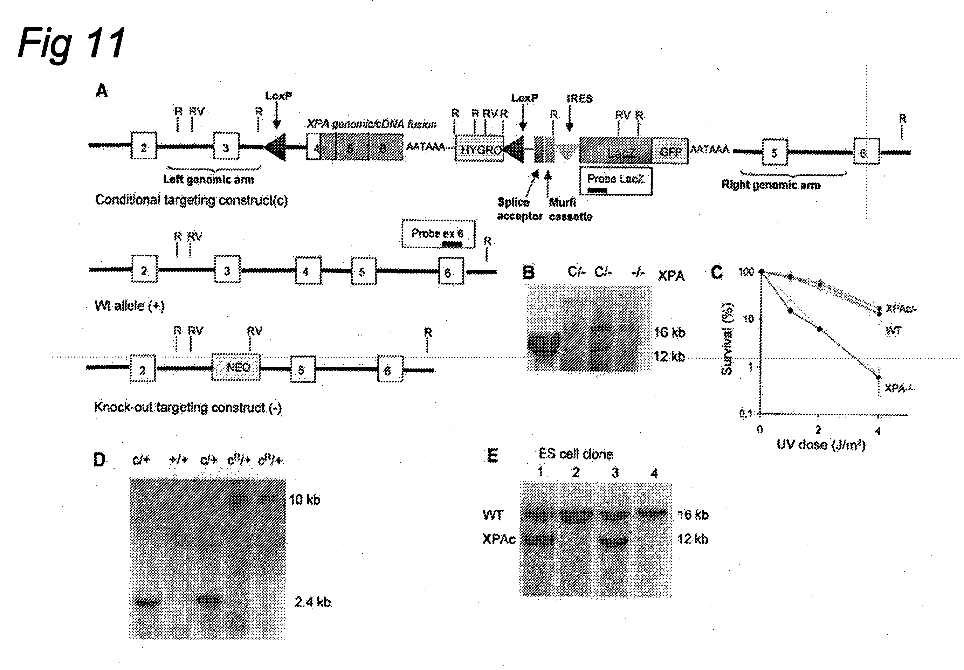

**Figure 11. Targeting of the mouse XPA conditional construct.** A) Schematic representation of part of the genomic structure of the mouse XPA gene. The coding parts of XPA exons 2 to 6 are indicated with open boxes. In the knock-out targeting construct, exon 3 and 4 were replaced by a Neomycin (NEO) selectable marker casette. In the conditional targeting construct, mouse XPA cDNA exon 4-6 was fused to the genomic exon 4. Next to the cDNA, a Hygromycin (HYGRO) selectable marker casette was placed for selection of the construct in ES cells. Between exon 3 and 4 and behind the HYGRO casette LoxP sequences were placed to allow recombination by Cre recombinase. Behind the second LoxP sequence, a LacZ-GFP fusion marker, accompanied by a splice acceptor, multiple reading frame insertion (Murfi) casette and Internal ribosomal entry site (IRES) were added. In this way, after recombination by Cre recombinase, the LacZGFP marker will be expressed, allowing visualization both in vivo and in vitro. Important restriction sites are depicted; R= EcoRI, RV= EcoRV. Two probes are indicated; probe exon 6 and probe LacZ, which were used for genotyping. B) Southern blot analysis of $Xpa^{-/-}$ ES cells targeted with the conditional construct. Digestion was done with EcoRI and hybridization was done with probe exon 6. This probe hybridized external to the targeting construct with a 16 kb knock-out EcoRI fragment and a 12 kb mutant EcoRI fragment. C) Survival of wt, $Xpa^{-/-}$ and $Xpa^{cl-}$ ES cells after UV damage. Cells were exposed to increasing doses of UV-C (254 nm). After 7 days, the number of proliferating cells was estimated from the amount of clone formation. D) Southern blot analysis of targeted ES cell clones. Digestion was done with EcoRI and hybridization was done with probe exon 6. This probe hybridized external to the targeting construct with a 16 kb WT EcoRI fragment and a 12 kb mutant EcoRI fragment. E) Southern blot analysis of c/+ ES cell clones transfected with Cre-recombinase. Digestion was done with EcoRV and hybridization was done with probe LacZ. This probe hybridized with a 2.4 kb conditional EcoRV fragment before Cre recombination and a 10 kb conditional EcoRI fragment after Cre recombination.

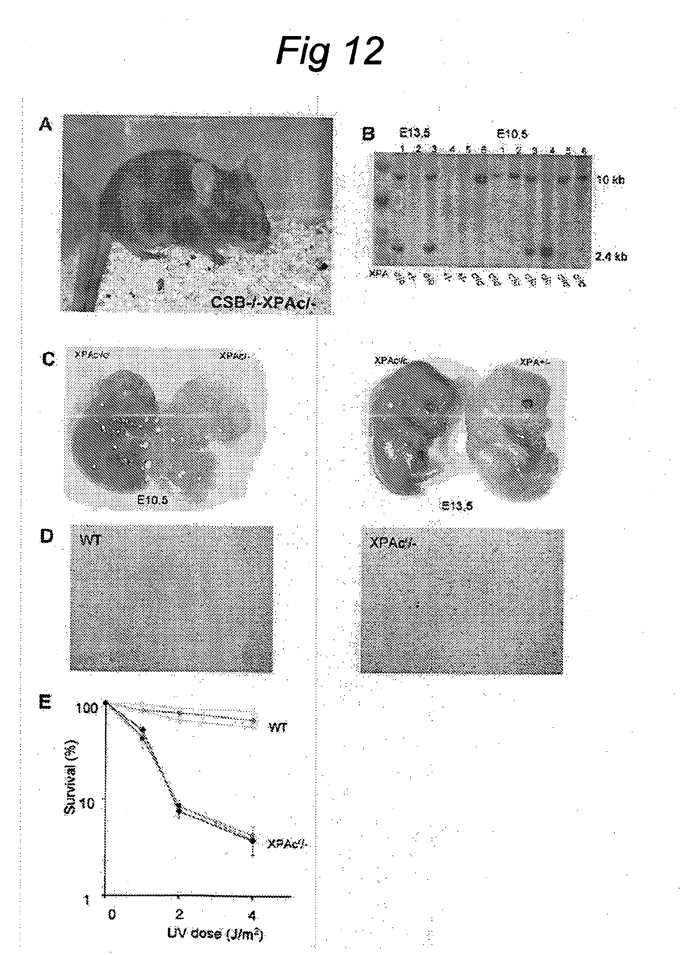

**Figure 12. Functionality and Cre recombination of the XPA conditional allele. A)** Picture of an 8 week old $Xpa^{cl}$-$Csb^{m/m}$ mouse, showing rescue of the $Xpa^{-/-}$-$Csb^{m/m}$ premature aging phenotype by introduction of an XPA conditional allele. B) Southern blot analysis of $Xpa^{+/-}$, $Xpa^{cl-}$, $Xpa^{clcr}$ and $Xpa^{crlcr}$ embryo DNA, day 10.5 and 13.5. Digestion was done with EcoRV and hybridization was done with probe LacZ. This probe hybridized with a 2.4 kb conditional EcoRV fragment before Cre recombination and a 10 kb conditional EcoRI fragment after Cre recombination. The XPA conditional allele was recombined out by CagCre, a non-inducible, ubiquitously expressed Cre-recombinase. C) LacZ staining of $Xpa^{+/-}$, $Xpa^{cl-}$, $Xpa^{clcr}$ and $Xpa^{crlcr}$ embryos, day 10.5 and 13.5. D) LacZ staining of wt and $Xpa^{crl-}$ MEFs. E) Survival of wt and $Xpa^{crl-}$ MEFs after UV damage. Cells were exposed to increasing doses of UV-C (254 nm). After 7 days, the number of proliferating cells was estimated from the amount of clone formation.

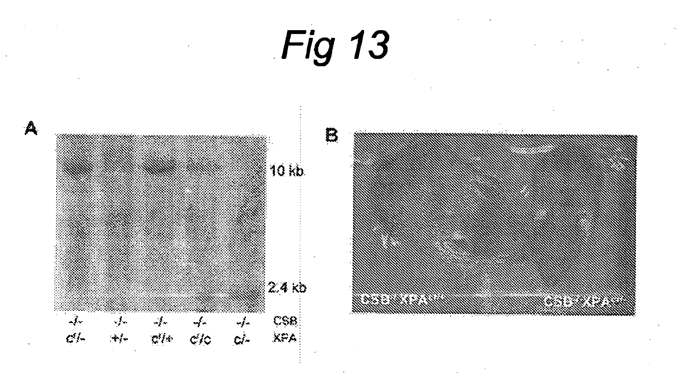

**Figure 13. Cre recombination of the XPA conditional allele in the mouse A)** Southern blot analysis of $Csb^{m/m}Xpa^{+/-}$, $Csb^{m/m}Xpa^{cl-}$, $Csb^{m/m}Xpa^{crl-}$, $Csb^{m/m}Xpa^{crl+}$ and $Csb^{m/m}Xpa^{clcr}$ mouse tail DNA. Digestion was done with EcoRV and hybridization was done with probe LacZ. This probe hybridized with a 2.4 kb conditional EcoRV fragment before Cre recombination and a 10 kb conditional EcoRI fragment after Cre recombination. The XPA conditional allele was recombined out by CagCre, a non-inducible, ubiquitously expressed Cre-recombinase, with expression upon conception. B) Picture of a $Csb^{m/m}Xpa^{crl-}$ and a $Csb^{m/m}Xpa^{crl+}$ littermate control, age 16 days. The phenotype of this $Csb^{m/m}Xpa^{crl-}$ mouse is the same as the previously published $Csb^{m/m}Xpa^{-/-}$ mouse, proving that total recombination of the XPA conditional allele gives rise to a functional knock-out.

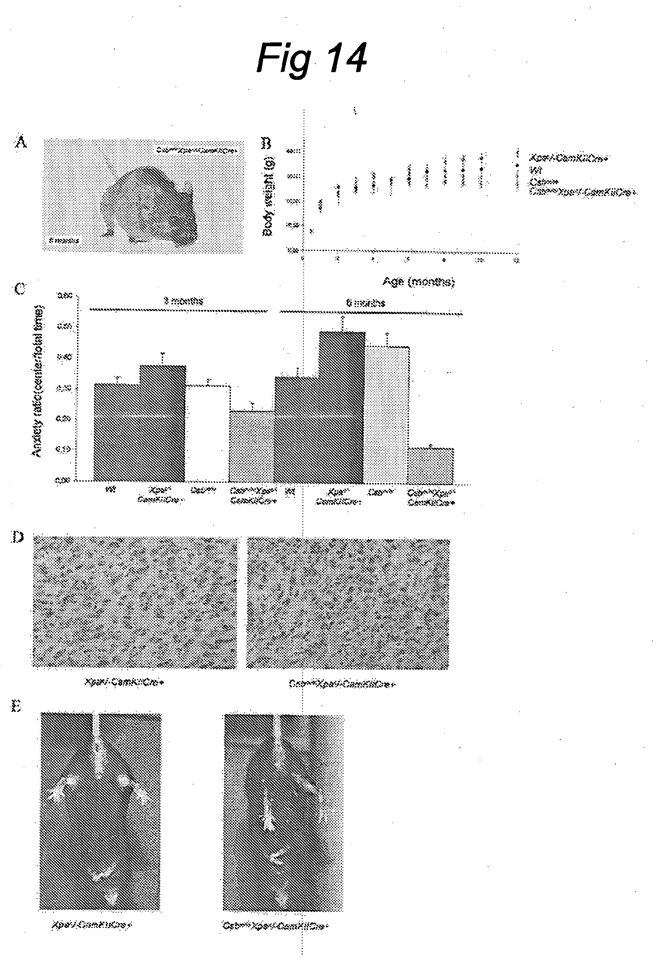

**Figure 14. Brain specific Cre recombination of the XPA conditional allele in a CSB deficient background A)** Picture of a $Csb^{m/m}Xpa^{cl}$-$CamkII\alpha$-$Cre+$ animal at the age of 6 months. B) Body weight curve of $Wt$, $Csb^{m/m}$, $Xpa^{cl}$-$CamKII\alpha$-$Cre+$ and $Csb^{m/m}Xpa^{cl}$-$CamKII\alpha$-$Cre+$. Beyond 8 months the weight of $Csb^{m/m}Xpa^{cl}$-$CamKII\alpha$-$Cre+$ animals starts to differ substantially from their control littermates, including $Csb^{m/m}$ animals. C) Bar graphs depicting the anxiety ratio (AR), calculated as center distance divided by total distance, of $Wt$, $Csb^{m/m}$, $Xpa^{cl}$-$CamKII\alpha$-$Cre+$ and $Csb^{m/m}Xpa^{cl}$-$CamKII\alpha$-$Cre+$ animals at the age of 3 and 6 months. A low AR is indicative of anxiety-like behaviour. At 3 months the AR of and $Csb^{m/m}Xpa^{cl}$-$CamKII\alpha$-$Cre+$ animals is significantly lower than that of the control groups (p<0.05), which becomes even more significant at the age of 6 months (p<0.001). D) Pictures of specific LacZ staining in the brains of $Xpa^{cl}$-$CamKII\alpha$-$Cre+$ and $Csb^{m/m}Xpa^{cl}$-$CamKII\alpha$-$Cre+$ animals. Staining was only found in the Lateral Septum that is connected to hippocampus and related to anxiety-like behaviour.

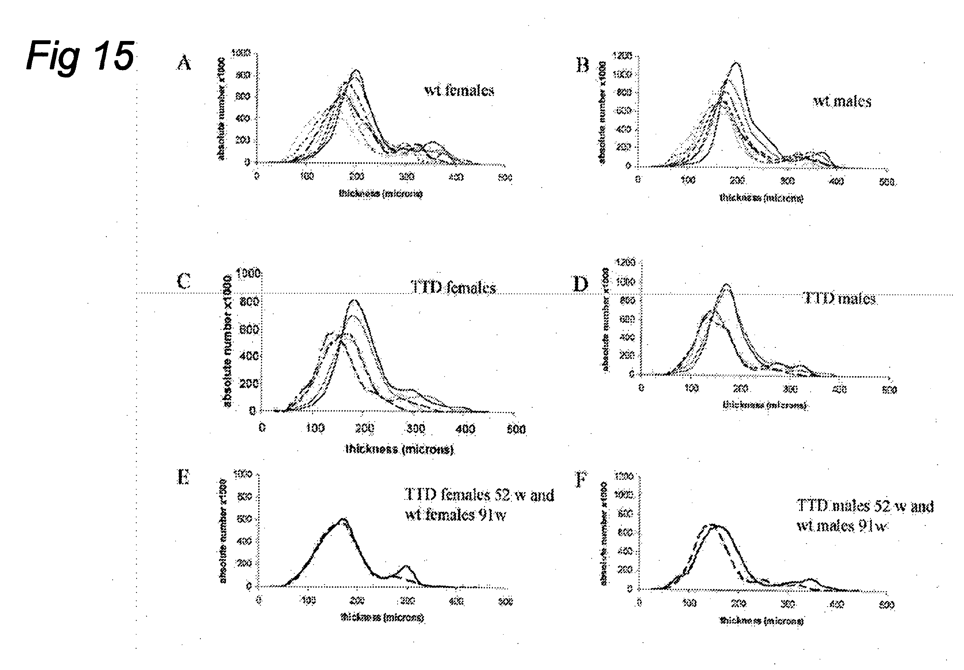

**Figure 15 Thickness distribution in ageing wild type females** (A) and males (B), ageing TTD females (C) and males (D) and 52-week-old TTD mice compared to 91-week-old wild type females (E) and males (F)

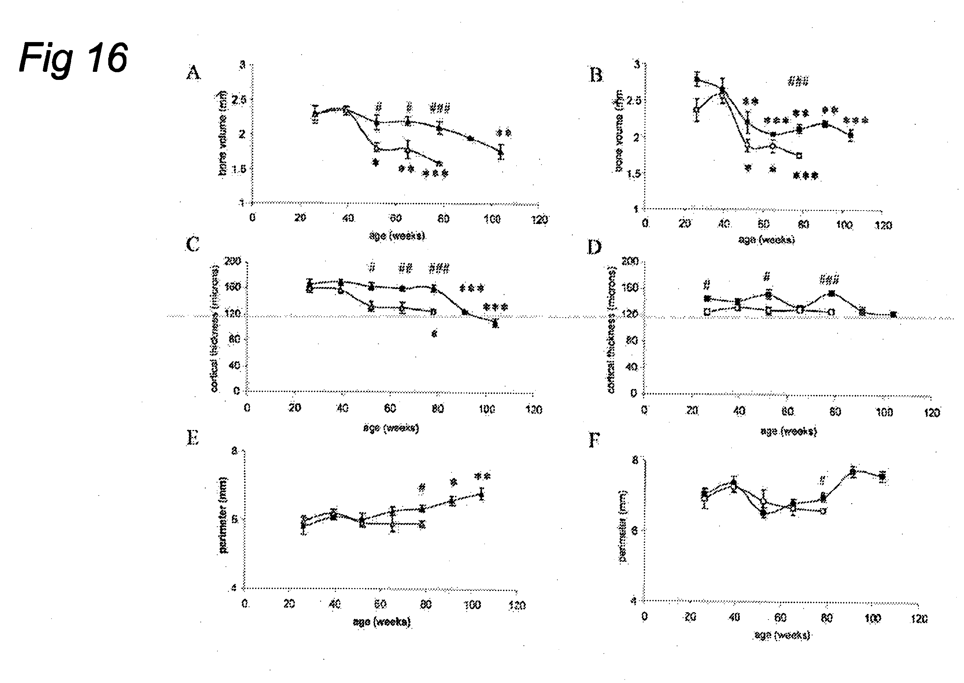

**Figure 16 Bone parameters in ageing.** Wild type females (closed triangles), TTD females (open triangles), wild type males (closed squares) and TTD males (open squares). Bone volume (A,B), cortical thickness (C,D) and perimeter (E,F). TTD mice compared to wild type animals: # p<0.05, ## p<0.01, ### p<0.001; TTD mice and wild type animals compared to their 26 week time point: * p<0.05, ** p<0.01, *** p<0.001; error bars: SEM

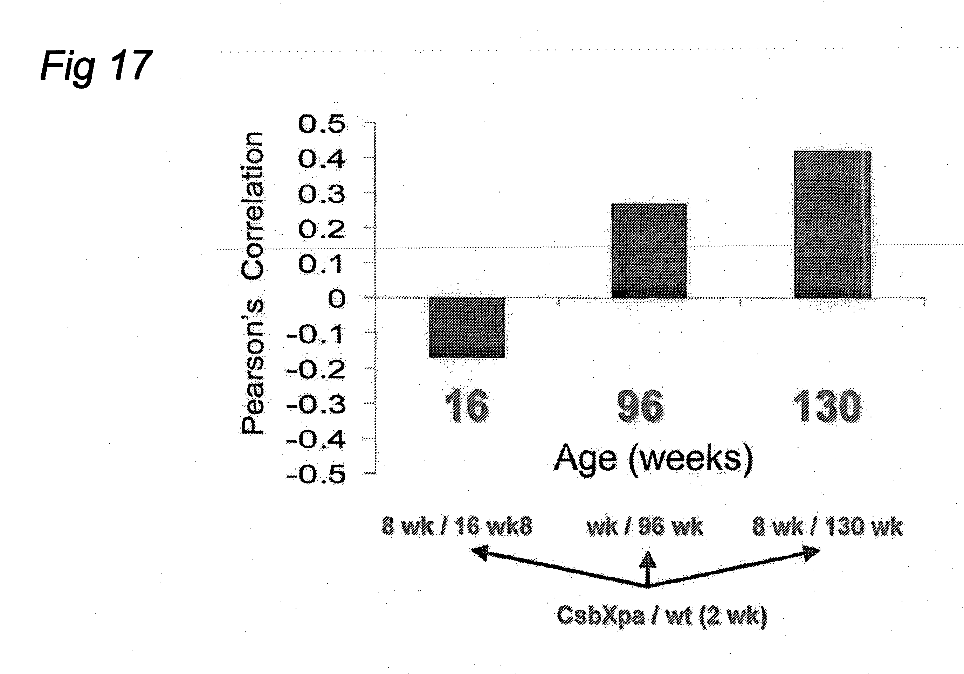

**Figure 17 Similarity of the liver transcriptome of $Csb^{m/m}/Xpa^{-/-}$ pups and naturally aged mice** Pearson's r correlation of 16, 96 and 130 week old mice with 15-day old $Csb^{m/m}/Xpa^{-/-}$ mice.

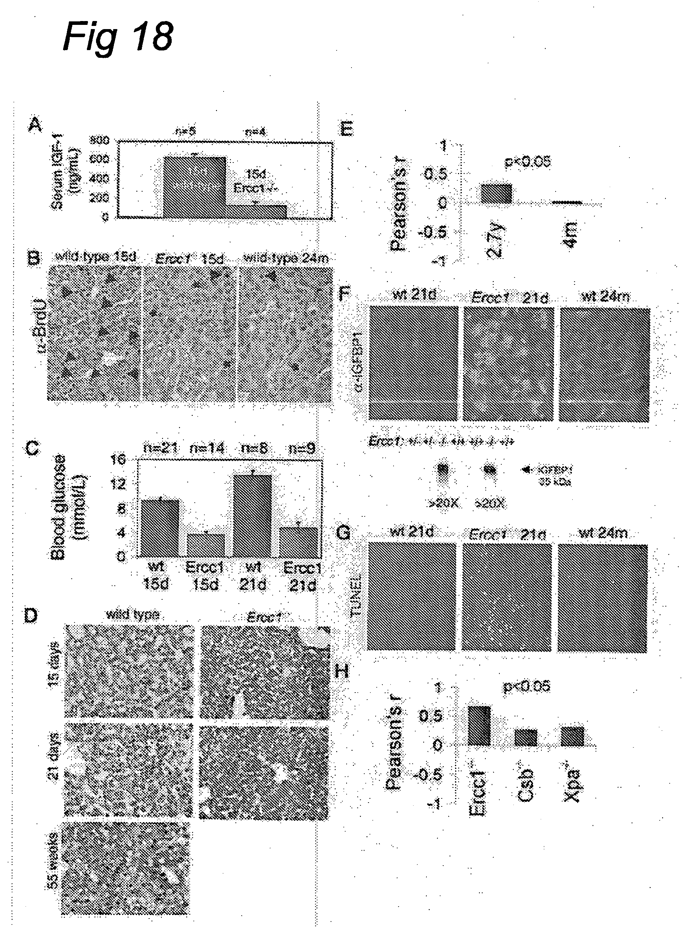

**Figure 18. Physiological changes in $Ercc1^{-/-}$ mice due to DNA repair defect. (A)** Serum IGF1 levels in 15 day-old wt and $Ercc1^{-/-}$ mice, measured by enzyme immunoassay. (B) Detection of proliferating cells using -BrdU. The synthetic nucleotide was injected into mice 30 min prior to sacrificing and incorporated into the DNA of replicating cells. Nuclei containing BrdU were detected by immunohistochemistry and horse radish peroxidase activity as described (arrows). Large polyploidy nuclei are apparent in the $Ercc1^{-/-}$ and aged mouse liver (asterisk). (C) Blood glucose levels of 15- and 21-day old $Ercc1^{-/-}$ mice and wt littermates. Average values for 8-21 animals per group are plotted $\pm$ the standard deviation. (D) Frozen liver sections from $Ercc1^{-/-}$ and wt mice of indicated ages stained for Oil Red O to detect triglyceride accumulation. (E) Pearson's correlation between *Ercc1*<sup>-/-</sup> mouse liver transcriptome and 2.7-year old and 4-month old mice (see Supplementary Table S3A). (F) Immunofluorescent detection of IGFBP1 in paraffin embedded mouse liver from a 21-day old, prematurely aged but not moribund, *Ercc1*<sup>-/-</sup> mouse and a wt littermate compared to the liver of a 2-year old mouse (100X magnification). Immunoblot of IGFBP1 levels in liver extracts of 21-day old *Ercc1*<sup>-/-</sup> mice and control littermates. (G) Measurement of apoptotic nuclei by TUNEL assay on serial sections of the same liver samples (20X magnification). (H) Pearson's correlation between liver transcriptomes of 15-day old *Ercc1*<sup>-/-</sup>, *Csb*<sup>m:m</sup> *and Xpa*<sup>-/-</sup> mice and *Csb*<sup>m/m</sup>/*Xpa*<sup>-/-</sup> mutants.

**Examples section**

Example 1

This example illustrates the application of micro-array analysis as a preferred readout for genome maintenance and ageing-related parameters in the method of the invention. Micro-arrays are a preferred way determining the effect of compounds in mammals used in the method of screening according to the invention. In this example, mRNA expression profiles of the liver are compared between young (15 days) and old (2 years) wt mice and compared with the mRNA profiles of *Ercc1*<sup>null/null</sup> (in this example hereafter referred to as *Ercc1*<sup>-/-</sup> mice) mice at 15 days that exhibit a combined NER/XLR/DSBR defect and display a pronounced segmental premature ageing phenotype that resembles that of severe XPF/ERCC1 (XFE) syndrome patients.

XPF-ERCC1 is an endonuclease required for multiple DNA repair pathways. Subtle mutations in *XPF* cause the cancer-prone syndrome xeroderma pigmentosum. We characterized a patient with a novel progeria and discovered a severe mutation in *XPF,* demonstrating that two distinct diseases stem from defects in this single protein. To gain insight into the mechanism of a DNA repair deficiency-induced progeria and its relationship to natural aging, we compared the gene expression profile of liver from mice genetically engineered to be deficient in XPF-ERCC1, young and old wildtype mice. There was significant overlap in the profiles of progeroid and aged mice indicating genotoxic and regenerative processes. The results strongly support a significant role for DNA repair in attenuating aging, implicate cytotoxic DNA damage in promoting aging and provide a rationale for the pleiotropy observed amongst progerias caused by DNA repair defects and natural aging.

Introduction:

Progeria encompasses a diverse set of spontaneous or inherited diseases characterized by the premature onset of signs and symptoms of aging. The relationship between progerias and natural aging is not known, but is important for understanding the causes of aging and developing practical models to study the aging process. However progerias are often segmental, or tissue-specific, making direct comparison to human aging unsatisfactory. There exist few direct comparisons between progeria and natural aging at the transcriptional or protein level, (1), but comparisons of cultured cells indicates parallels between the two.

Several inherited progerias are linked to defects in the cellular response to DNA damage, include Cockayne syndrome (CS), Werner, Rothmund Thomson, ataxia telangectasia and trichothiodystrophy (TTD) (2). This link suggests that an inappropriate response to DNA damage accelerates aging. Two of the human progerias, CS and TTD, are caused by a defect in nucleotide excision repair (NER). NER is a multi-step, multi-protein mechanism responsible for removing large bulky DNA lesions that distort the helical structure of DNA. Substrates for NER are identified in the genome either by the DNA damage recognition protein complex XPC-hHRad23B or during transcription if RNA Po1II stalls at the site of damage. CS and TTD are specifically caused by defects in transcription-coupled NER (*3*). In contrast, defects in the general NER mechanism cause the cancer-prone syndrome xeroderma pigmentosum (XP). XP patients have >1000-fold elevated risk of developing skin cancer in sun-exposed areas of the skin often in the first decade of life (*4*). However, XP patients have less pronounced premature aging compared to age-matched CS and TTD patients. These contrasting phenotypes suggest that identical DNA damages (substrates for NER) may contribute to cancer and aging, implicating the cellular response to that damage as critical to determining outcome.

XPF-ERCC1 is one of the structure-specific endonucleases required for NER (*5*). Both proteins, as well as their heterodimeric interaction, are highly conserved amongst eukaryotes (*5, 6*). Humans with subtle mutations in *XPF* have mild *XP* with cancer developing, on average, in the fourth decade of life (*7*) and patients with mutations in *Ercc1* are not known (*8*). This implies that XPF-ERCC1 is essential for human viability and therefore demands additional functions for the endonuclease beyond NER, since undetectable NER is not incompatible with life (*9*). Indeed, XPF-ERCC1 is required for a second DNA repair pathway: interstrand crosslink (ICL) repair (*10,* 11) and some types of mitotic recombination (*12-14*). ICLs are a unique class of DNA damage which involves covalent linkage of the two DNA strands, requiring a mechanism of repair distinct from NER (*15*). A novel progeria was discovered that we attribute to a severe mutation in *XPF* causing ICL hypersensitivity. Comparison of this progeria with natural aging implicates cytotoxic DNA damage as contributing to both.

*Materials and Methods*

**Characterization of XFE1RO patient fibroblasts.** Primary skin fibroblast cultures were established from a skin biopsy of patients. The cells were cultured in Hams F10 medium supplemented with 15% fetal calf serum and antibiotics. Cell strains studied included C5RO (normal), XFE1RO (new XP-F patient), XP42RO (typical XP-F patient with mild XP) as well as cells derived from an XP-C patient and a completely NER-deficient XP-A patient. Cell lines were immortalized by infection with a defective retrovirus expressing human telomerase reverse transcriptase (hTERT) as described (*54*). Expression levels of hTERT were determined by RT-PCR (*55*). Cellular survival was measured after exposure of primary fibroblasts to UV (UV-C, 254 nm) or immortalized fibroblasts to the crosslinking agent MMC and measuring clonogenic outgrowth, as previously described (*56*). Clonogenic survivals in MEFs were done with primary cell lines established from mouse embryos cultured at 3% $O_2$ (*57*). Capacity for NER and transcription coupled repair after UV damage were determined by unscheduled DNA synthesis (UDS) and RNA synthesis recovery, respectively, also as previously described (*56*). Complementation and sequence analysis. Patient cells were fused with a defined panel of XP patient primary fibroblasts cell lines using Sendai virus and assayed for UV-induced UDS after 24 h as described (*58*). Total RNA was isolated from the patient fibroblasts and reverse transcribed using random hexamer primers. The *hXPF* gene was amplified in two overlapping fragments from the cDNA and directly sequenced by standard protocols. The coding sequence of the *hXPF* gene was amplified from genomic DNA isolated from fibroblasts using exon-specific primers and sequenced. **Immunodetection of XPF-ERCC1**. Expression of XPF and ERCC1 proteins was detected by immunoblotting of 10 µg of whole cell extract (WCE) from immortalized fibroblast cultures using mouse monoclonal α-hXPF (Neomarkers; 1:1000) and affinity purified polyclonal α-hERCC1 antibodies (*59*).

Generation of *Ercc1$^{-/-}$* mice. Establishment of a genetically targeted *Ercc1$^{-/-}$* mice was previously described (*22*). Homozygous mutant mice were generated in a mixed FVB and C57B1/6 genetic background by the intercrossing of inbred *Erecc1$^{+/-}$* mice. Postnatal day 2, litters were culled to an average size of 5 pups to reduce competition for nursing. Genomic DNA was isolated from tail tissue and genotyped by PCR (*15*). **Analysis of Gait.** Ataxia was assessed by foot printing analysis of 3 wk old *Ercc1$^{-/-}$* and wt littermate mice as described (*60*). Briefly, the forepaws of the animals were painted with purple water-based paint, the hind paws with green. The mice were released into a barricaded passage 7 x 30 cm and allowed to escape to a darkened refuge at the end. Data was recorded after 3 trial runs on consecutive days.

**Autoradiography.** Mice were anaesthetized by intraperitoneal injection of ketalin and xylazine (120 and 7.5 µg/g body weight, respectively). Lateral films were taken at 2X magnification using a CGR Senograph 500T X-ray instrument operated at 30kV and 32mAS. A molybdeen focus (0.1mm) was used with a 65 cm focus-film distance and 32.5 cm focus-object distance. Kodak X-ray film (MIN-R MA 18 x 24 cm) and a Dupont Cronex low-dose mammography intensifying screen were used.

**RNA isolation and cDNA microarray analysis**. *Ercc1$^{-/-}$* and littermate controls in two genetic backgrounds were obtained from our colony at Erasmus Medical Center. Young (6 months) and old (26 months) C57B1/6 mice were obtained from the National Institute on Aging and shipped to the microarray core facility at the University of Texas Health Science Center, where they were housed for at least two weeks before experimentation. Mice were euthanized by cervical dislocation then the liver excised and examined for gross pathology before snap freezing in liquid nitrogen prior to processing. Total RNA was isolated using TriReagent (Sigma) and RNeasy kits (Qiagen). The purity of the RNA was determined by spectrophotometric measurements ($A_{260}/A_{280}$ > 1.8) and its integrity by denaturing gel electrophoresis. The RNA was precipitated with 4M ammonium acetate and ethanol. Fluorescently labeled cDNA substrates for microarray hybridization were produced by indirect labeling. Briefly, amino-allyl modified cDNAs were synthesized by reverse transcription using 15 µg of total RNA, oligo-dT primers (Invitrogen), Superscript RT (Invitrogen) and dNTP containing a 1:1:1:0.1:0.9 ratio of dATP:dCTP:dGTP:dTTP:amino-allyl-dUTP. The cDNAs were purified from the reaction mixture using Micron YM-30 filters and coupled with cyanine dyes (Cy3 or Cy5, Amersham Biosciences). The appropriate Cy3 (red) and Cy5 (green) labeled cDNAs were combined and repurified using Qiaquick PCR purification kit (Qiagen) and concentrated by speed vacuum drying. The samples were resuspended in DIG Easy Hyb buffer (Roche) containing 0.5 µg/µl yeast tRNA and 0.5 µg/µl sheared salmon sperm DNA then hybridized to cDNA microarray chips. The chips were prehybridized for 1h in a buffer containing 25% formamide, 5X saline sodium citrate, 0.1% sodium dodecyl sulfate and 10 mg/ml bovine serum albumin. Hybridizations were done at 48° for 16h, following which the slides were washed with different stringencies, dried and scanned using a dual laser Axon scanner.

The mouse cDNA chips containing 1912 features (Supplemental Table 1; http://microarray.stcbmlab.uthscsa.edu/mouse1912c.gal) in duplicate were printed on CMT-GAPS slides (Corning) at the Microarray Core Facility, University of Texas Health Science Center, San Antonio, TX from the GEM-1 mouse cDNA library (Incyte). The intensity values were quantitated using Spot (CSIRO) and normalized using the Statistics for Microarray Analysis (SMA; http://www.stat.berkeley.edu/users/terry/zarray/)software tools from Dr. Terry Speed, University of Berkeley, Berkeley, CA, which runs on "R" (http://www.r-proiect.org/). The average of the normalized data is represented as a scatter plot of the intensity ratio calculated as log2(red/green) ratio vs. the total intensity value calculated as $\log_2(\text{red} \times \text{green})^{1/2}$. Significance testing was done using the SAM software from Stanford University (http)://www.stat.stanford.edu/~tibs/SAM/ and SMA.

**Immunoanalyses and TUNEL assay.** 3 wk old mice were sacrificed by cervical dislocation. The liver was dissected out, fixed in 4% paraformaldehyde in sodium phosphate buffer, pH 7.4, at 4°C overnight, dehydrated and embedded in paraffin. Serial 5 $\mu$m sections were collected on Superfrost Plus slides (Fisher), dried at 37°C overnight and processed for immunohistochemistry using citric acid-based antigen retrieval as described (61). IGFBP-1 was detected with goat polyclonal IgG ($\alpha$-IGFBP-1 (Santa Cruz; 1:100) followed by rabbit $\alpha$-goat-fluorescein-ITC (Sigma; 1:500). The fraction of proliferating hepatocytes was measured by injecting the mice with 50 mg/kg bromodeoxyuridine (BrdU) in phosphate-buffered saline 30 min prior to sacrifice. Incorporation of BrdU into cellular DNA was detected using mouse monoclonal a-BrdU (Abcam; 1:50) and $\alpha$-mouse IgG-HRP conjugate (Sigma; 1:1000). Apoptosis was detected by TUNEL assay using the Promega Apoptosis detection system according to the manufacturer's instructions. For immunoblots, animal livers were dissected and submerged in 100 mM NaCl, 50 mM Tris-HCl, pH 7.5, 5 mM EDTA with 0.5% Triton on ice. The samples were sonicated using a Soniprep 150 (Sanyo) equipped with a microprobe at maximum amplitude for 10 sec. The protein content was measured using Coomassie Plus protein assay kit (Pierce) and 10 $\mu$g of each sample was electrophoresed, blotted and probed with: $\alpha$-cathepsin S (CalBiochem #219384, 1:1000); $\alpha$-IGFBP-1 (Santa Cruz #sc-6000; 1:1000), $\alpha$-p53 (Signet, #CM1; 1:1000); ($\alpha$-CYR61 [IGFBP-10], Abcam #ab2026; 1:100); $\alpha$-cytochrome P450 4A (GeneTex, #ab3573; 1:1500) or $\alpha$-cathepsin L (Abcam, #ab7454; 1:1000) by standard procedure. Band intensity was measured using Quantity One software (Bio-Rad).

### Results and Discussion

### A patient with a novel progeroid syndrome and XPF-ERCC1 deficiency.

A young boy was referred to us at the age of 15 with complaints of frequent sunburns and premature aging (Fig. 1; Case report Supplemental online material S1). Due to his photosensitivity, skin fibroblasts were obtained (cell line XFE1RO) and their sensitivity to UV light was compared to that of cells derived from patients with XP due to defective NER of UV photodimers (see Material and Methods; Supplemental online material S2). XFE1RO cells were ~6X more sensitive to UV in terms of survival than wild-type (wt) cells. This was intermediate to the sensitivity of cells from patients with mild (XP-C and -F complementation groups) and severe XP (XP-A) (Fig. 2A). DNA repair of UV damage was determined by measuring the amount of radiolabeled $^3$H-thymidine incorporated into cells (unscheduled DNA synthesis or UDS) after UV exposure (Fig. 2B). UDS in XFE1RO cells was only ~5% of the normal level, which is only slightly higher than the most severe XP patients with a complete absence of NER (complementation group A). RNA synthesis recovery after UV damage was measured as an indicator of transcription-coupled DNA repair (Fig. 2C). This too was severely affected in XFE1RO cells, to the same extent as NER-deficient cells from an XP-A patient and transcription-coupled NER deficient cells from a CS patient. In summary, XFE1RO cells demonstrated an almost complete absence of UV-induced DNA repair, which is a hallmark of XP, despite the fact that his clinical history was inconsistent with this diagnosis.

In spite of the unusual symptoms of the patient, the profound UV-sensitivity of his fibroblasts indicated a defect in NER. Thus complementation analysis was performed by fusing XFE1RO cells with fibroblasts of all XP groups -A to -G and measuring UV-induced UDS (Fig. 2D). XFE1RO cells corrected DNA repair of all groups except XP-F. This result was unexpected because previously reported XP-F patients all had substantial residual NER and, as a consequence, only mild symptoms of XP (*16, 17*). In order to confirm the assignment of XFE1RO to the XP-F complementation group, mRNA was isolated from the patient fibroblasts, reverse transcribed and sequenced, revealing a G→C transversion at position 478 in *Xpf* (Fig. 2E). This mutation predicted a non-conservative amino acid substitution of arginine 142 to proline ($R^{142}P$). Sequence analysis of the patient's genomic DNA demonstrated that he was homozygous for this mutation, consistent with recessive inheritance and parental consanguinity.

$R^{142}$ is conserved in all eukaryotes with the exception of *Arabidopsis thaliana,* which has a physicochemically related lysine residue at that position (Fig. 2F). $R^{142}$ resides in a highly conserved domain of the protein harboring conserved helicases motifs thought to be involved in DNA binding (*18*) and a series of leucine-rich motifs, which are often involved in protein:protein interactions (*19*). The $R^{142}P$ mutation is unique and the most N-terminal of the *Xpf* mutations reported (Fig. 2F). To determine if the mutation impacted XPF protein size or stability, whole cell extracts from XFE1RO fibroblasts were immunoblotted and screened with antibodies raised against full-length hXPF. The XPF protein level was significantly reduced (~10X) in XFE1RO cells compared to wt or cells from a patient with mild XP due to a mutation in *Xpf* (Fig. 2G). Similarly, ERCC1 protein levels were reduced in XFE1RO cells, although not to the same extent (Fig. 2H). These findings substantiate previous reports that ERCC1 protein levels are reduced in *Xpf* mutant Chinese hamster ovary cells and vice versa, indicating that XPF-ERCC1 protein interaction is required, at least to some extent, for stabilization of both proteins (*20*). Importantly, a trace amount of residual full length XPF was detected in patient XFE1RO cells, consistent with the notion that a complete absence of XPF-ERCC1 is incompatible with human life. In total, these data reveal that very low levels of XPF-ERCC1 cause progeria in humans.

Although XFE1RO cells showed almost a complete absence of UV-induced DNA damage repair or NER, cells derived from XP patients performed just as poorly in DNA repair assays (Fig. 2). Yet these patients (XP complementation group A) had severe XP without multi-organ progeria. Thus we conclude that the progeria in our patient was not caused by a defect in NER. Because XPF-ERCC1 is also implicated in DNA ICL repair (*10*), we measured the survival of XFE1RO cells after exposure to mitomycin C (MMC; Fig. 2I). XFE1RO fibroblasts were significantly more sensitive to ICL damage than wt cells, cells from an XP-F patient with mild XP or cells from an XP-A patient with severe XP and undetectable levels of NER Therefore a unique feature of human cells with severely reduced levels of XPF-ERCC1 is hypersensitivity to DNA ICLs, which could be the mechanistic basis for the progeria. However, the progeric features may be influenced by additional genetic and environmental factors, which we have not under control here, as we have seen a single mild XPF case also showing such an exaggerated in vitro ICL-sensitivity (data not shown). These unusual DNA repair characteristics, the unique constellation of symptoms and prominent progeroid features define a novel syndrome that we term XPF-ERCC1 (XFE1) progeroid syndrome.

**Comparison of patient XFE1RO with *Ercc1*⁻/⁻ mice.** Unlike humans, Ercc1- and Xpf-deficient mice are viable (*21-23*). However, the phenotype of the mice is extremely severe and like patient XFE1RO, quite distinct from NER-deficiency (*24*). *Ercc1*-deficient mice develop normally, are slightly dwarfed at birth (Fig. 3A), then die in the third week of life (Fig. 3B) of liver failure (*25*). Cachexia, epidermal atrophy, progressive renal and liver dysfunction as well as hepatocellular polyploidization and intra-nuclear inclusions in *Ercc1*⁻/⁻ mice were previously reported and are symptoms that can be associated with advanced age in mammals, suggesting that the mice age prematurely (22). We identified additional features in *Ercc1*⁻/⁻ mice consistent with progeria. By 10 days of age the mice were dystonic, manifested as tremors and an abnormal posturing (flexion rather than extension) when suspended by the tail (data not shown). Secondary dystonia can be caused by neurodegeneration, a common age-dependent phenomena in mammals. In addition, the *Ercc1*⁻/⁻ mice have progressive ataxia, further indicating neurodegeneration (Fig. 3C), sarcopenia (data not shown), decreased stress erythropoiesis (*26*) and kyphosis, suggestive of osteoporosis (Fig. 3D), all of which are symptoms frequently associated with mammalian aging (*27-29*). Importantly, like XFE1RO cells, *Ercc1*⁻/⁻ mice are deficient in XPF (Fig. 3E) and significantly more sensitive to drugs that cause DNA ICLs than other NER-deficient mouse embryonic fibroblasts (MEFs; Fig. 3F), demonstrating the similarity not only between the phenotype of the patient and the mouse model, but also the mechanistic basis for the phenotype.

Both the patient and *Ercc1*⁻/⁻ mice were phenotypically normal during early development (Fig. 1A and 3A); premature aging began in early prepubescence and progressed rapidly (Fig. 1B and 3B), resulting in premature death prior to sexual maturation. In addition to the old, wizened appearance of XFE1RO (Fig. 1B), progeroid symptoms included atrophy of the epidermis, visual and hearing loss, ataxia, cerebral atrophy, hypertension, renal and liver dysfunction, anemia, osteopenia, kyphosis, sarcopenia and weight loss (for a detailed comparison, see Table I). Amongst the symptoms readily measured in mice, the only progeroid feature observed in XFE1RO not observed in the *Ercc1*⁻/⁻ mice, was anemia, although we previously noted iron deposition in the spleen suggestive of a high turn-over of red blood cells (*22*) and decreased stress erythropoiesis (*26*), making it likely that peripheral anemia would occur if the mice lived longer. Therefore, both the patient XFE1RO and the *Ercc1*⁻/⁻ mice display progeria of the neurologic, dermatologic, musculoskeletal, hematopoietic, renal and hepatobiliary systems as a consequence of decreased XPF-ERCC1 DNA repair endonuclease.

**Microarray analysis of *Ercc1*⁻/⁻ mouse liver.** Having validated the *Ercc1*⁻/⁻ mouse as a *bona fide* model of the XFE1RO progeroid syndrome, we sought to shed light on the cause of the premature aging features by examining the gene expression profile in one of the most severely affected tissues compared to littermate controls. We selected liver because it was affected in both the patient and the mouse model and liver dysfunction in the mouse is accompanied by well-defined features of premature aging [nuclear polyploidization (*30*), occurrence of intranuclear inclusions (*22*)] as well as indications for accumulation of DNA damage [p53 stabilization (*21*)].

Total RNA isolated from the liver of 21 day old *Ercc1*⁻/⁻ mice was compared to that of wt littermates in two different experiments (Array defined in Supplemental Table 1). First, pooled RNA samples from 3 *Ercc1*⁻/⁻ and 3 littermate controls were compared, for two mixed genetic backgrounds (FVB/n:C57B1\6 and 129/Ola:C57B1\6) using at least 4 arrays, including dye swap, for each pool comparison (Fig. 4 and Table 2; respectively). In the second experiment we compared individual *Erccl*⁻/⁻ animals (in a 50:50 C57B1/6:FVB/n background) with littermate controls, in 3 random pairs (Supplemental Table 3). There was a significant degree of similarity between the results obtained for the two different genetic backgrounds: 50% of the most significantly differentially expressed genes appeared were identical in both backgrounds. This indicates that the *Ercc1*⁻/⁻ expression profile is not unique to a particular strain of mice. Nor did we detect a significant difference in results between the two experimental designs. Sixty-five percent of the genes identified as most significantly differentially expressed were identical to genes identified in the experiment with pooled samples. Finally, we developed a gene expression profile representing normal liver aging by doing a pair-wise comparison of 6 young (6 months) and 6 old (26 months) C57B1/6 mice in two separate experiments (Table 4).

Fig. 4 shows an example of the results obtained for the pooled comparison of *Ercc1*[-/-] versus littermate controls. Using SAM, we identified fourteen genes that were significantly overexpressed in *Ercc1*[-/-] mouse liver compared to control animals and 3 genes that were significantly underexpressed (Fig. 4B). More than 50% of the genes identified in *Ercc1*[-/-] mice were also significantly differentially expressed in aged mice (Fig. 4B). The probability of this overlap occurring randomly is < $10^{-4}$. These findings indicate that genome-wide changes in expression profiles observed in natural aging are to a significant degree reproduced in the 3-week old liver of *Ercc1*[-/-] mice, establishing the parallel between their progeria and natural aging at the fundamental level of gene expression. In addition these findings invoke a substantial role for DNA repair in the attenuation of aging and validate the use of mice with progeroid syndromes due to a defect in DNA metabolism as excellent study tools for understanding aspects of mammalian aging.

**Analysis of gene expression profile.** Several of the genes differentially expressed in aged and *Erccr1*[-/-] liver could be functionally linked. Insulin-like growth factor binding protein 1 (IGFBP-1), its ligand insulin like growth factor 1 (IGF-1) and fatty acid amide hydrolase (FAAH) levels are regulated by the growth hormone (GH)/glucocorticoid axis. IGFBP-1 is produced and secreted primarily by hepatocytes and sequesters circulating IGF-1, dampening its mitogenic activity. Elevated levels of IGFBP-1, coupled with low levels of IGF-1 and FAAH, as demonstrated by the microarray analysis and immunodetection (Fig. 5A and G), indicate decreased GH signaling in the mutant and aged mice (*31*). GH levels decline with age (*32*) providing physiologic corroboration of the microarray results. Serum levels of GH were normal in our progeroid patient despite his dwarfism (legend Fig. 1), indicating that GH deficiency was not the primary cause of the patient's progeria. The observation that the GH axis is disrupted in XPF-ERCC1 deficiency creates a link between catabolic pathways and DNA repair deficiencies, both of which are known to negatively influence longevity in mammals.

Further analysis of the microarray data yielded 3 clusters of differentially expressed genes in *Ercc1*[-/-] and aged mouse liver that provided interesting clues as to the mechanism of GH axis disruption and suggested a plausible scenario for the onset of aging. The first group was downstream effectors of the peroxisome proliferator activated receptor α (PPARα), including: IGFBP-1, cytochrome P450 4A10, cytochrome P450 4A14 and esterase 31. Since IGFBP-1 levels are up-regulated by PPARα (*33*), disruption of the GH axis may be a direct consequence of PPARα activation, reflecting a tight link between this two signaling cascades in mediating aging.

PPARα is a transcription factor that when activated increases fatty acid β-oxidation by regulating expression of lipid transport and metabolizing proteins (*34*). Enhanced expression of PPARα effectors in *Ercc1*[-/-] and aged mouse liver therefore indicates a state of elevated lipid peroxidation (LPO) (*35*). The endogenous ligands of PPARα are long, straight-chain free fatty acids. Thus this cascade may be triggered by break-down of cell membranes during hepatocyte toxicity. This scenario is supported by the second cluster of genes differentially expressed in *Ercc1*[-/-] and aged mouse liver, *i.e.* genes indicative of hepatocyte toxicity including angiogenin, $Ca^{2+}$-transporting ATPase and CEA-related cell adhesion molecule I. Hepatocellular toxicity is further supported by the presence of liver enzymes in the serum of the patient and mouse [Table I and (*21*)] as well as polyploidization of hepatocellular nuclei, which is characteristic of *Ercc1*[-/-] and aged wt liver (Fig. 6B) (*21, 36, 37*).

Importantly, activation of PPARα and elevated expression of IGFBP-1 are induced in rats or cultured hepatocytes treated with the DNA ICL agent cisplatin (*38*). In light of the fact that the unique defect in both *Erccr1*[-/-] mice and XFE1RO cells is an inability to repair DNA ICLs, it is possible that unrepaired endogenous ICLs initiate the events that culminate in spontaneous premature aging. DNA ICLs are in and of themselves extremely cytotoxic (*15*). Thus a likely scenario is that unrepaired ICLs cause hepatocellular death, membrane fatty acids are released from the dying cells, which activate PPARα and lead to the suppression of mitotic activity causing tissue aging. Interestingly, PPARα-induced LPO may also act as a source of endogenous DNA ICLs (*39*), providing a mechanism by which the accumulation of tissue damage could self-perpetuate. The overall similarity in the gene expression profile of *Ercc1*[-/-] and aged wt mouse liver, implies an important role for endogenous DNA ICLs in promoting aging not only in the case of a DNA repair-deficiency but also in repair-competent organisms.

The third major cluster of genes that were differentially expressed in *Ercc1*[-/-], and to a lesser extent aged wt mouse liver, are markers of liver regeneration and tissue remodeling, including S-adenosyl methionine synthetase, angiogenin, tubulin α4, α-mannosidase II, cathepsin L and fatty acid amide hydrolase(*40-43*). Fat specific protein 27 is a marker of adipocyte differentiation (*44*). Fatty change is a well-recognized intermediate of liver failure caused by hepatotoxins or dysregulation of lipid metabolism (*45*) as would result from chronic activation of the PPARα pathway. IGFBP-10 and CBFA2T3 are highly expressed in terminally differentiated or senescent cells (*46, 47*). These data further support the clinical picture of chronic liver injury and regeneration.

**Immunoanalyses and TUNEL assay.** For all gene products indicated as overexpressed by microarray analysis, and for which antibodies were commercially available, protein levels were compared in Ercc1-deficient, wt young and old mouse liver. IGFBP-1 was detected exclusively in the cytoplasm of hepatocytes and in erythrocytes within liver sinusoids (*48*). IGFBP-1 levels were significantly elevated in the *Ercc1*[-/-] mouse liver compared to the young wt mouse (Fig. 5A and C). IGFBP-1 levels were also elevated in the old wt mouse. These results correspond with the microarray data, which indicated a 3.7- and 1.9-fold increase in the *Ercc1*[-/-] and old wt mouse relative to the young wt mouse and provides support for the validity of the gene profile at the protein level. This was further confirmed by immunodetection of IGFBP-10, cytochrome P450 4A, and cathepsin S, all of which were overexpressed in *Ercc1*[-/-] and/or aged wt mouse liver compared to young wt mice, as predicted by gene expression profiling (Fig. 5C).

The TUNEL assay was used to assess rates of apoptosis in the mouse liver sections (Fig. 5B). Apoptotic nuclei were rare in the young wt mouse liver. However, nuclear fluorescence was common in the *Ercc1*[-/-] liver, consistent with a high rate of apoptosis. Levels were also modestly elevated in the aged wt mouse liver. These data support the contribution of hepatocyte cell death to the aging phenotype and implicate apoptotic pathways as a mediator of cell death. Interestingly, DNA damage commonly triggers cell death through p53-dependent apoptosis (*49*) and p53 levels are elevated in *Ercc1*[-/-] hepatocyte nuclei [Fig. 5C and(*21*)].

**Model for aging as a consequence of unrepaired DNA damage.** In total, the microarray data suggest a generalized mechanism by which aging could arise as a consequence of a defect in XPF-ERCC1 (Fig. 6A). We propose that endogenous DNA ICLs accumulate acutely in repair-deficient cells or chronically with aging. Cytotoxic DNA lesions trigger cell death or senescence (polyploidization). In tissues that are not post-mitotic, *e.g.* liver, the response to injury is an attempt to regenerate functional tissue through cell proliferation, differentiation and tissue remodeling. Excessive regeneration may further contribute to premature senescence of proliferative cells and lead to an inability to respond to mitogenic signals. Exposure to endogenous genotoxins is perpetual, thus the cycle persists and the regenerative capacity of a tissue is eventually eroded, leading to loss of tissue function and the onset of aging symptoms.

We measured the proliferative fraction of cells in the liver of the mice as an index of their relative regenerative capacity (Fig. 6B). Mice were injected with bromodeoxyuridine (BrdU), which is incorporated into the genomic DNA of cells replicating at the time of injection. Ten percent of the young wt mouse hepatocytes stained positively for BrdU, consistent with proliferation rates measured in other rodent models (*30*). In sharp contrast, BrdU-positive nuclei were greatly diminished in the *Ercc1*[-/-] and the aged wt mouse livers, reflecting highly attenuated rates of proliferation. This is consistent with our model in which aging of a tissue is directly correlated with diminished regenerative capacity. In support of our model gene profiling of skeletal muscle of old wt mice demonstrates overexpression of genes involved in stress response and apoptosis, which led to the proposal that aging is caused by a response to oxidative-induced tissue injury (*50*). Similarly, Cao, *et al.,* reported microarray data on aged mouse liver in which there was evidence for cellular stress, tissue fibrosis with decreased proliferation capacity (*51*).

Our model also offers an explanation as to why progeroid syndromes due to defects in DNA metabolism are pleiotropic and segmental. The key principle of the model is that cytotoxic damage causes aging. Cytotoxic DNA lesions include ICLs, DNA double strand breaks and transcription-blocking lesions. Organ-related differences in metabolism result in organ-specific spectra of spontaneous DNA lesions. Consequently different DNA repair mechanisms (ICL repair, double-strand break repair and transcription-coupled repair) are more or less essential to ward off aging in each tissue. Defects in transcription-coupled repair are associated primarily with neurologic symptoms, *e.g.* CS and trichothiodystrophy. In contrast, the epidermis, but not central nervous system, is affected in mice with genetic defects in non-homologous end-joining of double strand breaks (*52*).

In summary, we have identified a novel progeroid syndrome in man and mice, which is the consequence of a mutation in either *Xpf* or *Ercc1,* resulting in profound sensitivity to DNA ICLs. Microarray and immunohistochemical data support a mechanism of aging as a consequence of a cytotoxic response to DNA damage and subsequent loss of tissue regenerative capacity. Gene profiling of old wt mouse liver produced significant overlap with the profile obtained from *Ercc1*[-/-] mice, indicating that this mechanism may apply to natural mammalian aging and providing opportunity for intervention These mouse models are therefore extremely suitable for use in the method of screening for compounds according to the current invention and as exemplified in the following example 4.

**TABLE 1**. Comparison of the clinical features of a patient with mild xeroderma pigmentosum complementation group F to the progeroid patient with a severe mutation in *Xpf* (XFE1RO) and *Ercc1*[-/-] mice. Symptoms that can be associated with advanced age are indicated in italics.

| Symptom | XP patient with *Xpf* mutation | XFE1RO | *Ercc1*[-/-] mice |
|---|---|---|---|
| **Dermatologic** | | | |
| Photosensitivity | +/- | + | +[a,c] |
| *hyperpigmentation* | + | + | ? |
| *atrophic epidermis* | +/- | + | +[a] |
| *skin cancer* | 4th decade of life | - | - |

(continued)

| | | | |
|---|---|---|---|
| **Neurologic** | | | |
| *hearing loss* | - | + | ? |
| *visual impairment* | - | + | +[b] |
| *tremors* | rare | + | +[a] |
| *ataxia* | mild, rare | + | +[a] |
| *cerebral atrophy* | - | + | ? |
| **Cardiovascular** | | | |
| *Hypertension* | - | + | ? |
| **Renal** | | | |
| *Acidosis* | - | + | +[e] |
| **Hepatobiliary** | | | |
| *Serum liver enzymes* | normal | ↑ | ↑[c] |
| Serum bilirubin | normal | ? | ↑[c] |
| Ferritin | normal | ↑ | splenic deposits[a] |
| *Serum albumin* | normal | ↓ | ↓[c] |
| **Hematopoeitic** | | | |
| *Anemia* | - | + | -[b] |
| **Musculoskeletal** | | | |
| *Ostepenia* | - | + | ? |
| *Kyphosis* | - | + | +[d] |
| Dystonia | - | + | +[d] |
| *Sarcopenia* | - | + | +[d] |
| **Systemic** | | | |
| *growth retardation* | - | + | +[a,c] |
| *Cachexia* | - | + | +[a,c] |
| *aged appearance* | | + | +[a,c] |
| premature death | - | 16 yr | 3 wk[c,d] |

[a](22), [b]Niedernhofer, L.J. and Hoeijmakers J.H.J. unpublished data, [c](21),
[d]This study, [e](25), [f]γ-glutamyl transferase, alkaline phospatase and α-anti-trypsinase.

**Table 2.** Genes determined to be significantly differentially expressed in Ercc1-deficient mouse liver compared to littermate controls in an **129/Ola:C57Bl/6** mixed genetic background. RNA was pooled from 3 mice of each genotype and a single comparison made between mutant and control pools with reciprocal dye swapping. The names of the genes, their Genebank Accession number and the fold difference in expression are indicated. Genes indicated with # were similarly differentially expressed when an identical comparison was made, but with mice in an **FVB/n:C57Bl/6** mixed genetic background (Fig. 4). The asterisk * indicates genes that were also identified as significantly differentially expressed when *Erccr1[-/-]* mouse liver was compared to control littermates in a pair-wise comparison.

| Gene name | Gene ID | Fold change in old wild-type liver |
|---|---|---|
| insulin-like growth factor binding protein 1 # | W83086 | 9.5 * |
| cytochrome P450, 4a14 # | AA060595 | 3.0 * |
| fat specific gene 27 # | AA466094 | 2.8 * |

(continued)

| Gene name | Gene ID | Fold change in old wild-type liver |
|---|---|---|
| S-adenosylmethionine synthetase # | W29782 | 2.4 |
| protein phosphatase 3 | AA178283 | 2.0 |
| phenylalanine hydroxylase | AI323717 | 1.9 |
| Treacher Collins Franceschetti syndrome 1 | AA038551 | 1.8 |
| Faciogenital dysplasia homolog | AA272942 | 1.7 |
| cytochrome P450, 4A14 # | AA061737 | 1.7 * |
| cytochrome P450, 4a10 # | AA423149 | 1.7 * |
|  |  |  |
| fatty acid amide hydrolase # | AA269227 | -7.2 * |
| Talin | AA208883 | -4.3 |
| esterase 31 # | AA254921 | -2.8 * |
| hemoglobin , adult chain 1 | AA109900 | -2.6 |
| *Mus musculus* mRNA for Zn finger protein s11-6 | W83512 | -1.9 |
| polymeric immunoglobulin receptor | AA277571 | -1.8 |
| *Mus musculus* T10 mRNA | AI662826 | -1.8 |
| nuclear factor of activated T cells | AA521764 | -1.7 |
| *Mus musculus* mRNA for JKTBP | AA260901 | -1.7 |
| insulin-like growth factor 1 # | W10072 | -1.7 * |

**Table 3**. Genes identified as significantly differentially expressed in Ercc1-deficient mouse liver compared to wt littermate controls, using a single animal pair-wise comparison. Genes marked with an asterisk * were found to be similarly differentially expressed when Ercc1-deficient mouse liver was compared to control littermates using pooled samples representing multiple animals (Figs. 4).

| Gene name | Gene ID | Fold change in old wild-type liver |
|---|---|---|
| cytochrome P450, 4a14 * | AA060595 | 7.5 |
| fat specific gene 27 * | AA466094 | 4.4 |
| insulin-like growth factor binding protein 1* | W83086 | 3.7 |
| cytochrome P450, 4a10 * | AA109684 | 3.2 |
| tubulin α4 | W11746 | 1.9 |
| solute carrier family 27 (fatty acid transporter) | AA108401 | 1.8 |
| control for tubulin α4 | none | 1.8 |
| cytochrome P450, 4a14* | AA061737 | 1.8 |
| angiogenin | AA237829 | 1.7 |
| paraoxonase 1 | W98586 | 1.6 |

(continued)

| Gene name | Gene ID | Fold change in old wild-type liver |
|---|---|---|
| ATPase, Ca$^{2+}$ transporting, cardiac muscle* | W34420 | 1.6 |
| Murine Glvr-1 mRNA * | AA177949 | 1.6 |
| transcription termination factor 1* | AA049906 | 1.6 |
| CBFA2T3* | AA051563 | 1.6 |
| cathepsin L | AA174215 | 1.6 |
| Rad23A | AA061459 | 1.5 |
| transthyretin | W17647 | 1.5 |
| mannosidase 2, $\alpha$1* | W09023 | 1.5 |
| CEA-related cell adhesion molecule 1* | AA245546 | 1.4 |
| insulin-like growth factor binding protein 10* | AA423149 | 1.4 |
| | | |
| fatty acid amide hydrolase* | AA260227 | -14.1 |
| Esterase 31* | AA254921 | -4.8 |
| insulin-like growth factor 1* | W10072 | -2.4 |

**Table 4.** Microarray analysis of aged mouse liver RNA. The table indicates the identity of genes significantly differentially expressed in aged (26 mo) wt mouse liver compared to young (6 mo) wt mouse liver. The experiment included a pair-wise comparison of 6 animals of each age group. The names of the genes, their Genebank Accession number and the fold difference in expression are indicated. Genes indicated with an asterisk * were similarly differentially expressed in 3 wk old Ercc1-deficient mouse liver compared to wt littermates. Genes indicated with A were also differentially expressed in 3 wk old Ercc1-deficient mouse liver, but in the opposite direction. For gene products with antibodies commercially available, protein levels in extracts from aged wild-type mouse liver was compared to young wild-type mouse liver by immunoblot and the results are indicated in parentheses in the last column.

| Gene name | Gene ID | Fold change in old wild-type liver |
|---|---|---|
| ATP-binding cassette 2 | AA276156 | 3.1 |
| cytochrome P450, 4A14* | AA061737 | 2.9 (12X) |
| *Mus musculus* cleavage and polyadenylation | AA267638 | 2.4 |
| cathepsin S | AA178121 | 2.3 (1.8X) |
| histocompatibility 2, K region | W14540 | 2.1 |
| Lipocalin 2 | AA087193 | 1.9 |
| *Mus musculus* B lymphocyte chemoattractant | AA152885 | 1.8 |
| retinoic acid early transcript | A1451859 | 1.7 |
| insulin-like growth factor binding protein 1* | W83086 | 1.9 (20X) |
| cytochrome P450, 4A14* | AA109684 | 1.7 (12X) |
| inter -trypsin inhibitor, heavy chain* | AA062129 | 1.6 |
| CBFA2T3 identified gene homolog* | AA051563 | 1.6 |

(continued)

| Gene name | Gene ID | Fold change in old wild-type liver |
|---|---|---|
| murine mRNA for -subunit of T-cell receptor | AA265714 | 1.6 |
| S-adenosylmethionine synthetase* | W29782 | 1.5 |
| Rad23A* | AA061459 | 1.5 |
| Angiogenin* | AA237829 | 1.5 |
| histocompatibility 2, L region | AA221044 | 1.3 |
| cathepsin L* | AA174215 | 1.3 |
|  |  |  |
| fatty acid amide hydrolase* | AA260227 | -2.4 |
| CEA-related cell adhesion molecule Δ | AA245546 | -2.0 |
| glutathione S-transferase 2 | AA108370 | -1.9 |
| *Mus musculus* mRNA for N-acetylglucosamine | AA538322 | -1.7 |
| hemoglobin, adult chain 1 | AA109900 | -1.6 |
| serine protease inhibitor 2-1 | W83447 | -1.6 |
| -aminolevulinate dehydratase | AA222320 | -1.6 |
| transcription termination factor 1Δ | AA049906 | -1.5 |
| cytochrome P450 2F2 | AA220582 | -1.4 |
| esterase 31* | AA254921 | -1.3 |

References for example 1:

1. D. H. Ly, D. J. Lockhart, R A. Lerner, P. G. Schultz, Science 287, 2486 (Mar 31, 2000).
2. P. Hasty, J. Campisi, J. Hoeijmakers, H. van Steeg, J. Vijg, Science 299, 1355 (Feb 28, 2003).
3. D. Bootsma, K. Kraemer, J. Cleaver, J. H. J. Hoeijmakers, in The Genetic Basis of Human Cancer B. Vogelstein, K. W. Kinzler, Eds. (McGraw-Hill, New York, 1998) pp. 245-274.
4. K. H. Kraemer, Proc Natl Acad Sci U S A 94, 11 (1997).
5. A. M. Sijbers et al., Cell 86, 811 (1996).
6. M. van Duin et al., Cell 44, 913 (1986).
7. J. E. Cleaver, L. H. Thompson, A. S. Richardson, J. C. States, Hum Mutat 14, 9 (1999).
8. M. van Duin et al., Mutat Res 217, 83 (1989).
9. D. Bootsma, K. H. Kraemer, J. E. Cleaver, J. H. J. Hoeijmakers, in The Metabolic and Molecular Basis of Inherited Disease C. R Scriver et al., Eds. (McGraw-Hill, New York, 2001), vol. 1, pp. 677-703.
10. A. Westerveld et al., Nature 310, 425 (1984).
11. C. A. Hoy, L. H. Thompson, C. L. Mopney, E. P. Salazar, Cancer Res 45, 1737 (1985).
12. G. M. Adair et al., Embo J 19, 5552 (2000).
13. R G. Sargent et al., Nucleic Acids Res 28, 3771 (2000).
14. L. J. Niedernhofer et al., Embo J 20, 6540 (2001).
15. L. J. Niedernhofer et al., Mol Cell Biol 24 (2004).
16. Y. Matsumura, C. Nishigori, T. Yagi, S. Imamura, H. Takebe, Hum Mol Genet 7, 969 (1998).
17. A. M. Sijbers et al., J Invest Dermatol 110, 832 (1998).
18. J. Sgouros, P. H. Gaillard, R D. Wood, Trends Biochem Sci 24, 95 (Mar, 1999).
19. B. Kobe, A. V. Kajava, Curr Opin Struct Biol 11, 725 (2001).
20. M. Biggerstaff, D. E. Szymkowski, R. D. Wood, Embo J 12, 3685 (1993).

21. J. McWhir, J. Selfridge, D. J. Harrison, S. Squires, D. W. Melton, Nat Genet 5, 217 (1993).
22. G. Weeda et al., Curr Biol 7, 427 (1997).
23. M. Tian, R. Shinkura, N. Shinkura, F. W. Alt, Mol Cell Biol 24, 1200 (Feb, 2004).
24. A. de Vries et al., Nature 377, 169 (1995).
25. J. Selfridge, K. T. Hsia, N. J. Redhead, D. W. Melton, Nucleic Acids Res. 29, 4541 (2001).
26. J. Prasher et al., Embo J (in revision) (2004).
27. W. B. Cutler, E. Friedmann, E. Genovese-Stone, Am J Phys Med Rehabil 72, 219 (1993).
28. R. Roubenoff, J Nutr 129, 256S (1999).
29. Y. C. Liou et al., Nature 424, 556 (Jul 31, 2003).
30. S. Gupta, Semin Cancer Biol 10, 161 (2000).
31. C. S. Carter, M. M. Ramsey, W. E. Sonntag, Trends Genet 18, 295 (2002).
32. A. A. Toogood, Horm Res 60, 105 (2003).
33. M. J. Bartosiewicz, D. Jenkins, S. Penn, J. Emery, A. Buckpitt, J Pharmacol Exp Ther 297, 895 (2001).
34. J. C. Corton, S. P. Anderson, A. Stauber, Annu Rev Pharmacol Toxicol 40, 491 (2000).
35. T. Sakuma, R. Honma, S. Maguchi, H. Tamaki, N. Nemoto, Xenobiotica 31, 223 (2001).
36. C. Epstein, Proc. Natl. Acad Sciences 57, 327 (1967).
37. S. H. Sigal et al., Am J Physiol 276, G1260 (May, 1999).
38. Q. Huang et al., Toxicol Sci 63, 196 (2001).
39. L. J. Niedernhofer, J. S. Daniels, C. A. Rouzer, R E. Greene, L. J. Marnett, J Biol Chem 29, 29 (2003).
40. K. A. Olson, S. J. Verselis, J. W. Fett, Biochem Biophys Res Commun 242, 480 (1998).
41. H. A. Chapman, Jr., J. S. Munger, G. P. Shi, Am J Respir Crit Care Med 150, S155 (1994).
42. K. W. Moremen, P. W. Robbins, J Cell Biol 115, 1521 (1991).
43. B. Gil et al., Hepatology 24, 876 (1996).
44. U. Danesch, W. Hoeck, G. M. Ringold, J Biol Chem 267, 7185 (1992).
45. R S. Cotran, V. Kumar, S. L. Robbins, Pathologic basis of disease (W.B. Saunders Company, Philadelphia, ed. 4, 1989), pp.
46. B. D. Chang et al., Proc Natl Acad Sci USA 99, 3 89 (2002).
47. M. Kochetkova et al., Cancer Res 62, 4599 (2002).
48. R M. Popovici et al., J Clin Endocrinol Metab 86, 2653 (2001).
49. Y. Shen, E. White, Adv Cancer Res 82, 55 (2001).
50. C. K. Lee, R. G. Klopp, R. Weindruch, T. A. Prolla, Science 285, 1390 (1999).
51. S. X. Cao, J. M. Dhahbi, P. L. Mote, S. R Spindler, Proc Natl Acad Sci U S A 98, 10630 (2001).
52. P. Hasty, J. Vijg, Science 296, 1250 (2002).
53. J. N. Crawley, What's wrong with my mouse? (Wiley-Liss, New York, 2000), pp. 47-63.
54. M. M. Ouellette, L. D. McDaniel, W. E. Wright, J. W. Shay, R A. Schultz, Hum Mol Genet 9, 403 (Feb 12, 2000).
55. M. M. Ouellette, D. L. Aisner, I. Savre-Train, W. E. Wright, J. W. Shay, Biochem Biophys Res Commun 254, 795 (Jan 27, 1999).
56. J. de Boer et al., Cancer Res 59, 3489 (1999).
57. S. Parrinello et al., Nat Cell Biol 5, 741 (Aug, 2003).
58. W. Keijzer et al., Mutat Res 62, 183 (1979).
59. A. J. van Vuuren et al., Mutat Res 337, 25 (1995).
60. R J. Carter et al., J Neurosci 19, 3248 (1999).
61. W. Schul et al., Embo J 21, 4719 (2002).

## Example 2

### Early postnatal decrease of IGF-1/GH-R signaling in DNA repair deficient mice.

As mentioned in the application $Xpa^{null/null}/Csb^{G744ter/G744ter}$ mice (in this example hereafter referred to as $Xpa^{-/-}/Csb^{-/-}$ mice), in contrast to the single mutants for these genes, displayed severe growth retardation, kyphosis, ataxia, and motor dysfunction during early postnatal development [4]. We have applied functional genomic analysis in $Wt$, $Xpa^{-/-}$, $Csb^{-/-}$ and $Xpa^{-/-}/Csb^{-/-}$ 15 days old mouse livers to get insight into the underlying molecular pathways. Following total RNA isolation from four individual mouse livers (of each genotype) and subsequent hybridization to Affymetrix full mouse genome arrays (Affymetrix A version 2.0), our analysis revealed in $Xpa^{-/-}/Csb^{-/-}$ mice, but importantly not in littermate controls, the significant down regulation of genes associated with the IGF-1/GH-R growth signaling (Gh-r, IGF-1, IGFBP3, IGFBP4) as well as with the lactotroph (PrR) and thyrotroph functions (Diol and Dio2) (Figure 7). This marked decline was further accompanied by the significant increased expression of a number of genes associated with an antioxidant defense response (Gstt3, Gsr, Sodl, Hmox1, Ephoxl) (Figure 1) and the significantly decreased expression of genes associated with cytochrome P450 and NADPH oxidative metabolism (Figure 7). Among a number of significantly regulated genes displayed herein, Growth hormone receptor (Gh-R), insulin growth factor 1 (IGF-1), IGF binding protein 3 (IGFBP3), Prolactin receptor (PrR), glutathione s transferase (GSTT3), Heme oxygenase 1(Hmox1), Epoxide hydrolase 1 (Ephox1) and Apolipoprotein A4 (ApoA4) expression profiles were subjected to Real-time PCR verification (Figure 8).

The GH/IGF-1 signalling is known to decrease with advancing aging and has been shown to increase stress resistance, delay the age dependent functional decline and increase the life span of nematodes, flies and mice [5]. Interestingly, of the various genetic models that retard murine aging, four involve deficiency of pituitary endocrine action. The mutations $Prop1^{df}$ [6] and $Pit1^{dw}$ impede pituitary production of growth hormone (GH), thyroid stimulating hormone (TSH), and prolactin; reduce growth rate and adult body size; and increase adult life span by 40 to 60% [7, 8]. Small adults with similar improvement in longevity are also produced by a knockout of growth hormone receptor (GHR-KO) [9]. Without GH, the synthesis of circulating IGF-1 and plasma insulin are also suppressed as a result of enhanced sensitivity in the liver [10]. Powerful evidence for the direct role of IGF-1 signaling in the control of mammalian aging was provided by mutant mice for the IFG-1 receptor $Igf1r$ [11]. $Igf1r^{+/\cdots}$ mutant female mice exhibit minimal reduction in growth with no alterations in the age of sexual maturation, fertility, metabolism, food intake, or temperature. Importantly, the observed life extension described therein was also associated with increased tolerance of oxidative stress. The physiological relevance of these findings is markedly illustrated by the fact that Growth Hormone (Gh) and Insulin-like Growth Factor I (IGF-1) decrease with advancing aging in humans and mice [12, 13].

Despite the detrimental effects of ROS in DNA metabolism, free radicals do also participate in important physiological processes that benefit fitness, such as growth factor signal transduction [14]. Cells must, therefore, balance optimal energy production against the deleterious effects of ROS. This subtle trade off is highlighted by the various hormone deficient mouse mutants with extended life span as well as the age-dependent concomitant decrease in the expression of genes associated with the somatotroph, thyrotroph and lactotroph processes.

$Xpa^{-/-}/Csb^{-/-}$ mice are totally NER deficient mice and thus overloaded with endogenous DNA damage, an event that is present normally at later stages in life. The decreased GH/IGF1 signalling (along with the rest of the anabolic hormones described in Figure 1), may therefore reflect an adaptive response to minimize the deleterious effects from an actively ongoing metabolism, post pone growth until normoxic conditions prevail while attempting on the same time to increase the antioxidant response. Nevertheless, by doing so prematurely, the whole organismal homeostasis is readily perturbed. As a result the $Xpa^{-/-}/Csb^{-/-}$ mice suffer from severe growth defects and eventually die.

Here, our data demonstrate that DNA damage is the primary instigator of the observed hormonal response suggesting that, in mammals, the age-related decline in GH/IGF-1 growth signalling may comprise an adaptive response to the continual accumulation of endogenous DNA damage. In addition, due to the striking accelerated aging characteristics of $Xpa^{-/-}/Csb^{-/-}$ mice at both the molecular and organismal levels, the $Xpa^{-/-}/Csb^{-/-}$ mouse model may prove to be an invaluable tool to further explore the molecular basis of aging. These mouse models, and in particular the identified differentially expressed transcripts / genes, are therefore extremely suitable for use in the method of screening for compounds according to the current invention and as exemplified in the following example 4.

REFERENCES for example 2:

1. J. H. Hoeijmakers, Nature 411, 366 (2001)
2. de Boer J, Hoeijmakers JH. Carcinogenesis 21, 453-60.(2000).
3. G. J. Lithgow and M. S. Gill, Nature 421, 125 (2003)
4. Machiko Murai, Yasushi Enokido, Naoko Inamura, Masafumi Yoshino, Yoshimichi Nakatsu, Gijsbertus T. J. van der Horst, Jan H. J. Hoeijmakers, Kiyoji Tanaka, and Hiroshi Hatanaka. PNAS, 98: 13379 - 13384(2001).
5. Tatar M, Bartke A, Antebi A. Science 299:1346-51 (2003).
6. F. Muller, Ames Dwarf Mice. Science's SAGE KE 2001, tg11 (3 October 2001), http://sageke.sciencemag.org/cgi/content/full/sageke;2001/1/tg11.
7. H. M. Brown-Borg, K. E. Borg, C. J. Meliska, A. Bartke, Nature 384, 33 (1996)
8. K. Flurkey, J. Papaconstantinou, R. A. Miller, D. E. Harrison, Proc. Natl. Acad. Sci. U.S.A. 98, 6736 (2001).
9. K. T. Coschigano, D. Clemmons, L. L. Bellush, J. J. Kopchick, Endocrinology 141, 2608 (2000)
10. F. P. Dominici, S. J. Hauck, D. P. Argentino, A. Bartke, D. Turyn, J. Endocrinol. 173, 81 (2002)
11. M. Holzenberger, et al., Nature 421, 182 (2003)
12. Strasburger CJ, Bidlingmaier M, Wu Z, Morrison KM. Horm Res, 55 Suppl 2:100-5 (2001).
13. Longo VD, Finch CE. Science, 299:1342-6 (2003).
14. T. Finkel, IUBMB Life 52, 3 (2001)

Example 3

**Validity of other accelerated ageing features exhibited GM mutant mice for the normal process of ageing.**

The DNA repair disorder Cockayne syndrome (CS) encompasses a wide range of neurological abnormalities that are not found in the related disorder xeroderma pigmentosum (XP). Retinopathy is one of these features and is a valuable model organ to study the onset of a specific ageing-related condition in a quantitative fashion. The ocular pathology of CS is considered a hallmark of the disease. The phenomenon of retinal degeneration exhibited by CS mice is reminiscent to age-related macula degeneration occurring in the normal ageing population and can be a valuable model for this disease of the elderly. Similar strategies may be carried out for other ageing-related parameters in mammals.

DNA repair and genome maintenance is essential for the survival of photoreceptor cells, which are exposed to both endogenous oxidative stress and visible light and UV radiation, as illustrated below. The retina of $Csb^{m/m}$ mice of various ages was analyzed and compared with those of a mouse model for XP. $Csb$-deficient ($Csb^{G744ter/G744ter}$; TCR, deficient) and $Xpa$-deficient mice ($Xpa^{null/null}$; both GG-NER and TC-NER-deficient), as well as control mice, all in a C57B1/6 genetic background show a loss of photoreceptor cells in the retina. At 3 months of age, no difference was noticed between the genotypes, but in older $Csb^{m/m}$ mice, the ONL and the outer segment layer clearly were thinner than in wild type mice (Fig. 10A). Quantification of the number of ONL nuclei showed that the number of photoreceptor cells gradually decreased with age in $Csb^{m/m}$ mice, whereas wild type mice showed no cell loss (Fig 10B). This loss is ageing dependent. Csb-deficient mice are born with a normal number of photoreceptors, but this decreases by 10% after 5 months and to 50% after 12 months, as compared to wild type C67B16/J mice. In $Xpa$-deficient mice the decrease is slower, no significant loss at 9 months, 15% loss at 18 months and 40% at 30 months, as compared to wild type mice. The late onset of cell loss in Xpa-deficient mice is indicative of the neurodegeneration that is typical for XPA deficiency in human subjects. The cell loss was further analysed by a TUNEL assay for apoptosis on horizontal sections of retina of 3 and 11.5 months old mice. In wild type mice hardly any TUNEL positive profiles were detected. In $Csb^{m/m}$ mice clearly more positive cells were observed. These were almost exclusively located in the ONL (Fig 10C), but showed no overt regional specificity, occurring in central as well as peripheral retina.

In extrapolation: the scenario of DNA damage-related retinal degeneration observed in the $Csb$ and $Xpa$ deficient mice following a different speed is also relevant for the process of macula degeneration that occurs in an even slower pace in normal ageing and in elderly people. This example therefore illustrates and demonstrates the value and validity of the accelerated ageing in the GM mouse models as tools for understanding and influencing the process of normal ageing in humans.

Essentially similar findings were made by the inventors studying the processes of osteoporosis (for details, see example 8) and kyphosis and the onset of cachexia in the TTD mice compared with normal mouse mutants. The same holds for the early onset of infertility observed in female TTD mice when compared with wt control mice. All of the above ageing related phenotypes may be suitably applied in the method of testing compounds according to the current invention. Compounds and strategies of pharmaceutical intervention for these phenotypes, symptoms and disorders may be developed by treating or exposing these mice genetically modified mice with compounds or compositions, for instance to determine their effect on retinopathy and loss of photoreceptor cells.

Example 4

**Testing of compounds in NER deficient mice: Phenotypic effects of anti-oxidants on $Csb^{G744ter/G744ter}/$ $Xpa^{null/null}$ double mutant mice.**

This example shows the experimental set-up for screening for compounds that can inhibit, prevent and/or delay genome maintenance induced symptoms, in particular ageing-related symptoms, in mice exhibiting mutations in NER/TCR pathways, thereby illustrating the usefulness of the method of screening compounds according to the current invention.

The mouse model used in this example was the CSB$^{-/-}$/XPA$^{-/-}$ (double knock out, wherein $Csb^{G744ter/G744ter}/$ $Xpa^{null/null}$) mouse model, exhibiting a defect in GG-NER and TC-NER (XPA$^{-/-}$) and TCR in general (CSB$^{-/-}$). CSB$^{-/-}$ mice exhibit a mild ageing phenotype, a premature photoreceptor loss in the retina (example 3), while XPA mice are completely NER-defective but apart from strong cancer-predisposition and a slightly shorter life span fail to exhibit an overt phenotype to distinguish them from wild type mice. Interbreeding both mouse models however demonstrates that CSB$^{-/-}$/XPA$^{-/-}$ (double mutant) mice are born in sub-mendelian frequencies, exhibit stunted growth, kyphosis, ataxia, cachexia, osteoporosis and generally die in the third week after birth. Additionally these animals have an enhanced photoreceptor cell loss. The accumulation of oxidative DNA damage before and immediately after birth presumably negatively influences transcription and causes the premature ageing phenotype.

In order to investigate the effect of radical scavengers on the CSB/XPA double knockout mice, the effect of several compounds and compositions was monitored by the frequency of CSA/XPA dKO mice (closer to the expected mendelian frequency of 25%), an extended life-span (longer than the average three weeks for untreated dKO mice) and a delay or to some extent inhibit the premature ageing phenotype.

To obtain CSB/XPA double mutant mice the following crossing were done:

$$\text{(M) CSB}^{-/-}\text{XPA}^{+/-} \text{ x (F) CSB}^{-/-}\text{XPA}^{+/-}$$

$$\text{(M) CSB}^{-/-}\text{XPA}^{+/-} \text{ x (F) CSB}^{+/-}\text{XPA}^{-/-}$$

$$\text{(M) CSB}^{+/-}\text{XPA}^{-/-} \text{ x (F) CSB}^{+/-}\text{XPA}^{-/-}$$

$$\text{(M) CSB}^{+/-}\text{XPA}^{-/-} \text{ x (F) CSB}^{+/-}\text{XPA}^{-/-}$$

From these crossings CSB$^{-/-}$XPA$^{-/-}$ mice were born with a frequency of 9%, whereas the expected Mendelian frequency is 25%.

16 pregnant females received an osmotic pump, 7 x 30 mm, subcutaneously implanted under the skin on the back, for continuous release of Phosphate Buffered Saline (control) or 5% D-mannitol dissolved in Phosphate Buffered Saline. The offspring were genotyped following normal procedures (tail clipping and genomic DNA analysis by Southern blot analysis or PCR amplification) and monitored for life span.

Figures 9A and 9B show the increase in frequency of birth of XPA/CSB double KO mice after treatment with hydroxyl scavenger D-mannitol (experiment 1) and the increase in survival after birth (life span) respectively. Comparable results were obtained in experiments in which 2% D-Mannitol was administered to drinking water. Comparable results were also obtained with another scavenger: proline, which was equally effective in increasing the frequency of survival of dKO pups after birth.

Hence mannitol and proline may be used for the manufacture of a medicament for the treatment of the consequences of ageing and/or genome maintenance disorders. Moreover, mannitol or proline may be used for the manufacture of a medicament for the treatment of the consequences of ischemia, and reperfusion damage of (transplanted) organs and tissues. The medicament may also comprise foodcompositions with elevated levels of mannitol, proline and other anti-oxidants or radical scavengers.

This experiment illustrates the use of the method for screening of compounds according to the current invention, which uses animal models comprising mutations in NER genes and with impaired genome maintenance capability, preferably yielding a premature ageing phenotype, and positively identifies compounds capable of inhibiting, preventing or delaying premature ageing phenotypes.

Example 5

**Generation of a conditional *Xpa^conditional/null* mutant mouse.**

Example 2 describes a detailed phenotypical characterization of the Csb$^{m/m}$Xpa$^{-/-}$ mouse model, including liver transcriptome analysis, which has given new insights as to how a DNA repair defect affects the IGF/GH axis and induces a systemic response that is also occurring during natural aging. These animals provide a good model for the quick screening of compounds. At the same time, knocking out genes in one specific tissue, will make it possible to study the effects of compounds on age-related tissue pathology. Here, we describe the generation of a conditional Xpa mouse that allows (Cre-recombinase-mediated) tissue and time specific inactivation of the Xpa gene.

In order to knock out the Xpa gene in a tissue-specific and/or time-dependent manner, we have generated a targeting construct (Fig. 11A) in which exon 4 is fused to the mouse cDNA (including a synthetic polyA sequence), followed by a hygromycin (HYGRO) selectable marker (Fig. 11A). LoxP sites were introduced in exon 3 and downstream of the Hygro marker to allow Cre-mediated excision of the cDNA and selectable marker, ultimately leading to an Xpa allele that lacks exon 4. In order to visualize inactivation of the conditional Xpa allele, we inserted a LacZ-GFP fusion marker gene, preceded by a splice acceptor (SA) and an internal ribosomal entry sequence (IRES) to allow transcription and translation of the marker. A multiple reading frame insertion (Murfi), containing stopcodons in 3 reading frames and placed between the SA and IRES, prevents any translation of potential readthrough transcripts from the Hygro marker gene.

First, we transfected Xpa knockout ES cells with the conditional Xpa construct and obtained ES clones with one knockout and one targeted allele at a targeting frequency of 20% (Fig. 11B). Xpa$^{-/-}$ ES. cells are extremely sensitive to UV, and as shown in Fig. 11C, replacement of a knockout allele by a conditional Xpa allele is able to restore UV sensitivity to wild type (wt) levels, proving functionality of the XPA conditional construct. Next, we transfected Xpa$^{c/+}$ ES cells with a plasmid containing a Cre-recombinase. Southern blot analysis of individual clones revealed that, Cre recombinase was able to excise the floxed Xpa sequences (Fig. 11D). Finally, we transfected ES line IB 10 with the conditional Xpa construct and heterozygous Xpa$^{c/+}$ ES cells were obtained at a frequency of 17% (Fig. 11 E). After excluding chromosomal abnormalities and additional random integrations (data not shown), two different Xpa$^{c/+}$ ES cell clones were used for blastocyst injections. Germ line transmission was obtained for both clones. Heterozygous offspring (Xpa$^{c/+}$) from matings between chimeric males and C57BL/6 female mice was used for further breedings.

We bred Xpa$^{c/-}$ mice with the CagCre mouse line, which express Cre recombinase immediately after conception (1). Southern blot analysis of DNA from E10.5 and E13.5 embryos revealed that Cre-recombinase had efficiently recognized and excised the floxed Xpa DNA (Fig. 12A). When Cre recombinase excises the floxed XPA DNA, we should be able to detect expression of the LacZ marker. The isolated Xpa$^{cr/c}$, Xpa$^{cr/cr}$, Xpa$^{c/-}$ and Xpa$^{+/-}$ embryos were subjected to a LacZ staining. As expected, both Xpa$^{cr/c}$ and Xpa$^{cr/cr}$ embryos displayed LacZ staining, whereas both Xpa$^{c/-}$ and Xpa$^{+/-}$ did not (figure 12C). It has to be noted, however, that the expression was not very high. But, since the LacZ is expressed under the endogenous promoter of the XPA gene and XPA itself is normally not very highly expressed (2), we did not expect expression to be extremely high. These results show that Cre recombinase is able to recombine out the floxed piece of XPA DNA, after which the LacZ marker is expressed.

We intercrossed Csb$^{+/m}$Xpa$^{+/-}$ with Xpa$^{c/+}$, so that after multiple breedings, Csb$^{m/m}$Xpa$^{c/-}$ animals were generated (figure 12A). In contrast to Csb$^{m/m}$Xpa$^{-/-}$ mice, these animals were viable and developed normally, without any aberrant phenotype. The oldest Csb$^{m/m}$Xpa$^{c/-}$ mouse is now 121 weeks of age. These results prove that the XPA conditional construct is also functional in vivo. From comparable breedings as described above, we isolated wt and Xpa$^{cr/-}$ ES blastocysts. Even by using a confocal microscope, we could not detect any GFP signal above wt levels (data not shown). Additionally, we isolated three independent wt and Xpa$^{cr/-}$ MEF cell lines from day 13.5 embryos. These MEFs were subjected to FAKS analysis and also here we were not able to detect a GFP signal (data not shown). We performed a LacZ staining on the same cell lines, which showed that even though there was full recombination of the XPA conditional allele, we could only detect about 10 % of LacZ positive cells (figure 12D). These results together show that there is expression of the LacZGFP fusion marker, but not at very high levels and therefore it is difficult to detect.

Xpa$^{cr/r}$ should be sensitive to UV, since they are knock out for XPA. Three independent Xpa$^{cr/-}$ MEF lines were shown to be very sensitive to UV when compared to three independent wt MEF lines (figure 12E). The levels of sensitivity of the Xpa$^{cr/-}$ cells are similar to the sensitivity for UV previously shown for Xpa$^{-/-}$ MEFs .

One of the final, most important checks, was to determine if the recombined allele gives rise to a functional knock out phenotype in the mouse. We tested this by breeding the Xpa$^{cr/-}$ mouse into a CSB deficient background. As mentioned in example 2 Csb$^{m/m}$Xpa$^{-/-}$ mice are runted, fail to thrive and die within three weeks. Csb$^{m/m}$Xpa$^{cr/-}$ animals should have the exact same phenotype. By genotyping we confirmed the XPA status of each CSB deficient animal (figure 13A). As predicted, Csbm$^{m/m}$Xpa$^{cr/-}$ animals had the exact same phenotype as Csb$^{m/m}$Xpa$^{-/-}$ animals (figure 13B). We recorded weight, analyzed the walking pattern and determined their lifespan. In each of these aspects the Csb$^{m/m}$Xpa$^{cr/-}$ mice were the same as previously observed for Csb$^{m/rn}$Xpa$^{-/-}$ mice (data not shown), which proves that the recombined XPA allele gives rise to a functional XPA knock out allele in vivo.

References for example 5:

1. Sakai, K. and J. Miyazaki (1997). "A transgenic mouse line that retains Cre recombinase activity in mature oocytes irrespective of the cre transgene transmission." Biochem Biophys Res Commun 237(2): 318-24.
2. Layher, S. K. and J. E. Cleaver (1997). "Quantification of XPA gene expression levels in human and mouse cell lines by competitive RT-PCR" Mutat Res 383(1): 9-19.

Example 6

**Neurological phenotype of a brain specific conditional *Csb*$^{G744ter/G744ter}$/*Xpa*$^{null/null}$ double mutant mice.**

As shown in example 5, we have generated a conditional XPA mouse. By combining this mouse with the appropriate Cre-recombinase mouse, we can knock out the XPA gene in a time-dependent and tissue-specific fashion. Previously, Murai and coworkers had observed increased apoptosis in the cerebellum that coincided with the ataxia observed in these animals. Moreover, our transcriptome analysis of 15 day old Csb$^{m/m}$Xpa$^{c/-}$ livers revealed a systemic response, involving the IGF-1/GHR axis, which involves the hypothalamus, that closely mimicked aging. Additionally, we had found increased apoptosis in the retina of these animals. Therefore, the brain is to be a perfect candidate tissue to study.

We used mice expressing the *CamKIIα-Cre* recombinase (courtesy of S. Zeitlin). This Cre-recombinase is under the control of the CamKIIα promoter and the transgenic line we received, L7ag#13, expressed the Cre-recombinase throughout the adult brain with high levels in all forebrain structures and moderate levels in the cerebellum (1). The highest levels of recombination were detected after postnatal day 5. We intercrossed these Cre recombinase animals with *Csb^{m/m}* and *Xpa^{cl-}* animals to obtain *Csb^{m/m}Xpa^{cl-}-CamKIICre^+* animals. We weighed the animals each month together with *Wt, Csb^{m/m}* and *Xpa^{cl-}-CamKIICre^+* littermates. Initially, the animals were normal in size and bodyweight (Figure 14A). However, at the age of 6 months, we first noticed a difference in bodyweight between *Csb^{m/m}Xpa^{cl-}-Cam-KIICre^+* animals compared to the control littermates, which became more evident after the age of 8 months (figure 14B) It is known for *Csb^{m/m}* animals that their body weight is lower than Wt animals starting at 13 weeks of age. Yet, the weight of *Csb^{m/m}Xpa^{cl-}-CamKIICre^+* animals was even lower than that of *Csb^{m/m}* animals above the age of 8 months. At first we had also done footprint analysis as previously done for the *Csb^{m/m}Xpa^{-/-}* mouse, but these revealed no obvious abnormalities. We did, however, observe that during handling of the *Csb^{m/m}Xpa^{cl-}-CamKIICre^+* animals, they displayed anxiety-like behaviour. To further explore this, we tested them for 30 minutes in an open field along with *Wt, Csb^{m/m}* and *Xpa^{cl-}-CamKIICre^+* littermates at 3 and 6 months of age, n=6. The movement plot, which is derived by the computer after 30 minutes in the open field, already clearly shows that the *Csb^{m/m}Xpa^{cl-}-CamKIICre^+* move much less in the center. We calculated the anxiety ratio (AR) by dividing the distance spent in the center by the total distance. Movement time was the same for each mouse. A low AR is indicative of anxiety-like behaviour. The AR of *Csb^{m/m}Xpa^{cl-}-CamKIICre^+* animals was much lower than that of the control groups at 3 months of age ($p < 0.05$), and became even lower at 6 months of age ($p < 0.001$) (Figure 14C). This indeed shows that *Csb^{m/m}Xpa^{cl-}-CamKIICre^+* animals display anxiety-like behaviour that becomes worse in time. To relate this anxiety-like phenotype to a specific region of the brain, we stained the brains of *Wt, Csb^{m/m}, Xpa^{cl-} CamKIICre^+* and *Csb^{m/m}Xpa^{cl-}-CamKIICre^+* animals for LacZ, since a LacZ marker is expressed after recombination of the XPA gene. As expected, we only observed staining in the brains *Xpa^{cl-}-CamKIICre^+* and *Csb^{m/m}Xpa^{cl-}-CamKIICre^+* animals (figure 14D) and not of *Wt* and *Csb^{m/m}* animals (data not shown). This staining was purely restricted to the Lateral Septum (LS) that is situated near the hippocampus and has a role in anxiety-like behaviour. Furthermore, the staining seemed less in the *Csb^{m/m}Xpa^{cl-}-CamKIICre^+* animals and the LS was very irregular, as if cells were missing.

We repeated the Open Field test at an age of 50-60 weeks and the AR was still low as expected (data not shown) for most *Csb^{m/m} Xpa^{cl-}-CamKIICre^+* animals. However, three of the oldest *Csb^{m/m}Xpa^{cl-}-CamKIICre^+* animals (about 15 months) showed a reduced overall motility and a behaviour that resembled seizures. Even more striking, they appeared to have priapism (Fig 14E) and the maximum lifespan of these animals was reduced to about 16 months. Currently, we are following the *Csb^{m/m}Xpa^{cl-}-CamKIICre^+* animals in time and recording aberrant behaviour and lifespan.

References for example 6:

1. Dragatsis, I. and S. Zeitlin (2000). "CaMKIIalpha-Cre transgene expression and recombination patterns in the mouse brain." Genesis 26(2): 133-5.

Example 7

**Validity of the TTD mouse as a model for accelerated bone ageing.**

Age-related bone loss in the human population is well documented In postmenopausal women, accelerated loss of predominantly trabecular bone, due to increased number and activity of osteoclasts, is followed by a slow continuous phase of bone loss in which the density of trabecular bone reduces and cordial bone thins, leading to an increased fracture risk (1-3). In men age-related bone loss is also present but less pronounced as the drop in oestrogen levels responsible for rapid bone loss in females is absent. In addition, men have more pronounced periosteal apposition, i.e. bone formation on the outside of the bone (periost) (4).

Trichothiodystrophy (TTD) is a rare, autosomal recessive DNA repair disorder, in which patients present an array of symptoms, including photosensitivity, ichthyosis, brittle hair and nails, impaired intelligence, decreased fertility, short stature, an aged appearance and a reduced life span (5-7). In addition, skeletal abnormalities, like osteopenia together with osteosclerosis in the axial skeleton and proximal limbs and also axial and cranial osteosclerosis and demineralisation in the distal bones and have been described (6,8-15). We have generated a mouse model in which we mimicked a causative point mutation identified in the XPD gene of a photosensitive TTD patient (TTD1 Bel) (16). Previous work has shown that the phenotype of TTD mice very much resembles the symptoms of patients, including the presence of premature ageing features like skeletal changes (17). When crossed to a completely NER deficient XPA mouse (showing no features of premature aging itself), the premature aging features of the TTD mouse are dramatically enhanced.

We studied the changes in bone with ageing in both male and female wild type mice and premature ageing TTD mice in order to get insight into the processes of age-related skeletal changes and to assess the significance of DNA repair/basal transcription herein. This thorough analysis showed that the TTD mouse model is a very good model to study osteoporosis.

First, cortical bone analysis in female wild type mice revealed a progressive decline in 3D thickness distribution with age. Comparably, male wild type mice showed a decrease in 3D thickness distribution with age. Already at 52 weeks of age, TTD females reached the level that wild type females only reached at 91 weeks of age. At 52 weeks of age, the tibiae of TTD males have thinned even more than the tibiae of 91-week-old wild type males. Thus, cortical thinning occurs earlier in TTD mice than in wild type mice (Fig. 15).

Secondly, in wild type females diaphysial bone volume gradually decreased with age, only reaching significance at 104 weeks of age when compared to 26 week old wild type females. In TTD females, after 39 weeks bone volume rapidly decreased and was significantly lower than in age-matched wild type mice. Already at 52 weeks of age TTD females reached a similar bone volume as 91 and 104 weeks old wild type females. In line with the bone volume, wild type female tibiae maintained their cortical thickness up to 78 weeks of age and showed a decrease thereafter while TTD female tibiae already showed a rapid drop in cortical thickness after 39 weeks of age Bone volume in male wild type mice showed a clear drop after 39 weeks of age. From 52 weeks onward bone volume remained stable in wild type males. Unlike females, wild type males showed no significant decrease in cortical thickness with ageing. As in wild type animals, cordial thickness was lower in 26 and 39-week-old TTD males than in age-matched TTD females. With ageing, TTD males showed a similar pattern in bone volume and cortical thickness changes as wild type males albeit having significantly lower values than wild types at 78 weeks of age (Fig. 16 A-D).

Thirdly, wild type females showed a progressive increase in bone perimeter throughout life that reaches significance at 91 and 104 weeks of age compared to 26 week old females. In contrast, TTD females lacked this increase in perimeter showing a constant perimeter at all ages. After 39 weeks the perimeter decreased in both wild type and TTD males, but only wild type males showed an increase in perimeter at old age (Fig. 16 E+F).

Taken together, the TTD mouse is a valuable mouse model to study compounds that can counteract osteoporosis. Furthermore, since the TTD/XPA double mutant has an accelerated TTD phenotype, bone specific deletion of the XPA gene in the TTD mouse will result in accelerated osteoporosis, which than can be counteracted by chemical intervention. To this purpose, we have generated an XPA conditional mouse (XPAc) model (for details, see example 6). By crossing this to tissue-specific Cre recombinase mouse, we can knock-out the XPA gene in our tissue of interest, after which a LaczGFP marker is expressed to show where recombination has occurred. To study accelerated osteoporosis in TTD/XPAc mice, we will cross them to either osteoblast-specific Cre mice, in which Cre is under the control of the collagen Ia1 promoter (18), or osteoclast-specific Cre mice, in which Cre is under control of the cathepsin K promoter (19). Additionally we would like to use the chondrocyte-specific Cre, under control of the Co12a1 promoter (20), expressed in the cartilage and fat-specific Cre mice, with Cre under control of the aP2 promoter (21), expressed in white and brown adipose tissue. Together these mouse models will help us gain further insight into the mechanism of osteoporosis and will provide faster screening methods for osteoporosis medicine.

References for example 6

1. Riggs, B.L., S. Khosla, and L.J. Melton, 3rd, Sex steroids and the construction and conservation of the adult skeleton. Endocr Rev, 2002. 23(3): p. 279-302.

2. Seeman, E., Pathogenesis of bone fragility in women and men. Lancet, 2002. 359(9320): p. 1841-50.

3. Kawaguchi, H., et al., Independent impairment of osteoblast and osteoclast differentiation in klotho mouse exhibiting low-turnover osteopenia. J Clin Invest, 1999.104(3): p. 229-37.

4. Seeman, E., During aging, men lose less bone than women because they gain more periosteal bone, not because they resorb less endosteal bone. Calcif Tissue Int, 2001. 69(4): p. 205-8.

5. Bootsma, D., et al., Nucleotide excision repair syndromes: xeroderma pigmentosum, Cockayne syndrome and trichothiodystrophy, in The genetic basis of human cancer, B. Vogelstein and K.W. Kinzler, Editors. 1998, McGraw-Hill: New York. p. 245-74.

6. Itin, P.H., A. Sarasin, and M.R. Pittelkow, Trichothiodystrophy: update on the sulfur-deficient brittle hair syndromes. J Am Acad Dermatol, 2001. 44(6): p. 891-920; quiz 921-4.

7. Botta, E., et al., Analysis of mutations in the XPD gene in Italian patients with trichothiodystrophy: site of mutation correlates with repair deficiency, but gene dosage appears to determine clinical severity. Am J Hum Genet, 1998.

63(4): p. 1036-48.

8. Wakeling, E.L., et al., Central osteosclerosis with trichothiodystrophy. Pediatr Radiol, 2004. 34(7): p. 541-6.

9. Toelle, S.P., E. Valsangiacomo, and E. Boltshauser, Trichothiodystrophy with severe cardiac and neurological involvement in two sisters. Eur J Pediatr, 2001. 160(12): p. 728-31.

10. Kousseff, B.G. and N.B. Esterly, Trichothiodystrophy, IBIDS syndrome or Tay syndrome? Birth Defects Orig Artic Ser, 1988. 24(2): p. 169-81.

11. Przedborski, S., et al., Trichothiodystrophy, mental retardation, short stature, ataxia, and gonadal dysfunction in three Moroccan siblings. Am J Med Genet, 1990. 35(4): p. 566-73.

12. Civitelli, R., et al., Central osteosclerosis with ectodermal dysplasia: clinical, laboratory, radiologic, and histopathologic characterization with review of the literature. J Bone Miner Res, 1989. 4(6): p. 863-75.

13. Chapman, S., The trichothiodystrophy syndrome of Pollitt. Pediatr Radiol, 1988. 18(2): p. 154-6.

14. Price, V.H., et al., Trichothiodystrophy: sulfur-deficient brittle hair as a marker for a neuroectodermal symptom complex. Arch Dermatol, 1980. 116(12): p. 1375-84.

15. McCuaig, C., et al., Trichothiodystrophy associated with photosensitivity, gonadal failure, and striking osteosclerosis. J Am Acad Dermatol, 1993. 28(5 Pt 2): p. 820-6.

16. de Boer, J., et al., A mouse model for the basal transcription/DNA repair syndrome trichothiodystrophy. Mol Cell, 1998. 1(7): p. 981-90.

17. de Boer, J., et al., Premature aging in mice deficient in DNA repair and transcription. Science, 2002. 296(5571): p. 1276-9.

18. Castro, C. H., J. P. Stains, et al. (2003). "Development of mice with osteoblast-specific connexin43 gene deletion." Cell Commun Adhes 10(4-6): 445-50.

19. Chiu, W. S., J. F. McManus, et al. (2004). "Transgenic mice that express Cre recombinase in osteoclasts." Genesis 39(3): 178-85.

20. Ovchinnikov, D. A., J. M. Deng, et al. (2000). "Co12a1-directed expression of Cre recombinase in differentiating chondrocytes in transgenic mice." Genesis 26(2): 145-6.

21. Barlow, C., M. Schroeder, et al. (1997). "Targeted expression of Cre recombinase to adipose tissue of transgenic mice directs adipose-specific excision of loxP-flanked gene segments." Nucleic Acids Res 25(12): 2543-5.

Example 8

**Increased photoreceptor loss in the Csb mouse after exposure to ionizing radiation.**

As shown in example 3, $Csb^{m/m}$ mice have an age-related loss of photoreceptor cells in the retina. To examine whether oxidative DNA damage could be involved in the retinal degeneration in $Csb^{m/m}$ mice, we tested IR sensitivity, which is known to induce DNA damage of various types, including oxidative DNA damage, in the retina of $Csb^{m/m}$ mice

We performed whole body gamma-ray irradiations at a low dose (10 Gy), and measured the effects by counting apoptotic cells in sections stained by apoptosis assays. The effects of irradiation on apoptosis in the retina of $Csb^{m/m}$ and wt mice are summarized in Table 1. In wild type retina apoptosis levels were low and no significant increase was noticed after irradiation. In the retina of $Csb^{m/m}$ animals apoptosis in ONL and INL was increased by the irradiation, indicating that these retinal cells in $Csb^{m/m}$ mice are hypersensititve to ionizing radiation. The findings in example 3 and 9 show that the retina of the $Csb^{m/m}$ mouse is a sensitive read-out system for oxidative DNA damage. This makes it possible to study the effect of intervention on photoreceptor loss in the retina of $Csb^{m/m}$ mice both with and without exposure to ionizing radiation. As mentioned in example 4, CSB/XPA double mutant mice have accelerated photoreceptor cell loss, a premature aging phenotype, which indicates that the retina of these mice provides a good model organ to study the effect of intervention that is capable of preventing or delaying premature ageing phenotypes.

Table 1: Effect of genotype and irradiation on apoptosis in the retina

| | | X-ray irradiation | | *1-test* P | Two-way ANOVA *p* interaction |
|---|---|---|---|---|---|
| | | **0 Gy** | **10 Gy** | | |
| Wild type | ONL | $0.6 \pm 0.3$ | $0.8 \pm 0.4$ | 0.53 | <0.001 |
| $Csb^{m/m}$ | | $9.5 \pm 2.5$ | $20.3 \pm 4.2$ | 0.002 | |
| | *t-test p* | *0.0003* | *0.0004* | | |
| Wild type | INL | $0.11 \pm 0.12$ | $0.11 \pm 0.12$ | 1 | 0.003 |
| $Csb^{m/m}$ | | $0.4 \pm 0.3$ | $1.5 \pm 0.7$ | 0.024 | |

(continued)

| | | X-ray irradiation | | 1-test P | Two-way ANOVA *p* interaction |
|---|---|---|---|---|---|
| | | **0 Gy** | **10 Gy** | | |
| | *t-test p* | *0.12* | *0.01* | | |
| Wild type | GL | 0.04 ± 0.10 | 0.04 ± 0.10 | 1 | 0.058 |
| *Csb^{m/m}* | | 0.0 ± 0.0 | 0.46 ± 0.57 | 0.15 | |
| | *t-test p* | *0.36* | *0.18* | | |

<u>Example 9:</u>

**Similarity of accelerated aging in *Csb^{m/m}/Xpa^{-/-}* and *Ercc1^{-/-}* mutant mice and natural aging.**

It has been proposed that, in order to show whether a mouse mutant represents a valid model for aging, one should list those phenotypic features shared by the mutant and naturally aged mice or else define a set of aging traits and determine how many of these are also seen in the mutant mouse (1). Although, extremely short-lived mice display a number of age-related features, we sought to implement a full mouse genome approach to gain unbiased insight into their relevance to naturally aging mice:

**_Csb^{m/m}/Xpa^{-/-}_: mouse model**

To investigate whether and to which extent the changes in expression profiles in the liver of *Csb^{m/m}/Xpa^{-/-}* mice parallel the expression patterns in naturally aged mice, we classified all meaningful expression changes (those probe sets representing the 1865 genes) as having increased or decreased expression. We next weighed this data set against data sets obtained from a comparison of the full mouse genome transcriptome of 16-, 96- and 130-week old *wt* C57B1/6 mice (n=4) with that of 8-week old *wt* C57B1/6 mice (n=4). This approach assigned a correlation coefficient (Pearson's r) that is directly proportional to the fraction of genes in the 16-, 96- and 130-week old *wt* animal that change in a similar direction as in *Csb^{m/m}/Xpa^{-/-}* mice. Of note, no similarity could be identified between the DNA repair mutant mice and 16-week old *wt* mice (Pearson's r= - 0.26). Importantly, we identified a positive correlation between *Csb^{m/m}/Xpa^{-/-}* and 96-week old mice (r= 0.15), which was substantially further pronounced when a comparison was made with the 130-week old *wt* mouse group (r= 0.40, see inset Fig. 17). Importantly, these findings were equivalent when we applied the same approach over the whole mouse transcriptome including all Affymetrix probe sets with signals above the detection cut-off value, thus avoiding any initial preselection or introduction of bias. A calculation of the probability of observing a random correlation as strong as that found in the actual data set excluded the possibility that the observed similarity between *Csb^{m/m}/Xpa^{-/-}* mice and 96- and 130-week old *wt* mice were random events (Fisher exact test p ≤ 0.015)
The marked overall resemblance between the transcriptome of 15-day old *Csb^{m/m}/Xpa^{-/-}* and 130-week old *wt* livers, prompted us to examine whether the previously identified statistically significant over-represented biological processes in the liver of *Csb^{m/m}/Xpa^{-/-}* mice were also shared by naturally aged mice. This approach led us to identify a strikingly high degree of similarity between the short-lived, DNA-repair deficient mice and the 130-week old mice in the transcriptional profiles of those genes associated with the GH/IGF1 axis, the oxidative metabolism (i.e. glycolysis, Krebs and oxidative phosphorylation), the cytochrome P450 electron transport and the peroxisomal biogenesis (Table 1). Importantly, however, the dampening of both the somatotroph axis and the oxidative metabolism was more pronounced (in terms of the number of identified genes, Suppl. Table S4) in 130-week old mice compared to that of 96-week old mice and *Csb^{m/m}/Xpa^{-/-}* mice, while it was virtually absent in 16-week old mice. The marked resemblance of the genome-wide transcriptome of 2-week old *Csb^{m/m}/Xpa^{-/-}* mice to that of old (> 90 weeks) rather than young (8 weeks) wt animals as well as the early onset of a transcriptional response associated with normal aging unmistakably points to premature aging in the *Csb^{m/m}/Xpa^{-/-}* mouse model.

**Table 1. Analysis of the *Csb_{m/m}/Xpa_{-/-}* liver transcriptome** Significant gene expression changes identified in the livers of *Csb_{m/m}/Xpa_{-/-}*, *Csb_{m/m}* and *Xpa_{-/-}* mice compared to livers from wt littermate controls.

| Code | Title | Symbol | *Csb_{m/m}Xpa_{-/-}* | | *Xpa_{-/-}* | | *Csb_{m/m}* | |
|---|---|---|---|---|---|---|---|---|
| *The IGF-1/GH growth axis* | | | FC | P-value | FC | P-value | FC | P-value |
| 1448556_at | prolactin receptor | Prlr | -2.03 | 0.000 | -1.3 | 0.10 | -1.10 | 0.66 |

(continued)

| Code | Title | Symbol | $Csb_{m/m}Xpa_{-/-}$ | | $Xpa_{-/-}$ | | $Csb_{m/m}$ | |
|---|---|---|---|---|---|---|---|---|
| *The IGF-1/GH growth axis* | | | FC | P- | FC | P-value | FC | P- |
| 1419519_at | insulin-like growth factor 1 | Igfl | -2.13 | 0.000 | -1.1 | 0.16 | -1.13 | 0.19 |
| 1422826_at | IGF-binding protein, acid labile subunit | Igfals | -2.36 | 0.000 | 1.13 | 0.18 | 1.06 | 0.21 |
| 1421991_a_at | IGF-binding protein 4 | Igfbp4 | -1.74 | 0.000 | -1.1 | 0.82 | -1.16 | 0.39 |
| 14S8268_8_at | IGF-binding protein 3 | Igfbp3 | -1.44 | 0.001 | -1.1 | 0.25 | 1.06 | 0.59 |
| 1422826_at | IGF-binding protein, acid labile subunit | Igfals | -236 | 0.000 | 1.06 | 0.53 | 1.13 | 0.26 |
| 1417962_s_at | growth hormone receptor | Ghr | -1.53 | 0.000 | 1.03 | 0.15 | 1.10 | 0.05 |
| 1425458_a_at | growth factor receptor bound protein 10 | Grb10 | 1.84 | 0.000 | 1.11 | 0.59 | 1.24 | 0.19 |
| 1427777_x_at | fibroblast growth factor receptor 4 | Fgfr4 | -1.32 | 0.009 | -1.1 | 0.71 | -1.19 | 0.11 |
| 1421841_at | fibroblast growth factor receptor 3 | Fgfr3 | -1.43 | 0.001 | -1.3 | 0.03 | -1.12 | 0.87 |
| 1450869_at | fibroblast growth factor 1 | Fgf1 | -1.38 | 0.003 | -1.2 | 0.09 | -1.01 | 0.84 |
| 1435663_at | estrogen receptor 1 (alpha) | Esr1 | -1.91 | 0.001 | -1.1 | 0.32 | -1.31 | 0.12 |
| 1417991_at | deiodinase, iodothyronine, type I | Dio1 | -2.12 | 0.000 | 1 | 0.28 | -1.04 | 0.28 |
| *Carbohydrate metabolism* | | | | | | | | |
| 1423644_at | aconitase 1 | Aco1 | -1.26 | 0.002 | 1.05 | 0.50 | -1.00 | 0.96 |
| 1422577_at | citrate synthase | Cs | -1.28 | 0.006 | 1.16 | 0.20 | -1.03 | 0.20 |
| 1419146_a_at | glucokinase | Gck | -6.59 | 0.004 | 1.16 | 0.28 | -1.01 | 0.66 |
| 1424815_at | glycogen synthase 2 | Gys2 | 1.78 | 0.000 | -1 | 0.22 | -1.01 | 0.30 |
| 1459522_s_at | glycogenin 1 | Gyg1 | 1.26 | 0.014 | 1.05 | 0.153 | 1.03 | 0.364 |
| 1417741_at | liver glycogen phosphorylase | Pyg1 | -1.42 | 0.000 | 1.02 | 0.50 | 1.06 | 0.03 |
| *Steroid metabolism and biosynthesis* | | | | | | | | |
| 1417871_at | hydroxysteroid (17-beta) dehydrogenase 7 | Hsd17b7 | -1.50 | 0.000 | -1.3 | 0.14 | -1.32 | 0.13 |
| 1449038_at | hydroxysteroid 11-beta dehydrogenase I | Hsd11b1 | -1.32 | 0.001 | 1.1 | 0.34 | 1.01 | 0.71 |
| 1460192_at | oxysterol binding protein-like 1A | Osbp11a | -1.38 | 0.000 | -1.1 | 0.50 | -1.08 | 0.52 |
| 1427345_a_at | sulfotransferase family 1A, member 1 | Sult1a1 | -1.29 | 0.002 | -1.1 | 0.69 | -1.05 | 0.59 |

(continued)

| Code | Title | Symbol | $Csb_{m/m}Xpa_{-/-}$ | | | $Xpa_{-/-}$ | | $Csb_{m/m}$ | |
|------|-------|--------|------|------|------|------|------|------|------|
| The IGF-1/GH growth axis | | | FC | P- | FC | P-value | FC | P- | |
| 1419528_at | sulfotransferase, hydroxysteroid preferring 2 | Sth2 | -1.61 | 0.000 | -1.1 | 0.92 | -1.20 | 0.26 |
| Cytochrome | (Cyt) P450, NADH-and NADPH-dependent Oxidative metabolism | | | | | | | | |
| 1418821_at | Cyt. P450, family 2, subfam. a, polyp. 12 | Cyp2a12 | -1.51 | 0.000 | 1.06 | 0.21 | -1.06 | 0.33 |
| 1422257_s_at | Cyt. P450, family 2, subfam. b, polyp. 10 | Cyp2b10 | -2.81 | 0.001 | -1.4 | 0.75 | -1.39 | 0.75 |
| 1449479_at | Cyt. P450, family 2, subfam. b, polyp. 13 | Cyp2b13 | -2.24 | 0.002 | -1.2 | 0.77 | -1.27 | 0.98 |
| 1425645_s_at | Cyt. P450, family 2, subfam. b, polyp. 20 | Cyp2b20 | -2.94 | 0.001 | **-1.3** | 0.95 | -1.42 | 0.36 |
| 1419590_at | Cyt. P450, family 2, subfam. b, polyp. 9 | Cyp2b9 | -1.55 | 0.000 | -1.1 | 0.78 | -1.04 | 0.49 |
| 1417651_at | Cyt. P450, family 2, subfam. c, polyp. 29 | Cyp2c29 | -1.59 | 0.004 | -1.4 | 0.09 | -1.41 | 0.09 |
| 1440327_at | Cyst P450, family 2, subfam. c, polyp. 70 | Cyp2c70 | -2.58 | 0.001 | -1.2 | 0.15 | -1.23 | 0.58 |
| 1448792_a_at | Cyst P450, family 2, subfam. f, polyp. 2 | Cyp2f2 | -2.36 | 0.001 | 1.76 | 0.05 | 1.30 | 0.43 |
| 1417532_at | Cyt. P450, family 2, j, polyp. 5 subfam. | Cyp2j5 | -3.08 | 0.000 | -1.5 | 0.10 | -1.31 | 0.36 |
| 1418767_at | Cyt. P450, family 4, subfam, f, polyp. 13 | Cyp4f13 | -1.67 | 0.008 | -1.1 | 0.46 | -1.40 | 0.36 |
| 1419559_at | Cyt. P450, family 4, subfam. f, polyp. 14 | Cyp4f14 | -3.58 | 0.000 | 1.57 | 0.06 | -1.00 | 0.92 |
| 1417070_at | Cyt. P450, family 4, subfam. v, polyp. 3 | Cyp4v3 | -1.41 | 0.001 | 1.04 | 0.40 | -1.09 | 0.81 |
| 1422100_at | Cyt. P450, family 7, subfam. a, polyp. 1 | Cyp7a1 | -2.36 | 0.006 | 1.14 | 0.86 | -1.39 | 0.24 |
| 1417429_at | flavin containing monooxygenase 1 | Fmo1 | -1.38 | 0.000 | 1.04 | 0.23 | 1.00 | 0.57 |
| 1422904_at | flavin containing monooxygenase 2 | Fmo2 | -4.51 | 0.008 | -2.2 | 0.40 | -2.05 | 0.49 |
| 1449525_at | flavin containing monooxygenase 3 | Fmo3 | -14.19 | 0.004 | -2 | 0.84 | -2.34 | 0.59 |

(continued)

| Code | Title | Symbol | $Csb_{m/m}Xpa_{-/-}$ | | | $Xpa_{-/-}$ | | $Csb_{m/m}$ | |
| --- | --- | --- | --- | --- | --- | --- | --- | --- | --- |
| **The IGF-1/GH growth axis** | | | FC | P- | FC | P-value | FC | | P- |
| 1423908_at | NADH dehydrogenase (ubiquinone) | Fe-S protein 8 Ndufs8 | -1.23 | 0.002 | 1.02 | 0.87 | -1.04 | | 0.13 |
| **Andoxidant** | **and detoxification response** | | | | | | | | |
| 1422438_at | epoxide hydrolase 1, microsomal | Ephx1 | 2.10 | 0.000 | 1.14 | 0.30 | -1.12 | | 0.02 |
| 1421816_at | glutathione reductase 1 | Gsr | 1.2 | 0.009 | 1.19 | 0.18 | -1.01 | | 0.95 |
| 1421041_s_at | glutathione S-transferase, alpha 2 (Yc2) | Gsta2 | 1.90 | 0.003 | -1.2 | 0.09 | -1.59 | | 0.51 |
| 1416842_at | glutathione S-transferase, mu 5 | Gstm5 | 1.29 | 0.000 | 1.02 | 0.82 | -1.02 | | 0.34 |
| 1449575_a_at | glutathione S-transferase, pi 2 | Gstp2 | 1.40 | 0.000 | 1.07 | 0.64 | 1.07 | | 0.69 |
| 1417883_at | glutathione S-transferase, theta 2 | Gstt2 | 2.76 | 0.000 | 1.18 | 0.13 | -1.30 | | 0.05 |
| 1448239_at | heme oxygenase (decycling) 1 | Hmox1 | 2.43 | 0.000 | -1.5 | 0.04 | -1.17 | | 0.12 |
| 1452592_at | microsomal glutathione S-transferase 2 | Mgst2 | 2.89 | 0.000 | 1.02 | 0.29 | 1.10 | | 0.29 |
| 1448300_at | microsomal glutathione S-transferase 3 | Mgst3 | 1.49 | 0.001 | 1.29 | 0.04 | 1.02 | | 0.10 |
| 1430979_a_at | peroxiredoxin 2 | Prdx2 | 1.61 | 0.001 | 1.75 | 0.01 | 1.34 | | 0.26 |
| 1416292_at | peroxiredoxin 3 | Prdx3 | 1.27 | 0.010 | 1.24 | 0.19 | 1.09 | | 0.18 |
| 1451124_at | superoxide dismutase 1, soluble | Sod1 | 1.22 | 0.003 | 1.26 | 0.03 | 1.16 | | 0.10 |
| 1415996_at | thioredoxin interacting protein | Txnip | 2.11 | 0.008 | 1.05 | 0.49 | 1.05 | | 0.42 |
| 1440221_at | thioredoxin-like | Txn1 | 1.50 | 0.002 | 1.26 | 0.58 | 1.17 | | 0.83 |
| **Peroxisomal** | **biogenesis** | | | | | | | | |
| 1416679_at | ATP-binding cassette, sub-family D member | 3 Abcd3 | -1.29 | 0.000 | 1.03 | 0.63 | 1.03 | | 0.60 |
| 1449442_at | peroxisomal biogenesis factor 11a | Pex11a | -1.78 | 0.010 | -1.97 | 0.04 | -1.60 | | 0.37 |
| 1451213_at | peroxisomal biogenesis factor 11b | Pex11b | -1.24 | 0.010 | -1.03 | 0.82 | -1.05 | | 0.69 |

**Fatty acid biosynthesis and elongation**

(continued)

| Code | Title | Symbol | $Csb_{m/m}Xpa_{-/-}$ | | | $Xpa_{-/-}$ | | $Csb_{m/m}$ | |
|------|-------|--------|------|------|------|------|------|------|------|
| The IGF-1/GH growth axis | | | FC | P- | FC | P-value | FC | | P- |
| 1455994_x_at | ELOVL1 long chain fatty acid elongation | Elovll | 1.28 | 0.001 | 1.24 | 0.03 | 1.22 | | 0.08 |
| 1417403_at | ELOVL6, long chain fatty acid elongatiion | Elov16 | 1.37 | 0.001 | 1.18 | 0.23 | 1.09 | | 0.38 |
| 1415823_at | stearoyl-Coenzyme A desaturase 2 | Scd2 | 1.39 | 0.001 | 1.29 | 0.15 | 1.23 | | 0.45 |
| 1424119_at | protein kinase beta 1 non-catalytic subunit | Prkab1 | 1.44 | 0.000 | 1.28 | 0.15 | 1.11 | | 0.80 |
| 1418438_at | fatty acid binding protein 2, intestinal | Fabp2 | 1.40 | 0.001 | 1.09 | 0.21 | 1.13 | | 0.03 |
| 1416021_a_at | fatty acid binding protein 5, epidermal | Fabp5 | 1.63 | 0.000 | -1.2 | 0.17 | 1.14 | | 0.57 |
| 1450779_at | fatty acid binding protein 7, brain | Fabp7 | 2.16 | 0.000 | 1.52 | 0.21 | 1.66 | | 0.05 |
| 1425875_a_at | leptin receptor | Lepr | 2.78 | 0.000 | -1.6 | 0.02 | -1.3 | | 0.07 |
| 1420715_a_at | peroxisome proliferator activated receptor gamma Pparg | | 1.99 | 0.000 | 1.53 | 0.01 | 1.11 | | 0.73 |
| 1417900_a_at | very low density lipoprotein receptor | Vldlr | 1.81 | 0.001 | 1.01 | 0.86 | -1.1 | | 0.40 |

### $Ercc1^{-/-}$ mouse model:

Although the XFE patient (see example 1) and $Ercc1^{-/-}$ mouse model showed clear signs of premature aging in a distinct set of tissues, we wished to determine the extent of the parallels with normal aging. The genome-wide shift in expression observed in the $Ercc1^{-/-}$ mice offers a comprehensive readout for identifying physiological changes and provides a powerful platform to compare with genome-wide expression shifts in normal aging. Initial cDNA and Affymetrix microarray experiments pointed to a substantial overlap between transcriptional responses of 20-day-old (non-moribund) $Ercc1^{-/-}$ mice and 2-year-old wt mice (Tables 2 and 3). To confirm, as well as to extend these findings over the Affymetrix full mouse transcriptome platform, we first classified the previously identified set of 1675 genes as having an increased or decreased expression change relative to the wt controls and compared them to those obtained when the livers of 4-month and 2.7-year old mice were contrasted against those of 2-month old young adult controls (n=4). This approach assigned a correlation coefficient (Pearson's r) that was proportional to the percentage of genes with the same direction of expression change between the $Ercc1^{-/-}$, the 4-month and 2.7-year old mice. Strikingly, this analysis revealed $Ercc1^{-/-}$ mice to share a striking degree of correlation with the 2.7-year old mice but not with 4-month old mice (Pearson's r: 0.32 vs. 0.03, $p \leq 0.05$) demonstrating that, despite the dramatic difference in age and genetic background, there are strong parallels between the progeria caused by the deficiency in XPF-ERCC1 and natural aging at the fundamental level of gene expression (Fig. 18E). Furthermore, the correlation in the transcriptional profiles of $Ercc1^{-/-}$ and naturally aged mice was even more pronounced when the same approach was restricted to those biological themes previously identified as significantly over-represented in the $Ercc1^{-/-}$ mouse liver (Table 4) demonstrating the commonality of the identified responses between $Ercc1^{-/-}$ progeria and the natural aging process at the level of genome-wide expression shifts.

**Table 2.** Genes identified as significantly differentially expressed in Ercc1-deficient mouse liver compared to wt littermate controls, using a single animal pair-wise comparison. Genes highlighted with an # were found to be similarly differentially expressed when $Ercc1_{-/-}$ mouse liver was compared to control littermates using pooled samples representing multiple animals.

| Gene name | Gene ID | Fold change in oldwild- type liver |
|---|---|---|
| cytochrome P450, 4a14 # | AA060595 | 7.5 |
| fat specific gene 27 # | AA466094 | 4.4 |
| insulin-like growth factor binding protein 1 # | W83086 | 3.7 |
| cytochrome P450, 4a10 # | AA109684 | 3.2 |
| tubulin $\alpha$4 | W11746 | 1.9 |
| solute carrier family 27 (fatty acid transporter) | AA108401 | 1.8 |
| control for tubulin $\alpha$4 | none | 1.8 |
| cytochrome P450, 4a14 # | AA061737 | 1.8 |
| angiogenin | AA237829 | 1.7 |
| paraoxonase 1 | W98586 | 1.6 |
| ATPase, Ca2+transporting, cardiac muscle # | W34420 | 1.6 |
| murine Glvr-1 mRNA # | AA177949 | 1.6 |
| transcription termination factor 1 # | AA049906 | 1.6 |
| CBFA2T3 # | AA051563 | 1.6 |
| cathepsin L | AA174215 | 1.6 |
| Rad23A | AA061459 | 1.5 |
| transthyretin | W17647 | 1.5 |
| mannosidase 2, $\alpha$1 # | W09023 | 1.5 |
| CEA-related cell adhesion molecule 1 # | AA245546 | 1.4 |
| insulin-like growth factor binding protein 10 # | AA423149 | 1.4 |
|  |  |  |
| fatty acid amide hydrolase # | AA260227 | -14.1 |
| esterase 31 # | AA254921 | -4.8 |
| insulin-lik # | W10072 | -2.4 |

**Table 3.** Gene expression profile of aged mouse liver RNA. The table indicates the identity of genes significantly differentially expressed in aged (26 mo) wt mouse liver compared to young (6 mo) wt mouse liver in a C57B1/6 background. The experiment included a pair-wise comparison of 6 animals of each age group. The names of the genes, their Genebank Accession number and the fold difference in expression are indicated. Genes highlighted with an # were similarly differentially expressed in 3 wk-old $Ercc1_{-/-}$ mouse liver compared to wt littermates. Genes highlighted with ** were also differentially expressed in 3 wk old $Ercc1_{-/-}$ mouse liver, but in the opposite direction.

| Gene name | Gene ID | Fold change in oldwild-type liver |
|---|---|---|
| ATP-binding cassette 2 | AA276156 | 3.1 |
| cytochrome P450, 4A14 # | AA061737 | 2.9 |

(continued)

| Gene name | Gene ID | Fold change in oldwild-type liver |
|---|---|---|
| *Mus musculus*cleavage and polyadenylation | AA267638 | 2.4 |
| cathepsin S | AA178121 | 2.3 |
| histocompatibility 2, K region | W 14540 | 2.1 |
| lipocalin 2 | AA087193 | 1.9 |
| *Mus musculus*B lymphocyte chemoattractant | AA152885 | 1.8 |
| retinoic acid early transcript y | A1451859 | 1.7 |
| insulin-like growth factor binding protein 1 # | W83086 | 1.9 |
| cytochrome P450, 4A14 # | AA109684 | 1.7 |
| inter-$\alpha$-trypsin inhibitor, heavy chain # | AA062129 | 1.6 |
| CBFA2T3 identified gene homolog # | AA051563 | 1.6 |
| murine mRNA for $\beta$-subunit of T-cell receptor | AA265714 | 1.6 |
| S-adenosylmethionine synthetase # | W29782 | 1.5 |
| Rad23A # | AA061459 | 1.5 |
| angiogenin # | AA237829 | 1.5 |
| histocompatibility 2, L region | AA221044 | 1.3 |
| cathepsin L # | AA174215 | 1.3 |
| | | |
| fatty acid amide hydrolase # | AA260227 | -2.4 |
| CEA-related cell adhesion molecule ** | AA245546 | -2.0 |
| glutathione S-transferase $\pi$2 | AA108370 | -1.9 |
| *Mus musculus*mRNA for N-acetylglucosamine | AA538322 | -1.7 |
| hemoglobin $\alpha$, adult chain 1 | AA109900 | -1.6 |
| serine protease inhibitor 2-1 | W83447 | -1.6 |
| $\delta$-aminolevulinate dehydratase | AA222320 | -1.6 |
| transcription termination factor 1 ** | AA049906 | -1.5 |
| cytochrome P450 2F2 | AA220582 | -1.4 |
| esterase 31 # | AA254921 | -1.3 |

To confirm the most important biological responses predicted from the microarray analysis to be shared by natural aging and XFE progeria, we used immunodetection to compare *Ercc1*$^{-/-}$ mouse liver to that of young (21 day-old) wt littermates and aged mice. IGFBP1 levels were extremely elevated in 21 day-old *Ercc1*$^{-/-}$ and aged mouse liver, (Fig 18F). The fraction of proliferative cells was dramatically reduced in aged wt mouse liver relative to young wt mice, similar to *Ercc1*$^{-/-}$ mouse liver (Fig. 18B). Hepatocytes with polyploidy nuclei, a hallmark of aged liver (2), were common in both the *Ercc1*$^{-/-}$ and aged mouse liver (Fig. 18B). There was increased accumulation of triglycerides in aged liver, demonstrating energy storage rather than utilization, as seen in the *Erec1-/-* mouse liver (Fig. 18D). Finally, apoptotic cells were markedly elevated in *Ercc1-/-* mouse liver compared to wt littermates, and modestly elevated in aged liver (Fig. 18G). These data validate the microarray results and emphasize the strong physiological and pathological parallels between XFE progeria and the natural aging process.

## Comparison of *Csb^{m/m}/Xpa^{-/-}* and *Ercc1^{-/-}* mouse models:

*Erec1^{-/-}* and *CsY^{m/m}/Xpa^{-/-}* animals represent distinct DNA repair-deficient mouse models with a broad spectrum of partially overlapping as well as distinct progeroid features. To examine whether, and to what extent, the phenotypic parallels and differences are also reflected at the fundamental level of gene expression, we applied the same approach as before and compared the previously identified set of 1675 differentially expressed genes to those of *Csb^{m/m}/Xpa^{-/-}* mice obtained in the same fashion (accompanying manuscript). This approach revealed *Csb^{m/m}/Xpa^{-/-}* mice to demonstrate a significantly greater similarity with *Ercc1^{-/-}* mice (Pearson's r: 0.65, p<0.05, Fig. 18H and Table 5) than with *Csb^{m/m}* or *Xpa^{-/-}* single mutants (Pearson's r: 0.26 and 0.31, respectively, Fig. 18H). Importantly, these findings were also equivalent when the full mouse transcriptome of *Csb^{m/m}/Xpa^{-/-}* mice was compared to that of *Ercc1^{-/-}* (Pearson's r: 0.43, p<0.05), *Csb^{m/m}* or *Xpa^{-/-}* single mutant littermates (Pearson's r: 0.29 and 0.30, respectively) despite the difference in genetic background (pure C57Bl/6, versus F1 hybrid with FVB) demonstrating the uniformity and physiological significance of the response to unrepaired DNA damage.

Although there were significant parallels between the expression profiles of 15-day *Ercc1^{-/-}* and *Csb^{m/m}/Xpa^{-/-}* mice, there were also quantitative and qualitative differences; for example the prominent up-regulation pro-apoptotic genes and down-regulation of inhibitors of apoptosis in the *Ercc1^{-/-}* liver expression profile, the robust up-regulation of the IGFBP1, which is strongly induced in rodent models exposed to the crosslinking agent cisplatin [Fig. 18F; (3)] and the specific up-regulation of DNA damage repair genes *(e.g. Rad51).*

**Table 4**

| Code | Gene Title | Gene Symbol | *Ercc1-/-* fc | p | 2.7-year old fc | p |
|---|---|---|---|---|---|---|
| The IGF/GH somatotroph axis and additional mitogenic signals | | | | | | |
| 1419519_at | Insulin-like growth factor 1 | Igf1 | -1.50 | 0.002 | -1.48 | 0.009 |
| 1434413_at | Insulin-like growth factor 1 | Igf1 | -1.38 | 0.011 | -1.36 | 0.005 |
| 1437401_at | Insulin-like growth factor 1 | Igf1 | -1.64 | 0.000 | -1.37 | 0.001 |
| 1452014_a_at | Insulin-like growth factor 1 | Igf1 | -1.74 | 0.000 | -1.30 | 0.043 |
| 1421992_a_at | insulin-like growth factor binding protein 4 | Igfbp4 | -1.19 | 0.010 | -1.16 | 0.462 |
| 1422826_at | Insulin-like growth factor binding protein, acid labile subunit | Igfals | -1.68 | 0.003 | -2.28 | 0.009 |
| 1417962_s_at | Growth hormone receptor | Ghr | -1.25 | 0.046 | -1.51 | 0.142 |
| 1417991_at | deiodinase, iodothyronine, type I | Dio1 | -2.35 | 0.000 | -1.69 | 0.086 |
| 1418938_at | deiodinase, iodothyronine, type II | Dio2 | -1.27 | 0.010 | -3.13 | 0.139 |
| 1421841_at | fibroblast growth factor receptor 3 | Fgfr3 | -2.02 | 0.000 | -2.66 | 0.032 |
| 1450869_at | fibroblast growth factor 1 | Fgf1 | -1.51 | 0.000 | -2.42 | 0.003 |
| 1423136_at | fibroblast growth factor 1 | Fgf1 | -1.51 | 0.000 | -1.91 | 0.008 |
| 1450282_at | fibroblast growth factor 4 | Fgf4 | -1.36 | 0.001 | -1.67 | 0.015 |
| 1425796_a_at | fibroblast growth factor receptor 3 | Fgfr3 | -1.32 | 0.004 | -2.45 | 0.024 |
| 1451912_a_at | fibroblast growth factor receptor-like 1 | Fgfrl1 | 1.14 | 0.028 | -1.36 | 0.247 |
| Carbohydrate | metabolism | | | | | |
| 1419148_a_at | glucokinase | Gck | -6.87 | 0.001 | -1.92 | 0.138 |
| 1425303_at | glucokinase | Gck | -6.81 | 0.000 | -2.24 | 0.104 |
| 1459522_s_at | glycogenin 1 | Gyg1 | 1.46 | 0.000 | 1.37 | 0.438 |
| 1456728_x_at | aconitase 1 | Aco1 | -1.33 | 0.000 | -2.20 | 0.009 |
| 1416737_at | glycogen synthase 3, brain | Gys3 | 1.31 | 0.004 | 2.17 | 0.006 |
| 1424815_at | glycogen synthase 2 | Gys2 | 1.85 | 0.002 | -2.40 | 0.042 |

(continued)

| Code | Gene Title | Gene Symbol | *Ercc1-/-* | | 2.7-year old | |
|---|---|---|---|---|---|---|
| | | | fc | p | fc | p |
| **Cytochrome P450 oxidative metabolism** | | | | | | |
| 1419559_at | cytochrome P450, family 4, subfamily f, polypeptide 14 | Cyp4f14 | -2.92 | 0.000 | -2.09 | 0.018 |
| 1417531_at | cytochrome P450, family 2, subfamily j, polypeptide 5 | Cyp2j5 | -2.65 | 0.000 | -1.59 | 0.051 |
| 1417532_at | cytochrome P450, family 2, subfamily j, polypeptide 5 | Cyp2j5 | -2.41 | 0.000 | -2.90 | 0.039 |
| 1448792_a_at | cytochrome P450, family 2, subfamily f, polypeptide 2 | Cyp2f2 | -2.28 | 0.000 | -1.42 | 0.271 |
| 1422230_s_at | cytochrome P450, family 2, subfamily a, polypeptide 4 and 5 | Cyp2a4 /// Cyp2a5 | -2.26 | 0.003 | 1.06 | 0.889 |
| 1450715_at | cytochrome P450, family 1, subfamily a, polypeptide 2 | Cyp1a2 | -2.25 | 0.000 | -2.48 | 0.002 |
| 1417017_at | cytochrome P450, family 17, subfamily a, polypeptide 1 | Cyp17a1 | -1.95 | 0.000 | -1.02 | 0.971 |
| 1449565_at | cytochrome P450, family 2, subfamily g, polypeptide 1 | Cyp2g1 | -1.79 | 0.029 | 1.63 | 0.259 |
| 1425645_s_at | cytochrome P450, family 2, subfamily b, polypeptide 10 | Cyp2b10 | -1.69 | 0.011 | -2.35 | 0.410 |
| 1451787_at | cytochrome P450, family 2, subfamily b, polypeptide 10 | Cyp2b10 | -1.68 | 0.014 | -2.23 | 0.437 |
| 1418780_at | cytochrome P450, family 39, subfamily a, polypeptide 1 | Cyp39a1 | -1.57 | 0.007 | -3.52 | 0.184 |
| 1422257_s_at | cytochrome P450, family 2, subfamily b, polypeptide 10 | Cyp2b10 | -1.56 | 0.031 | -3.04 | 0.294 |
| 1421741_at | cytochrome P450, family 3, subfamily a, polypeptide 16 | Cyp3a16 | -1.54 | 0.012 | 3.33 | 0.023 |
| 1444138_at | Cytochrome P450, family 2, subfamily r, polypeptide 1 | Cyp2r1 | -1.52 | 0.005 | -1.89 | 0.016 |
| 1425365_a_at | cytochrome P450, family 2, subfamily d, polypeptide13 | Cyp2d13 | -1.50 | 0.037 | -2.51 | 0.016 |
| 1431803_at | cytochrome P450, family 2, subfamily d, polypeptide13 | Cyp2d13 | -1.49 | 0.008 | -2.86 | 0.030 |
| 1438743_at | cytochrome P450, family 7, subfamily a, polypeptide 1 | Cyp7a1 | -1.43 | 0.017 | -1.37 | 0.653 |
| 1418767_at | cytochrome P450, family 4, subfamily f, polypeptide 13 | Cyp4f13 | -1.37 | 0.028 | -1.98 | 0.016 |
| 1424273_at | cytochrome P450, family 2, subfamily c, polypeptide70 | Cyp2c70 | -1.37 | 0.000 | -1.02 | 0.910 |
| 1417590_at | cytochrome P450, family 27, subfamily a, polypeptide1 | Cyp27a1 | -1.30 | 0.007 | -1.41 | 0.075 |
| 1417070_at | cytochrome P450, family 4, subfamily v, polypeptide 3 | Cyp4v/3 | -1.28 | 0.001 | -2.51 | 0.004 |
| 1423244_at | similar to Cytochrome P450, family 2, subfamily c, polypeptide 40 | LOC433 247 | -1.25 | 0.003 | -1.50 | 0.106 |
| 1419590_at | cytochrome P450, family 2, subfamily b, polypeptide 9 | Cyp2b9 | -1.22 | 0.004 | 14.44 | 0.041 |

(continued)

| Code | Gene Title | Gene Symbol | *Ercc1-/-* | | 2.7-year old | |
|---|---|---|---|---|---|---|
| | | | fc | p | fc | p |
| 1418821_at | cytochrome P450, family 2, subfamily a, polypeptide 12 | Cyp2a12 | -1.22 | 0.000 | -1.42 | 0.085 |
| 1430172_a_at | cytochrome P450, family 4, subfamily f, polypeptide 16 | Cyp4f16 | 1.28 | 0.002 | 4.05 | 0.319 |
| 1455457_at | cytochrome P450, family 2, subfamily c, polypeptide50 | Cyp2c50 /// Cyp2c54 | 4.10 | 0.000 | 1.25 | 0.600 |

**NADH- and NADPH-dependent oxidative metabolism**

| Code | Gene Title | Gene Symbol | fc | p | fc | p |
|---|---|---|---|---|---|---|
| 1416366_at | NADH dehydrogenase (ubiquinone) 1, subcomplex unknown, 2 | Ndufc2 | -1.38 | 0.007 | -1.48 | 0.001 |
| 1434212_at | NADH dehydrogenase (ubiquinone) Fe-S protein 8 | Ndufs8 | -1.35 | 0.001 | -1.87 | 0.188 |
| 1434213_x_at | NADH dehydrogenase (ubiquinone) Fe-S protein 8 | Ndufs8 | -1.24 | 0.000 | -1.31 | 0.021 |
| 1438166_x_at | NADH dehydrogenase (ubiquinone) Fe-S protein 4 | Ndufs4 | -1.24 | 0.034 | -1.65 | 0.124 |
| 1423908_at | NADH dehydrogenase (ubiquinone) Fe-S protein 8 | Ndufs8 | -1.19 | 0.002 | -1.43 | 0.017 |
| 1452790_x_at | NADH dehydrogenase (ubiquinone) 1 alpha subcomplex, 3 | Ndufa3 | -1.17 | 0.018 | -1.14 | 0.169 |
| 1422241_a_at | NADH dehydrogenase (ubiquinone) 1 alpha subcomplex, 1 | Ndufa1 | -1.16 | 0.001 | -1.27 | 0.005 |
| 1428464_at | NADH dehydrogenase (ubiquinone) 1 alpha subcomplex, 3 | Ndufa3 | -1.16 | 0.009 | -1.09 | 0.468 |
| 1416834_x_at | NADH dehydrogenase (ubiquinone) 1 beta subcomplex, 2 | Ndufb2 | -1.15 | 0.022 | -1.29 | 0.052 |
| 1447919_x_at | NADH dehydrogenase (ubiquinone) 1, alpha/beta subcomplex, 1 | Ndufab1 | -1.15 | 0.025 | -2.48 | 0.010 |
| 1451096_at | NADH dehydrogenase (ubiquinone) Fe-S protein 2 | Ndufs2 | -1.12 | 0.042 | -1.21 | 0.167 |
| 1455036_s_at | NADH dehydrogenase (ubiquinone) 1, subcomplex unknown, 2 | Ndufc2 | -1.07 | 0.049 | -1.24 | 0.014 |
| 1417429_at | flavin containing monooxygenase 1 | Fmo1 | -1.14 | 0.033 | -2.46 | 0.019 |

**Peroxisomal biogenesis**

| Code | Gene Title | Gene Symbol | fc | p | fc | p |
|---|---|---|---|---|---|---|
| 1419365_at | peroxisomal biogenesis factor 11a | Pex11a | -2.25 | 0.000 | -1.80 | 0.075 |
| 1451213_at | peroxisomal biogenesis factor 11b | Pex11b | -1.38 | 0.000 | -2.16 | 0.021 |
| 1448910_at | peroxisomal trans-2-enoyl-CoA reductase | Pecr | -1.26 | 0.037 | -1.76 | 0.036 |
| 1430015_st | peroxisomal, testis specific 1 | Pxt1 | -1.25 | 0.009 | -2.03 | 0.070 |

(continued)

| Code | Gene Title | Gene Symbol | Ercc1-/- | | 2.7-year old | |
|---|---|---|---|---|---|---|
| | | | fc | p | fc | p |
| 1451226_at | peroxisomal biogenesis factor 6 | Pex6 | -1.13 | 0.028 | -2.01 | 0.012 |
| 1422471_at | peroxisomal biogenesis factor 13 | Pex13 | -1.09 | 0.009 | -1.68 | 0.005 |

table 5

| code | Gene Title | Gene Symbol | Csb $_{m/m}$ Xpa $_{-/-}$ | | Ercc1 $_{-/-}$ | | 2.7-year old | |
|---|---|---|---|---|---|---|---|---|
| | | | fc | p | fc | p | fc | p |
| **The IGF/GH somatotroph axis and additional mitogenic signals** | | | | | | | | |
| 1419519_at | insulin-like growth factor 1 | Igf1 | -2.13 | 0.000 | -1.50 | 0.002 | -1.48 | 0.009 |
| 1434413_at | Insulin-like growth factor 1 | Igf1 | -1.82 | 0.000 | -1.38 | 0.011 | -1.36 | 0.005 |
| 1437401_at | Insulin-like growth factor 1 | Igf1 | -1.43 | 0.001 | -1.64 | 0.000 | -1.37 | 0.001 |
| 1452014_a_at | Insulin-like growth factor 1 | Igf1 | -2.02 | 0.000 | -1.74 | 0.000 | -1.30 | 0.043 |
| 1454159_a_at | Insulin-like growth factor binding protein 2 | Igfbp2 | 1.15 | 0.008 | 1.09 | 0.031 | -2.22 | 0.012 |
| 1423062_at | insulin-like growth factor binding protein 3 | Igfbp3 | -1.38 | 0.000 | -1.41 | 0.000 | 1.60 | 0.150 |
| 1421992_a_at | insulin-like growth factor binding protein 4 | Igfbp4 | -1.43 | 0.000 | -1.19 | 0.010 | -1.16 | 0.462 |
| 1423584_at | insulin-like growth factor binding protein 7 | Igfbp7 | -1.11 | 0.089 | 1.20 | 0.050 | 1.04 | 0.906 |
| 1422826_at | Insulin-like growth factor binding protein, acid labile subunit | Igfals | -2.36 | 0.000 | -1.68 | 0.003 | -2.28 | 0.009 |
| 1451871_a_at | Growth hormone receptor | Ghr | -2.21 | 0.012 | -1.25 | 0.249 | -2.37 | 0.002 |
| 1417962_s_at | Growth hormone receptor | Ghr | -1.53 | 0.000 | -1.25 | 0.046 | -1.51 | 0.142 |
| 1451501_a_at | Growth hormone receptor | Ghr | -1.51 | 0.001 | -1.18 | 0.098 | -1.50 | 0.193 |
| 1459948_at | Growth hormone receptor | Ghr | -1.08 | 0.642 | -1.16 | 0.463 | -3.98 | 0.070 |
| 1458832_at | Growth hormone receptor | Ghr | -1.53 | 0.038 | -1.05 | 0.794 | -1.88 | 0.023 |
| 1448556_at | prolactin receptor | Prtr | -2.03 | 0.000 | -1.86 | 0.003 | -1.13 | 0.806 |
| 1425853_s_at | prolactin receptor | Prior | -1.87 | 0.004 | -1.62 | 0.007 | -1.20 | 0.464 |
| 1421382_at | prolactin receptor | Prtr | -1.96 | 0.000 | -1.58 | 0.000 | 1.09 | 0.833 |
| 1450226_at | prolactin receptor | Prtr | -1.76 | 0.001 | -1.58 | 0.002 | -1.17 | 0.542 |
| 1451844_at | prolactin receptor | Prtr | -1.39 | 0.020 | -1.31 | 0.021 | 1.17 | 0.554 |
| 1451850_at | prolactin receptor | Prtr | -1.01 | 0.566 | -1.01 | 0.478 | -1.04 | 0.951 |

(continued)

| code | Gene Title | Gene Symbol | Csb $_{m/m}$ | | Xpa $_{-/-}$ | | Ercc1 $_{-/-}$ | | 2.7-year old | |
|---|---|---|---|---|---|---|---|---|---|---|
| | | | fc | p | fc | p | fc | p | fc | p |
| 1417991_at | deiodinase, iodothyronine, type I | Dio1 | -2.12 | 0.000 | -2.35 | 0.000 | -1.69 | 0.086 | | |
| 1418938_at | deiodinase, iodothyronine, type II | Dio2 | -1.30 | 0.015 | -1.27 | 0.010 | -3.13 | 0.139 | | |
| 1426081_a_at | deiodinase, iodothyronine, type II | Dio2 | -1.09 | 0.433 | -1.13 | 0.179 | -1.72 | 0.231 | | |
| 1418937_at | deiodinase, iodothyronine, type II | Dio2 | 1.00 | 1.000 | 1.00 | 1.000 | 1.19 | 0.543 | | |
| 1421841_at | fibroblast growth factor receptor 3 | Fgfr3 | -1.43 | 0.001 | -2.02 | 0.000 | -2.66 | 0.032 | | |
| 1450869_at | fibroblast growth factor 1 | Fgf1 | -1.38 | 0.003 | -1.51 | 0.000 | -2.42 | 0.003 | | |
| 1423136_at | fibroblast growth factor 1 | Fgf1 | -1.16 | 0.173 | -1.51 | 0.000 | -1.91 | 0.008 | | |
| 1450282_at | fibroblast growth factor 4 | Fgf4 | -1.12 | 0.235 | -1.36 | 0.001 | -1.67 | 0.015 | | |
| 1425796_a_at | fibroblast growth factor receptor 3 | Fgfr3 | -1.28 | 0.028 | -1.32 | 0.004 | -2.45 | 0.024 | | |
| 1451912_a_at | fibroblast growth factor receptor-like 1 | Fgfrl1 | -1.06 | 0.416 | 1.14 | 0.028 | -1.36 | 0.247 | | |
| 1452661_st | transferrin receptor | Tfrc | -1.05 | 0.540 | -1.77 | 0.002 | 1.17 | 0.445 | | |
| 1442049_at | Transferrin | Trf | 1.02 | 0.890 | 1.65 | 0.001 | -1.33 | 0.457 | | |
| **Carbohydrate metabolism** | | | | | | | | | | |
| 1419146_a_at | glucokinase | Gck | -6.59 | 0.004 | -6.87 | 0.001 | -1.92 | 0.138 | | |
| 1425303_at | glucokinase | Gck | -4.80 | 0.004 | -6.81 | 0.000 | -2.24 | 0.104 | | |
| 1459522_s_at | glycogenin 1 | Gyg1 | 1.26 | 0.014 | 1.46 | 0.000 | 1.37 | 0.438 | | |
| 1456728_x_at | aconitase 1 | Aco1 | -1.15 | 0.016 | -1.33 | 0.000 | -2.20 | 0.009 | | |
| 1416737_at | glycogen synthase 3, brain | Gys3 | 1.15 | 0.150 | 1.31 | 0.004 | 2.17 | 0.006 | | |
| 1424815_at | glycogen synthase 2 | Gys2 | 1.78 | 0.000 | 1.85 | 0.002 | -2.40 | 0.042 | | |
| **Cytochrome P450 oxidative metabolism** | | | | | | | | | | |
| 1419559_at | cytochrome P450, family 4, subfamily f, polypeptide 14 | Cyp4f14 | -3.58 | 0.000 | -2.92 | 0.000 | -2.09 | 0.018 | | |
| 1417531_at | cytochrome P450, family 2, subfamily J, polypeptide 5 | Cyp2j5 | -2.51 | 0.000 | -2.65 | 0.000 | -1.59 | 0.051 | | |
| 1417532_at | cytochrome P450, family 2, subfamily j, polypeptide 5 | Cyp2j5 | -3.08 | 0.000 | -2.41 | 0.000 | -2.90 | 0.039 | | |

(continued)

| code | Gene Title | Gene Symbol | Csb $_{m/m}$ | | Xpa $_{-/-}$ | | Ercc1 $_{-/-}$ | | 2.7-year old | |
|---|---|---|---|---|---|---|---|---|---|---|
| | | | fc | p | fc | p | fc | p | fc | p |
| 1448792_a_at | cytochrome P450, family 2, subfamily f, polypeptide 2 | Cyp2f2 | -2.36 | 0.001 | -2.28 | 0.000 | -1.42 | 0.271 | | |
| 1422230_a_at | cytochrome P450, family 2, subfamily a, polypeptide 4 /// cytochrome P450, | Cyp2a4 /// Cyp2a5 | -1.59 | 0.067 | -2.26 | 0.003 | 1.06 | 0.889 | | |
| 1450715_at | cytochrome P450, family 1, subfamily a, polypeptide 2 | Cyp1a2 | -1.53 | 0.031 | -2.25 | 0.000 | -2.48 | 0.002 | | |
| 1417017_at | cytochrome P450, family 17, subfamily a, polypeptide 1 | Cyp17a1 | -1.41 | 0.029 | -1.95 | 0.000 | -1.02 | 0.971 | | |
| 1449565_at | cytochrome P450, family 2, subfamily g, polypeptide 1 | Cyp2g1 | -2.43 | 0.011 | -1.79 | 0.029 | 1.63 | 0.259 | | |
| 1425645_a_at | cytochrome P450, family 2, subfamily b, polypeptide 10 | Cyp2b10 | -2.94 | 0.001 | -1.69 | 0.011 | -2.35 | 0.410 | | |
| 1451787_at | cytochrome P450, family 2, subfamily b, polypeptide 10 | Cyp2b10 | -2.22 | 0.005 | -1.68 | 0.014 | -2.23 | 0.437 | | |
| 1418780_at | cytochrome P450, family 39, subfamily a, polypeptide 1 | Cyp39a1 | -1.37 | 0.021 | -1.57 | 0.007 | -3.52 | 0.184 | | |
| 1422257_s_at | cytochrome P450, family 2, subfamily b, polypeptide 10 | Cyp2b10 | -2.81 | 0.001 | -1.56 | 0.031 | -3.04 | 0.294 | | |
| 1421741_at | cytochrome P450, family 3, subfamily a, polypeptide 16 | Cyp3a16 | -1.34 | 0.086 | -1.54 | 0.012 | 3.33 | 0.023 | | |
| 1444138_at | cytochrome P450, family 2, subfamily r, polypeptide 1 | Cyp2r1 | -1.27 | 0.120 | -1.52 | 0.005 | -1.89 | 0.016 | | |
| 1425365_a_at | cytochrome P450, family 2, subfamily d, polypeptide 13 | Cyp2d13 | -2.17 | 0.006 | -1.50 | 0.037 | -2.51 | 0.016 | | |
| 1431803_at | cytochrome P450, family 2, | Cyp2d13 | -2.05 | 0.001 | -1.49 | 0.008 | -2.86 | 0.030 | | |

(continued)

| code | Gene Title | Gene Symbol | Csb _m/m_ | | Xpa _-/-_ | | Ercc1 _-/-_ | | 2.7-year old | |
|---|---|---|---|---|---|---|---|---|---|---|
| | | | fc | p | fc | p | fc | p | fc | p |
| | subfamily d, polypeptide 13 | | | | | | | | | |
| 1438743_at | cytochrome P450, family 7, | Cyp7a1 | -2.28 | 0.000 | -1.43 | 0.017 | -1.37 | 0.653 | | |
| | subfamily a, polypeptide 1 | | | | | | | | | |
| 1418767_at | cytochrome P450, family 4, | Cyp4f13 | -1.67 | 0.008 | -1.37 | 0.028 | -1.98 | 0.016 | | |
| | subfamily f, polypeptide 13 | | | | | | | | | |
| 1424273_at | cytochrome P450, family 2, | Cyp2c70 | -1.56 | 0.000 | -1.37 | 0.000 | -1.02 | 0.910 | | |
| | subfamily c, polypeptide 70 | | | | | | | | | |
| 1417590_at | cytochrome P450, family 27, | Cyp27a1 | -1.33 | 0.012 | -1.30 | 0.007 | -1.41 | 0.075 | | |
| | subfamily a, polypeptide 1 | | | | | | | | | |
| 1417070_at | cytochrome P450, family 4, | Cyp4v3 | -1.41 | 0.001 | -1.28 | 0.001 | -2.51 | 0.004 | | |
| | subfamily v, polypeptide 3 | | | | | | | | | |
| 1423244_at | similar to Cytochrome P450, family 2, subfamily c, polypeptide 40 | LOC4332 47 | -1.06 | 0.379 | -1.25 | 0.003 | -1.50 | 0.106 | | |
| 1419590_at | cytochrome P450, family 2, | Cyp2b9 | -1.55 | 0.000 | -1.22 | 0.004 | 14.44 | 0.041 | | |
| | subfamily b, polypeptide 9 | | | | | | | | | |
| 1418821_at | cytochrome P450, family 2, | Cyp2a12 | -1.51 | 0.000 | -1.22 | 0.000 | -1.42 | 0.085 | | |
| | subfamily a, polypeptide 12 | | | | | | | | | |
| 1430172_a_at | cytochrome P450, family 4, | Cyp4f16 /// LOC4330 95 | 1.18 | 0.036 | 1.28 | 0.002 | 4.05 | 0.319 | | |
| | subfamily f, polypeptide 16 | | | | | | | | | |
| 1419040_at | cytochrome P450, family 2, | Cyp2d22 | -1.27 | 0.089 | 1.30 | 0.015 | -1.51 | 0.179 | | |
| | subfamily d, polypeptide 22 | | | | | | | | | |
| 1449309_at | cytochrome P450, family 8, | Cyp8b1 | 1.03 | 0.774 | 1.41 | 0.001 | -5.20 | 0.000 | | |
| | subfamily b, polypeptide 1 | | | | | | | | | |
| 1424853_s_at | cytochrome P450, family 4, | Cyp4a10 | 1.84 | 0.023 | 1.91 | 0.010 | -1.17 | 0.795 | | |

(continued)

| code | Gene Title | Gene Symbol | Csb $_{m/m}$ | Xpa $_{-/-}$ | Ercc1 $_{-/-}$ | | 2.7-year old | |
|---|---|---|---|---|---|---|---|---|
| | | | fc | p | fc | p | fc | p |
| | subfamily a, polypeptide 10 | /// BC01347 6 | | | | | | |
| 1419349_a_at | cytochrome P450, family 2, subfamily d, polypeptide 9 | Cyp2d9 | 1.75 | 0.000 | 1.98 | 0.000 | -1.97 | 0.029 |
| 1419704_at | cytochrome P450, family 3, subfamily a, polypeptide 41 | Cyp3a41 | 1.65 | 0.035 | 2.14 | 0.039 | -1.25 | 0.557 |
| 1424576_s_at | cytochrome P450, family 2, subfamily c, polypeptide 44 | Cyp2c44 | 1.75 | 0.000 | 2.50 | 0.000 | -1.59 | 0.049 |
| 1455457_at | cytochrome P450, family 2, subfamily c, polypeptide 50 | Cyp2c50 /// Cyp2c54 | 3.23 | 0.006 | 4.10 | 0.000 | 1.25 | 0.600 |
| **NADH-dependent oxidative metabolism** | | | | | | | | |
| 1416366_at | NADH dehydrogenase (ubiquinone) 1, subcomplex unknown, 2 | Ndufc2 | 1.07 | 0.517 | -1.38 | 0.007 | -1.48 | 0.001 |
| 1434212_at | NADH dehydrogenase (ubiquinone) Fe-S protein 8 | Ndufs8 | -1.56 | 0.000 | -1.35 | 0.001 | -1.87 | 0.188 |
| 1434213_x_at | NADH dehydrogenase (ubiquinone) Fe-S protein 8 | Ndufs8 | -1.16 | 0.001 | -1.24 | 0.000 | -1.31 | 0.021 |
| 1438166_x_at | NADH dehydrogenase (ubiquinone) Fe-S protein 4 | Ndufs4 | -1.02 | 0.852 | -1.24 | 0.034 | -1.65 | 0.124 |
| 1423908_at | NADH dehydrogenase | Ndufs8 | -1.23 | 0.002 | -1.19 | 0.002 | -1.43 | 0.017 |
| 1452790_x_at | (ubiquinone) Fe-S protein 8 NADH dehydrogenase (ubiquinone) 1 alpha subcomplex, 3 | Ndufa3 | -1.04 | 0.540 | -1.17 | 0.018 | -1.14 | 0.169 |
| 1422241_a_at | NADH dehydrogenase | Ndufa1 | -1.07 | 0.119 | -1.16 | 0.001 | -1.27 | 0.005 |

(continued)

| code | Gene Title | Gene Symbol | Csb $_{m/m}$ fc | p | Xpa $_{-/-}$ fc | p | Ercc1 $_{-/-}$ fc | p | 2.7-year old fc | p |
|---|---|---|---|---|---|---|---|---|---|---|
| | (ubiquinone) 1 alpha subcomplex, 1 | | | | | | | | | |
| 1428464_at | NADH dehydrogenase (ubiquinone) 1 alpha subcomplex, 3 | Ndufa3 | -1.05 | 0.361 | -1.16 | 0.009 | | | -1.09 | 0.468 |
| 1416834_x_at | NADH dehydrogenase (ubiquinone) 1 beta subcomplex, 2 | Ndufb2 | -1.07 | 0.161 | -1.15 | 0.022 | | | -1.29 | 0.052 |
| 1447919_x_at | NADH dehydrogenase (ubiquinone) 1, alpha/beta subcomplex, 1 | Ndufab1 | -1.01 | 0.874 | -1.15 | 0.025 | | | -2.48 | 0.010 |
| 1422186_s_at | diaphorase 1 (NADH) | Dia1 | 1.06 | 0.115 | -1.13 | 0.048 | | | -1.86 | 0.001 |
| 1451096_at | NADH dehydrogenase (ubiquinone) Fe-S protein 2 | Ndufs2 | -1.11 | 0.052 | -1.12 | 0.042 | | | -1.21 | 0.167 |
| 1455036_s_at | NADH dehydrogenase (ubiquinone) 1, subcomplex unknown, 2 | Ndufc2 | -1.05 | 0.187 | -1.07 | 0.049 | | | -1.24 | 0.014 |
| 1448427_at | NADH dehydrogenase (ubiquinone) 1 alpha subcomplex, 6 (B14) | Ndufa6 | -1.02 | 0.289 | 1.07 | 0.004 | | | -1.53 | 0.018 |
| 1423737_at | NADH dehydrogenase (ubiquinone) Fe-S protein 3 | Ndufs3 | -1.02 | 0.338 | 1.13 | 0.000 | | | -1.69 | 0.003 |
| 1448934_at | NADH dehydrogenase (ubiquinone) 1 alpha subcomplex 10 | Ndufa10 | -1.04 | 0.507 | 1.18 | 0.000 | | | -1.31 | 0.141 |
| 1424065_at | NADH dehydrogenase (ubiquinone) 1 alpha subcomplex, 4 | Ndufa4 | -1.00 | 0.954 | 1.19 | 0.000 | | | -1.52 | 0.019 |

**NADPH-dependent oxidative metabolism**

(continued)

| code | Gene Title | Gene Symbol | Csb $_{m/m}$ Xpa $_{-/-}$ | | Ercc1 $_{-/-}$ | | 2.7-year old | |
|------|-----------|-------------|------|------|------|------|------|------|
| | | | fc | p | fc | p | fc | p |
| 1449525_at | flavin containing monooxygenase 3 | Fmo3 | -14.19 | 0.004 | -2.60 | 0.014 | -1.46 | 0.609 |
| 1435459_at | flavin containing monooxygenase 2 | Fmo2 | -2.80 | 0.000 | -1.38 | 0.029 | 1.36 | 0.299 |
| 1453435_a_at | flavin containing monooxygenase 2 | Fmo2 | -2.46 | 0.000 | -1.32 | 0.038 | -1.14 | 0.554 |
| 1417429_at | flavin containing monooxygenase 1 | Fmo1 | -1.38 | 0.000 | -1.14 | 0.033 | -2.46 | 0.019 |
| **Antioxidant response** | | | | | | | | |
| 1423891_at | glutathione S-transferase, theta 3 | Gstt3 | -1.93 | 0.001 | -1.46 | 0.008 | -1.25 | 0.618 |
| 1456036_x_at | Glutathione S-transferase omega 1 | Gsbo1 | -1.18 | 0.109 | -1.37 | 0.003 | -1.73 | 0.056 |
| 1452135_at | glutathione peroxidase 6 | Gpx6 | -1.08 | 0.572 | -1.35 | 0.013 | 1.54 | 0.509 |
| 1416411_at | glutathione S-transferase, mu 2 | Gstm2 | -1.31 | 0.004 | -1.25 | 0.002 | -1.30 | 0.113 |
| 1418186_at | glutathione S-transferase, theta 1 | Gstt1 | -1.09 | 0.229 | -1.23 | 0.001 | -1.49 | 0.111 |
| 1460671_at | glutathione peroxidase 1 | Gpx1 | -1.12 | 0.002 | -1.08 | 0.004 | -1.00 | 0.979 |
| 1421817_at | glutathione reductase 1 | Gsr | 1.18 | 0.009 | 1.11 | 0.040 | -1.58 | 0.010 |
| 1451695_a_at | glutathione peroxidase 4 | Gpx4 | 1.12 | 0.062 | 1.20 | 0.007 | -1.08 | 0.475 |
| 1449575_a_at | glutathione S-transferase, pi 1 | Gstp1 | 1.40 | 0.000 | 1.24 | 0.006 | -1.38 | 0.022 |
| 1417836_at | glutathione peroxidase 7 | Gpx7 | 1.21 | 0.139 | 1.29 | 0.037 | -1.13 | 0.554 |
| 1427473_at | glutathione S-transferase, mu 3 | Gstm3 | -1.16 | 0.271 | 1.30 | 0.041 | -1.01 | 0.977 |
| 1427474_s_at | glutathione S-transferase, mu 3 | Gstm3 | -1.27 | 0.065 | 1.31 | 0.025 | 1.16 | 0.641 |
| 1416416_x_at | glutathione S-transferase, mu 1 | Gstm1 | 1.06 | 0.443 | 1.35 | 0.006 | -1.06 | 0.589 |
| 1416842_at | glutathione S-transferase, mu 5 | Gstm5 | 1.29 | 0.000 | 1.39 | 0.000 | -1.21 | 0.120 |
| 1448330_at | glutathione S-transferase, mu 1 | Gstm1 | 1.04 | 0.701 | 1.59 | 0.002 | -1.09 | 0.532 |
| 1416368_at | glutathione S-transferase, alpha 4 | Gsta4 | 1.02 | 0.889 | 1.65 | 0.000 | -1.74 | 0.238 |
| 1421041_s_at | glutathione S-transferase, alpha 2 (Yc2) | Gsta2 | 1.90 | 0.003 | 3.29 | 0.000 | 1.41 | 0.483 |
| 1421040_a_at | glutathione S-transferase, alpha 2 (Yc2) | Gsta2 | 1.74 | 0.016 | 3.44 | 0.000 | -1.03 | 0.946 |
| 1417883_at | glutathione S-transferase, theta 2 | Gstt2 | 2.76 | 0.000 | 4.13 | 0.000 | -2.13 | 0.073 |

(continued)

| code | Gene Title | Gene Symbol | Csb $_{m/m}$ fc | Xpa $_{-/-}$ p | Ercc1 $_{-/-}$ fc | p | 2.7-year old fc | p |
|---|---|---|---|---|---|---|---|---|
| 1416399_a_at | heme oxygenase (decycling) 2 | Hmox2 | -1.06 | 0.369 | -1.24 | 0.003 | -1.16 | 0.470 |
| 1448239_at | heme oxygenase (decycling) 1 | Hmox1 | 2.43 | 0.000 | 1.52 | 0.011 | 13.24 | 0.262 |
| 1448499_a_at | epoxide hydrolase 2, cytoplasmic | Ephx2 | 1.03 | 0.604 | 1.27 | 0.000 | -2.10 | 0.008 |
| 1422438_at | epoxide hydrolase 1, microsomal | Ephx1 | 2.10 | 0.000 | 2.89 | 0.000 | -1.25 | 0.079 |
| 1423869_s_at | thloredoxin reductase 3 | Txnrd3 | -1.56 | 0.007 | -1.38 | 0.006 | -1.08 | 0.660 |
| 1449623_at | thloredoxin reductase 3 | Txnrd3 | -1.15 | 0.315 | -1.35 | 0.009 | -1.56 | 0.015 |
| 1436951_x_at | thloredoxin domain containing 9 | Txndc9 | 1.01 | 0.799 | -1.26 | 0.000 | -1.34 | 0.044 |
| 1439184_s_at | thioredoxin-like 5 | Txnl5 | -1.04 | 0.526 | -1.23 | 0.005 | -1.38 | 0.005 |
| 1423868_at | thioredoxin reductase 3 | Txnrd3 | -1.22 | 0.053 | -1.18 | 0.044 | -2.19 | 0.002 |
| 1451195_a_at | thioredoxin domain containing 1 | Txndc1 | -1.13 | 0.038 | -1.12 | 0.020 | 1.51 | 0.242 |
| 1421529_a_at | thioredoxin reductase 1 | Txnrd1 | 1.08 | 0.259 | 1.17 | 0.012 | -1.06 | 0.453 |
| 1451091_at | thioredoxin domain containing 5 | Txndc5 | 1.22 | 0.073 | 1.21 | 0.017 | -1.21 | 0.078 |
| 1457598_at | Thioredoxin-like 2 | Txnl2 | -1.21 | 0.087 | 1.22 | 0.047 | -1.43 | 0.362 |
| 1423746_at | thloredoxin domain containing 5 | Txndc5 | 1.10 | 0.385 | 1.30 | 0.028 | -1.20 | 0.159 |
| 1415997_at | thioredoxin interacting protein | Txnip | 1.39 | 0.053 | 1.81 | 0.012 | 1.14 | 0.852 |
| 1415996_at | thioredoxin interacting protein | Txnip | 2.11 | 0.008 | 1.90 | 0.002 | 1.11 | 0.800 |
| Peroxisomal biogenesis | | | | | | | | |
| 1419365_at | peroxisomal biogenesis factor 11a | Pex11a | -1.61 | 0.003 | -2.25 | 0.000 | -1.80 | 0.075 |
| 1449442_at | peroxisomal biogenesis factor 11a | Pex11a | -1.78 | 0.010 | -1.62 | 0.006 | -1.36 | 0.368 |
| 1451213_at | peroxisomal biogenesis factor 11b | Pex11b | -1.24 | 0.010 | -1.38 | 0.000 | -2.16 | 0.021 |
| 1448910_at | peroxisomal trans-2-enoyl-CoA reductase | Pecr | -1.38 | 0.009 | -1.26 | 0.037 | -1.76 | 0.036 |
| 1430015_at | peroxisomal, testis specific 1 | Pxt1 | -1.04 | 0.684 | -1.25 | 0.009 | -2.03 | 0.070 |
| 1451226_at | peroxisomal biogenesis factor 6 | Pex6 | -1.40 | 0.000 | -1.13 | 0.028 | -2.01 | 0.012 |
| 1422471_at | peroxisomal biogenesis factor 13 | Pex13 | -1.16 | 0.000 | -1.09 | 0.009 | -1.68 | 0.005 |

(continued)

| code | Gene Title | Gene Symbol | Csb $_{m/m}$ Xpa $_{-/-}$ | | Ercc1 $_{-/-}$ | | 2.7-year old | |
|---|---|---|---|---|---|---|---|---|
| | | | fc | p | fc | p | fc | p |
| 1422076_at | peroxisomal acyl-CoA thioesterase 2B | MGI:2159 621 | -1.10 | 0.399 | 1.24 | 0.034 | -1.85 | 0.103 |
| 1422925_s_at | peroxisomal acyl-CoA thioesterase 2A | MGI:2159 619 | 1.04 | 0.828 | 1.70 | 0.001 | -1.62 | 0.453 |

References for example 9:

1. R A. Miller, Science 310,44 (21 october, 2005).
2. S. Gupta, Semin Cancer Biol 10, 161 (Jun, 2000)
3. Q. Huang et al., Toxicol Sci 63,196 (2001).

**Claims**

1. A method for the selection of a compound, or a mixture of compounds, said method comprising the steps of:

   - providing a genetically altered mouse, wherein said mouse has at least one mutation in a gene encoding a protein of the Nucleotide Excision Repair (NER) pathway, said mutation causing a premature ageing phenotype as compared to a mouse lacking said mutation;
   - exposing said genetically altered mouse, or a part derived there form, to a compound, or a mixture of compounds;
   - determining the effect of the compound, or the mixture of compounds, on the premature ageing phenotype of said genetically altered mouse; and
   - selecting the compound, or the mixture of compounds, that prevents, inhibits, delays or reduces said premature aging phenotype in said genetically altered mouse,

   wherein said mutation is in a gene selected from the group consisting of: *Xpa, Xpb, Xpc, Xpd, Xpe, Xpf Xpg, Xpv, Csa, Csb, Ttda, HR23A, HR23B* and *Ercc1,* and wherein said premature ageing phenotype is a shortened life span, increased osteoporosis and/or increased retinal degeneration as compared to a mouse lacking said mutation.

2. The method according to claim 1, wherein said protein is also part of the Transcription-Coupled Repair (TCR) pathway.

3. The method according to claim 1, wherein said mutation is equivalent to or mimics a human Cockayne Syndrome (CS), a Xeroderma pigmentosum (XP), a combined Xeroderma Pigmentosum - Cockayne Syndrome (XPCS), trichothiodystrophy (TTD), a Cerebro-Oculo-Facio-Skeletal syndrome (COFS) or an XPF-ERCCI syndrome causing allele in the *Csa, Csb, Xpb, Xpd, Xpg, Xpf, Ttda* or *Ercc1* genes.

4. The method according to claim 3, wherein the human Cockayne, COFS or XPCS syndrome causing mutation is selected from the group consisting of CS-associated mutations in; the human *Csa* gene: $Csa^{null/null}$, Y322ter, the human *Csb* gene: $Csb^{null}$, Q184ter, R453ter, W517ter, R670W, R735ter, G744ter, W851R, Q854ter, R947ter, P1042L, P1095R, R1213G, the human *Xpd* gene: G602D, G675R, 669fs708ter, the human *Xpb* gene: F99S, FS740, and for the human *Xpg* gene: R263ter, 659ter.

5. The method according to any one of claims 1 to 4, wherein said genetically altered mouse has a mutation in at least two different genes each encoding a protein of the NER pathway.

6. The method according to claim 5, wherein said genetically altered mouse has a combination of mutations selected from the group consisting of: $Csa^{null/null}/Xpa^{null/null}$, $Csa^{null/null}/Xpc^{null/null}$, $Csb^{G744ter/G744ter}/Xpa^{null/null}$, $Csb^{G744ter/G744ter}/Xpc^{null/null}$, $Xpd^{G602D/G602D}/Xpa^{null/null}$, $Xpd^{R722W/R722W}/Xpa^{null/null}$, and $Xpd^{G602D/R722W}/Xpa^{null/null}$.

**Patentansprüche**

1. Verfahren zur Auswahl einer Verbindung oder eines Gemischs von Verbindungen, wobei das Verfahren die folgenden Schritte umfasst:

   Bereitstellen einer genetisch veränderten Maus, wobei die Maus zumindest eine Mutation in einem Gen aufweist, das ein Protein des Nucleotidexzisionsreparatur(NER)-Weges codiert, wobei die Mutation einen Phänotyp frühzeitigen Alterns im Vergleich zu einer Maus, der diese Mutation fehlt, aufweist;
   Exponieren der genetisch veränderten Maus oder eines aus ihr gewonnen Teils gegenüber einer Verbindung oder einem Gemisch von Verbindungen;
   Bestimmen der Wirkung der Verbindung oder des Gemischs von Verbindungen auf den Phänotyp frühzeitigen Alterns der genetisch veränderten Maus; und
   Auswählen derjenigen Verbindung oder desjenigen Gemischs von Verbindungen, das den Phänotyp frühzeitigen Alterns in der genetisch veränderten Maus verhindert, hemmt, verzögert oder verringert,
   wobei die Mutation in einem Gen liegt, das aus der Gruppe bestehend aus *Xpa, xpb, Xpc, Xpd, Xpe, Xpf, Xpg, Xpv, Csa, Csb, Ttda, HR23A, HR23B* und *Ercc1* ausgewählt wird, und wobei der Phänotyp frühzeitigen Alterns in einer verkürzten Lebenserwartung, verstärkter Osteoporose und/oder verstärkter Degeneration der Netzhaut im Vergleich zu einer Maus ohne die Mutation besteht.

2. Verfahren nach Anspruch 1, wobei das Protein auch Teil des transkriptionsgekoppelten Reparatur(TCR)-weges ist.

3. Verfahren nach Anspruch 1, wobei die Mutation einem humanen Cockayne-Syndrom (CS), Xeroderma pigmentosum (XP), einem kombinierten Xeroderma pigmentosum-Cockayne-Syndrom (XPCS), Trichothiodystrophie (TTD), einem Zerebro-okulo-fazio-skelettalen Syndrom (COFS) oder einem XPF-ERCC1-Syndrom, welche ein Allel in den Genen *Csa, Csb, Xpb, Xpd, Xpg, Xpf, Ttda* oder *Ercc1* verursachen, äquivalent ist oder dieses nachahmt.

4. Verfahren nach Anspruch 3, wobei die Mutation, welche das humane Cockayne-, COFS- oder XPCS-Syndrom verursacht, aus einer Gruppe ausgewählt wird, bestehend aus mit CS assoziierten Mutationen in dem humanen Gen *Csa : CSa$^{null/null}$,* Y322ter, dem humanen Gen *Csb: Csb$^{null}$,* Q184ter, R453ter, W517ter, R670W, R735ter, G744ter, W851R, Q854ter, R947ter, P1042L, P1095R, R1213G, dem humanen Gen *Xpd:* G602D, G675R, 669fs708ter, dem humanen Gen *Xpb:* F99S, FS740, und in dem humanen Gen *Xpg:* R263ter, 659ter.

5. Verfahren nach einem der Ansprüche 1 bis 4,
   wobei die genetisch veränderte Maus eine Mutation in mindestens zwei verschiedenen Genen aufweist, die jeweils ein Protein des NER-Weges codieren.

6. Verfahren nach Anspruch 5,
   wobei die genetisch veränderte Maus eine Kombination von Mutationen aufweist, die aus der Gruppe ausgewählt werden, bestehend aus : *Csa$^{null/null}$/Xpa$^{null/null}$, CSa$^{null/null}$/Xpc$^{null/null}$, CSb$^{G744ter/G744ter}$/Xpa$^{null/null}$, Csb$^{G744ter/G744ter}$/Xpc$^{null/null}$, Xpd$^{G602D/G602D}$/Xpa$^{null/null}$, Xpd$^{R722W/R722W}$/Xpa$^{null/null}$* und *Xpd$^{G602D/R22W}$/Xpa$^{null/null}$.*

**Revendications**

1. Procédé de sélection d'un composé, ou d'un mélange de composés, ledit procédé comprenant les étapes consistant à :

   - fournir une souris génétiquement modifiée, ladite souris présentant au moins une mutation sur un gène codant pour une protéine de la voie de Réparation par Excision de Nucléotides (REN), ladite mutation entraînant un phénotype de vieillissement prématuré comparé à une souris ne présentant pas ladite mutation ;
   - exposer ladite souris génétiquement modifiée, ou une partie provenant de celle-ci, à un composé ou à un mélange de composés ;
   - déterminer l'effet du composé, ou du mélange de composés, sur le phénotype de vieillissement prématuré de ladite souris génétiquement modifiée ; et
   - sélectionner le composé, ou le mélange de composés, qui empêche, inhibe, retarde ou limite l'apparition dudit phénotype de vieillissement prématuré de ladite souris génétiquement modifiée,

dans lequel ladite mutation porte sur un gène sélectionné dans le groupe composé *de : Xpa, Xpb, Xpc, Xpd, Xpe, Xpf, Xpg, Xpv, Csa, Csb, Ttda, HR23A, HR23B* et *Ercc1,* et dans lequel ledit phénotype de vieillissement prématuré consiste en une réduction de la durée de vie, une forte ostéoporose et/ou une forte dégénérescence rétinienne comparé à une souris ne présentant pas ladite mutation.

2. Procédé selon la revendication 1, dans lequel ladite protéine participe également à la voie de Réparation Couplée à la Transcription (TCR, *Transcription-Coupled Repair*).

3. Procédé selon la revendication 1, dans lequel ladite mutation est équivalente à ou imite un allèle des gènes *Csa, Csb, Xpb, Xpd, Xpg, Xpf, Ttda* ou *Erccl* responsable chez l'homme d'un Syndrome de Cockayne (CS), d'une épithéliomatose pigmentaire (Xeroderma pigmentosum, XP), d'une association Xeroderma pigmentosum-Syndrome de Cockayne (XPCS), d'une trichothiodystrophie (TTD), d'un syndrome cérébro-oculo-facio-squelettique (COFS) ou d'un syndrome XPF-ERCC1.

4. Procédé selon la revendication 3, dans lequel la mutation responsable chez l'homme du syndrome de Cockayne, du syndrome COFS ou de XPCS est sélectionnée dans le groupe constitué par les mutations associées au CS sur : le gène humain *Csa : Csa^{null/nul},* Y322ter; le gène humain *Csb : Csb^{nul},* Q184ter, R453ter, W517ter, R670W, R735ter, G744ter, W851R, Q854ter, R947ter, P1042L, P1095R, R1213G ; le gène humain *Xpd :* G602D, G675R, 669fs708ter ; le gène humain *Xpb :* F99S, FS740 ; et pour le gène humain *Xpg :* R263ter, 659ter.

5. Procédé selon l'une quelconque des revendications 1 à 4,
dans lequel ladite souris génétiquement modifiée présente une mutation sur au moins deux gènes différents codant chacun pour une protéine de la voie REN.

6. Procédé selon la revendication 5, dans lequel ladite souris génétiquement modifiée présente une combinaison de mutations choisies dans le groupe comprenant : *Csa^{null/nul}/Xpa^{null/nul}, Gsa^{null/nul}/Xpc^{null/nul}, Csb^{G744ter/G744ter}/Xpa^{null/nul}, Csb^{G744ter/G744ter}/Xpc^{null/nul}, Xpd^{G602D/G602D}/Xpa^{null/nul}, Xpd^{R722RW/R722W}/Xpa^{null/nul}* et *Xpd^{G602D/R722W}/Xpa^{null/nul},*

# Fig 1a

# Fig 1b

# Fig 2

# Fig 3

*Fig 4* A

*Fig 4* B

| Gene name | Gene ID | Fold change in *Ercc1* | Fold change in aging |
|---|---|---|---|
| cyt P450, 4a14 | AA060595 | 7.5 | 2.9 |
| fat specific gene 27 | AA466094 | 4.4 | 2.1 |
| IGFBP -1 | M83086 | 3.7 | 1.9 |
| cyt P450, 4a10 | AA109684 | 3.2 | 1.7 |
| SAM synthetase | M29782 | 2.1 | 1.5 |
| tubulin a4 | W11746 | 1.9 | n.d. |
| cyt P450, 4a14A | A061737 | 1.8 | 1.7 |
| angiogenin | AA237829 | 1.7 | 1.5 |
| transcription termination factor 1 | AA049906 | 1.6 | -1.5 |
| ATPase, Ca transporter | W34420 | 1.6 | n.d. |
| Murine Glvr-1 | AA177949 | 1.6 | n.d. |
| CBFA2T3 | AA051563 | 1.6 | 1.6 |
| mannosidase 2, a1 | W09023 | 1.5 | n.d. |
| CEA-related cell adhesion molecule 1 | AA245546 | 1.4 | -1.4 |
| IGFBP-10 | AA423149 | 1.4 | n.d. |
| fatty acid amide hydrolase | AA260227 | -14.2 | -2.4 |
| esterase 31 | AA254921 | -4.8 | -1.3 |
| IGF-1 | W10072 | -2.4 | n.d. |

Fig 5

Fig 6

# Fig 7

Transcriptional profiling of 15 days old CSB-/-XPA-/- mouse livers

| ProbeSet_ID | Gene | | csb-/-xpa-/- |
|---|---|---|---|
| **Insulin growth factors** | | | |
| 1437481_at | insulin-like growth factor 1 | Igf1 | |
| 1452014_a_at | insulin-like growth factor 1 | Igf1 | |
| 1419519_at | insulin-like growth factor 1 | Igf1 | |
| 1423062_at | insulin-like growth factor binding protein 3 | Igfbp3 | |
| 1421992_a_at | insulin-like growth factor binding protein 4 | Igfbp4 | |
| 1421991_a_at | insulin-like growth factor binding protein 4 | Igfbp4 | |
| 1422826_at | insulin-like growth factor binding protein, acid subunit | | |
| **Growth hormone receptor** | | | |
| 1417762_s_at | growth hormone receptor | Ghr | |
| 1451501_a_at | growth hormone receptor | Ghr | |
| 1451871_a_at | growth hormone receptor | Ghr | |
| **Prolactin receptor** | | | |
| 1421382_at | prolactin receptor | Prlr | |
| 1450226_at | prolactin receptor | Prlr | |
| 1425333_a_at | prolactin receptor | Prlr | |
| 1448556_at | prolactin receptor | Prlr | |
| **Thyroid hormone** | | | |
| 1418936_at | type II 5-deiodothyronine deiodinase | Dio2 | |
| 1422857_at | thyroid hormone receptor interactor 4 | Trip4 | |
| 1417391_at | deiodinase, iodothyronine type I | Dio1 | |
| 1422973_a_at | thyroid hormone responsive SPOT14 | Thrsp | |
| **Transferrin receptor** | | | |
| 1423857_a_at | transferrin receptor | Tfrc | |
| AFFX-TransRecMur/X57349_3_at | transferrin receptor | Tfrc | |
| 1422986_a_at | transferrin receptor | Tfrc | |
| **Antioxidant defense** | | | |
| 1451124_at | superoxide dismutase 1 | Sod1 | |
| 1421040_a_at | glutathione-S-transferase, alpha 2 | Gsta2 | |
| 1421041_a_at | glutathione-S-transferase, alpha 2 | Gsta2 | |
| 1417883_at | glutathione S-transferase, theta 2 | Gstt2 | |
| 1418349_at | glutathione-S-transferase, mu 5 | Gstm5 | |
| 1448576_a_at | glutathione S-transferase, pi 2 | Gstp2 | |
| 1421517_at | glutathione reductase 1 | Gsr | |
| 1426030_x_at | Glyoxalase I | Glo1 | |
| 1422439_at | epoxide hydrolase 1 | Ephx1 | |
| 1448239_at | heme oxygenase 1 | Hmox1 | |

| ProbeSet_ID | Gene | | csb-/-xpa-/- |
|---|---|---|---|
| **Oxidation (Cytochrome P450)** | | | |
| 1418743_at | cytochrome P450, 27a1 | Cyp27a1 | |
| 1418921_at | cytochrome P450, 2b12 | Cyp2b12 | |
| 1422267_s_at | cytochrome P450, 2b10 | Cyp2b10 | |
| 1448470_at | cytochrome P450, 2b13 | Cyp2b13 | |
| 1425045_s_at | cytochrome P450, 2b23 | Cyp2b23 | |
| 1418980_at | cytochrome P450, 2b9 | Cyp2b9 | |
| 1417651_at | cytochrome P450, 2c29 | Cyp2c29 | |
| 1424273_at | cytochrome P450, 2c70 | Cyp2c70 | |
| 1446032_at | cytochrome P450, 2c70 | Cyp2c70 | |
| 1448752_a_at | cytochrome P450, 2f2 | Cyp2f2 | |
| 1417531_at | cytochrome P450, 2j5 | Cyp2j5 | |
| 1417532_at | cytochrome P450, 2j5 | Cyp2j5 | |
| 1419059_at | cytochrome P450, 4f14 | Cyp4f14 | |
| **NADPH-oxid. metabolism** | | | |
| 1417423_at | flavin containing monooxygenase 1 | Fmo1 | |
| 1453435_a_at | flavin containing monooxygenase 2 | Fmo2 | |
| 1422904_at | flavin containing monooxygenase 2 | Fmo2 | |
| 1432459_at | flavin containing monooxygenase 2 | Fmo2 | |
| 1422855_s_at | flavin containing monooxygenase 2 | Fmo2 | |
| 1440525_at | flavin containing monooxygenase 3 | Fmo3 | |
| 1418998_at | | Kmo | |

*Fig 8*

Real-time PCR verification of selected gene targets in CSB-/-XPA-/-, CSB-/- and XPA-/- mouse livers.

## Fig 9a

## Fig 9b

**Fig 10** A

**Fig 10** B

**Fig 10** C

# Fig 11

**A**

Conditional targeting construct(c)

Wt allele (+)

Knock-out targeting construct (-)

**B** C/- C/- -/- XPA

16 kb
12 kb

**C**

XPAc/-
WT
XPA-/-

**D** c/+ +/+ c/+ cᴮ/+ cᴮ/+

10 kb

2.4 kb

**E** ES cell clone
1 2 3 4

WT 16 kb
XPAc 12 kb

EP 1 815 245 B1

# Fig 12

# Fig 13

# Fig 14

Fig 15

Fig 16

Fig 17

# *Fig 18*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Boer J et al.** *Science,* 17 May 2002, vol. 296 (5571), 1276-9
- **Waard H et al.** *Mol Cell Biol.,* September 2004, vol. 24 (18), 7941-8
- **Hasty P ; Campisi J ; Hoeijmakers J ; Steeg H ; Vijg J.** *Science,* 28 February 2003, vol. 299 (5611), 1355-9
- **Hasty P ; Vijg J.** *Ageing Cell,* 2004, vol. 3, 55-65
- **Hasty P. ; Vijg J.** *Ageing Cell,* 2004, vol. 3, 67-69
- **Miller RA.** *Ageing Cell,* 2004, vol. 3, 47-51
- **Miller R.A.** *Ageing Cell,* 2004, vol. 3, 52-53
- **Miller RA.** *Science,* October 2005, vol. 310 (5747), 441-3
- **Ausubel et al.** Current protocols in molecular biology. Wiley Interscience, 2004
- **Hoeijmakers.** Genome maintenance mechanisms for preventing cancer. *Nature,* 17 May 2001, vol. 411 (6835), 366-74
- **Nouspikel T ; Hanawalt PC.** DNA repair in terminally differentiated cells. *DNA Repair,* 22 January 2002, vol. 1 (1), 59-75
- **Campisi, J.** *Trends Cell Biol,* 2001, vol. 11, 27-31
- **Bernstein, C., H. et al.** *Mutat. Res.,* 2002, vol. 511, 145-78
- **Hasty et al.** *Science,* 2003, vol. 299, 1355-9
- **Mitchell, J. R. ; J. H. Hoeijmakers ; L. J. Niedernhofer.** *Curr Opin Cell Biol,* 2003, vol. 15, 232-40
- **J.H. Hoeijmakers.** *Nature,* 2001
- **Niedernhofer et al.** *EMBO Journal,* 2001
- **Tian et al.** *Mol Cell Biol.,* March 2004, vol. 24 (6), 2237-42
- **Giglia-Mari et al.** *Nature Genetics,* 2004
- **Boer et al.** *Cancer Res.,* 1998
- **Horst et al.** *Cell,* 1997
- **Boer et al.** *Cancer Research,* 1999
- **Boer et al.** *Science,* 2002
- **Hoeijmakers.** *Nature,* 2001
- **Hasty et al.** *Science,* 2003
- **Hasty ; Vijg.** *Aging Cell,* 2004
- **Ausubel et al.** Current Protocols in Molecular Biology. Wiley Interscience, 2004
- **Cleaver et al.** *Human Mutation,* 1999, vol. 14, 9-22
- **Itin et al.** *J Am Acad Dermatol,* 2001, vol. 44, 891-920
- **Giglia-Mari G et al.** *Nat Genet.,* July 2004, vol. 36 (7), 714-9
- **Riis, B. ; L. Risom ; S. Loft ; H. E. Poulsen.** *DNA Repair,* 2002, vol. 1, 419-24
- **Vijg J. et al.** *Mech Ageing Dev.,* December 1997, vol. 98 (3), 189-202
- **D. H. Ly ; D. J. Lockhart ; R A. Lerner ; P. G. Schultz.** *Science,* 31 March 2000, vol. 287, 2486
- **P. Hasty ; J. Campisi ; J. Hoeijmakers ; H. van Steeg ; J. Vijg.** *Science,* 28 February 2003, vol. 299, 1355
- **D. Bootsma ; K. Kraemer ; J. Cleaver ; J. H. J. Hoeijmakers.** The Genetic Basis of Human Cancer. McGraw-Hill, 1998, 245-274
- **K. H. Kraemer.** *Proc Natl Acad Sci U S A,* 1997, vol. 94, 11
- **A. M. Sijbers et al.** *Cell,* 1996, vol. 86, 811
- **M. van Duin et al.** *Cell,* 1986, vol. 44, 913
- **J. E. Cleaver ; L. H. Thompson ; A. S. Richardson ; J. C. States.** *Hum Mutat,* 1999, vol. 14, 9
- **M. van Duin et al.** *Mutat Res,* 1989, vol. 217, 83
- **D. Bootsma ; K. H. Kraemer ; J. E. Cleaver ; J. H. J. Hoeijmakers et al.** The Metabolic and Molecular Basis of Inherited Disease. McGraw-Hill, 2001, vol. 1, 677-703
- **A. Westerveld et al.** *Nature,* 1984, vol. 310, 425
- **C. A. Hoy ; L. H. Thompson ; C. L. Mopney ; E. P. Salazar.** *Cancer Res,* 1985, vol. 45, 1737
- **G. M. Adair et al.** *Embo J,* 2000, vol. 19, 5552
- **R G. Sargent et al.** *Nucleic Acids Res,* 2000, vol. 28, 3771
- **L. J. Niedernhofer et al.** *Embo J,* 2001, vol. 20, 6540
- **L. J. Niedernhofer et al.** *Mol Cell Biol,* 2004, vol. 24
- **Y. Matsumura ; C. Nishigori ; T. Yagi ; S. Imamura ; H. Takebe.** *Hum Mol Genet,* 1998, vol. 7, 969
- **A. M. Sijbers et al.** *J Invest Dermatol,* 1998, vol. 110, 832
- **J. Sgouros ; P. H. Gaillard ; R D. Wood.** *Trends Biochem Sci,* March 1999, vol. 24, 95

- **B. Kobe ; A. V. Kajava.** *Curr Opin Struct Biol,* 2001, vol. 11, 725
- **M. Biggerstaff ; D. E. Szymkowski ; R. D. Wood.** *Embo J,* 1993, vol. 12, 3685
- **J. McWhir ; J. Selfridge ; D. J. Harrison ; S. Squires ; D. W. Melton.** *Nat Genet,* 1993, vol. 5, 217
- **G. Weeda et al.** *Curr Biol,* 1997, vol. 7, 427
- **M. Tian ; R. Shinkura ; N. Shinkura ; F. W. Alt.** *Mol Cell Biol,* February 2004, vol. 24, 1200
- **A. de Vries et al.** *Nature,* 1995, vol. 377, 169
- **J. Selfridge ; K. T. Hsia ; N. J. Redhead ; D. W. Melton.** *Nucleic Acids Res.,* 2001, vol. 29, 4541
- **J. Prasher et al.** *Embo J,* 2004
- **W. B. Cutler ; E. Friedmann ; E. Genovese-Stone.** *Am J Phys Med Rehabil,* 1993, vol. 72, 219
- **R. Roubenoff.** *J Nutr,* 1999, vol. 129, 256S
- **Y. C. Liou et al.** *Nature,* 31 July 2003, vol. 424, 556
- **S. Gupta.** *Semin Cancer Biol,* 2000, vol. 10, 161
- **C. S. Carter ; M. M. Ramsey ; W. E. Sonntag.** *Trends Genet,* 2002, vol. 18, 295
- **A. A. Toogood.** *Horm Res,* 2003, vol. 60, 105
- **M. J. Bartosiewicz ; D. Jenkins ; S. Penn ; J. Emery ; A. Buckpitt.** *J Pharmacol Exp Ther,* 2001, vol. 297, 895
- **J. C. Corton ; S. P. Anderson ; A. Stauber.** *Annu Rev Pharmacol Toxicol,* 2000, vol. 40, 491
- **T. Sakuma ; R. Honma ; S. Maguchi ; H. Tamaki ; N. Nemoto.** *Xenobiotica,* 2001, vol. 31, 223
- **C. Epstein.** *Proc. Natl. Acad Sciences,* 1967, vol. 57, 327
- **S. H. Sigal et al.** *Am J Physiol,* May 1999, vol. 276, G1260
- **Q. Huang et al.** *Toxicol Sci,* 2001, vol. 63, 196
- **L. J. Niedernhofer ; J. S. Daniels ; C. A. Rouzer ; R E. Greene ; L. J. Marnett.** *J Biol Chem,* 2003, vol. 29, 29
- **K. A. Olson ; S. J. Verselis ; J. W. Fett.** *Biochem Biophys Res Commun,* 1998, vol. 242, 480
- **H. A. Chapman ; Jr. ; J. S. Munger ; G. P. Shi.** *Am J Respir Crit Care Med,* 1994, vol. 150, 155
- **K. W. Moremen ; P. W. Robbins.** *J Cell Biol,* 1991, vol. 115, 1521
- **B. Gil et al.** *Hepatology,* 1996, vol. 24, 876
- **U. Danesch ; W. Hoeck ; G. M. Ringold.** *J Biol Chem,* 1992, vol. 267, 7185
- **R S. Cotran ; V. Kumar ; S. L. Robbins.** Pathologic basis of disease. 1989
- **B. D. Chang et al.** *Proc Natl Acad Sci USA,* 2002, vol. 99 (3), 89
- **M. Kochetkova et al.** *Cancer Res,* 2002, vol. 62, 4599
- **R M. Popovici et al.** *J Clin Endocrinol Metab,* 2001, vol. 86, 2653
- **Y. Shen ; E. White.** *Adv Cancer Res,* 2001, vol. 82, 55
- **C. K. Lee ; R. G. Klopp ; R. Weindruch ; T. A. Prolla.** *Science,* 1999, vol. 285, 1390
- **S. X. Cao ; J. M. Dhabhi ; P. L. Mote ; S. R Spindler.** *Proc Natl Acad Sci U S A,* 2001, vol. 98, 10630
- **P. Hasty ; J. Vijg.** *Science,* 2002, vol. 296, 1250
- **J. N. Crawley.** What's wrong with my mouse. Wiley-Liss, 2000, 47-63
- **M. M. Ouellette ; L. D. McDaniel ; W. E. Wright ; J. W. Shay ; R A. Schultz.** *Hum Mol Genet,* 12 February 2000, vol. 9, 403
- **M. M. Ouellette ; D. L. Aisner ; I. Savre-Train ; W. E. Wright ; J. W. Shay.** *Biochem Biophys Res Commun,* 27 January 1999, vol. 254, 795
- **J. de Boer et al.** *Cancer Res,* 1999, vol. 59, 3489
- **S. Parrinello et al.** *Nat Cell Biol,* August 2003, vol. 5, 741
- **W. Keijzer et al.** *Mutat Res,* 1979, vol. 62, 183
- **A. J. van Vuuren et al.** *Mutat Res,* 1995, vol. 337, 25
- **R J. Carter et al.** *J Neurosci,* 1999, vol. 19, 3248
- **W. Schul et al.** *Embo J,* 2002, vol. 21, 4719
- **J. H. Hoeijmakers.** *Nature,* 2001, vol. 411, 366
- **de Boer J ; Hoeijmakers JH.** *Carcinogenesis,* 2000, vol. 21, 453-60
- **G. J. Lithgow ; M. S. Gill.** *Nature,* 2003, vol. 421, 125
- **Machiko Murai ; Yasushi Enokido ; Naoko Inamura ; Masafumi Yoshino ; Yoshimichi Nakatsu ; Gijsbertus T. J. ; Horst, Jan H. J. ; Hoeijmakers, Kiyoji Tanaka ; Hiroshi Hatanaka.** *PNAS,* 2001, vol. 98, 13379-13384
- **Tatar M ; Bartke A ; Antebi A.** *Science,* 2003, vol. 299, 1346-51
- **Muller.** Ames Dwarf Mice. *Science's SAGE KE 2001, tg11,* 03 October 2001, //sageke.science-mag.org/cgi/content/full/sageke;2001/1/tg11
- **H. M. Brown-Borg ; K. E. Borg ; C. J. Meliska ; A. Bartke.** *Nature,* 1996, vol. 384, 33
- **K. Flurkey ; J. Papaconstantinou ; R. A. Miller ; D. E. Harrison.** *Proc. Natl. Acad. Sci. U.S.A.,* 2001, vol. 98, 6736
- **K. T. Coschigano ; D. Clemmons ; L. L. Bellush ; J. J. Kopchick.** *Endocrinology,* 2000, vol. 141, 2608
- **F. P. Dominici ; S. J. Hauck ; D. P. Argentino ; A. Bartke ; D. Turyn.** *J. Endocrinol.,* 2002, vol. 173, 81
- **M. Holzenberger et al.** *Nature,* 2003, vol. 421, 182
- **Strasburger CJ ; Bidlingmaier M ; Wu Z, Morrison KM.** *Horm Res,* 2001, vol. 55 (2), 100-5

- **Longo VD ; Finch CE.** *Science,* 2003, vol. 299, 1342-6
- **T. Finkel.** *IUBMB Life,* 2001, vol. 52, 3
- **Sakai, K. ; J. Miyazaki.** A transgenic mouse line that retains Cre recombinase activity in mature oocytes irrespective of the cre transgene transmission. *Biochem Biophys Res Commun,* 1997, vol. 237 (2), 318-24
- **Layher, S. K. ; J. E. Cleaver.** Quantification of XPA gene expression levels in human and mouse cell lines by competitive RT-PCR. *Mutat Res,* 1997, vol. 383 (1), 9-19
- **Dragatsis, I. ; S. Zeitlin.** CaMKIIalpha-Cre transgene expression and recombination patterns in the mouse brain. *Genesis,* 2000, vol. 26 (2), 133-5
- **Riggs, B.L. ; S. Khosla ; L.J. Melton.** 3rd, Sex steroids and the construction and conservation of the adult skeleton. *Endocr Rev,* 2002, vol. 23 (3), 279-302
- **Seeman, E.** Pathogenesis of bone fragility in women and men. *Lancet,* 2002, vol. 359 (9320), 1841-50
- **Kawaguchi, H. et al.** Independent impairment of osteoblast and osteoclast differentiation in klotho mouse exhibiting low-turnover osteopenia. *J Clin Invest,* 1999, vol. 104 (3), 229-37
- **Seeman, E.** During aging, men lose less bone than women because they gain more periosteal bone, not because they resorb less endosteal bone. *Calcif Tissue Int,* 2001, vol. 69 (4), 205-8
- Nucleotide excision repair syndromes: xeroderma pigmentosum, Cockayne syndrome and trichothiodystrophy. **Bootsma, D. et al.** The genetic basis of human cancer. McGraw-Hill, 1998, 245-74
- **Itin, P.H. ; A. Sarasin ; M.R. Pittelkow.** Trichothiodystrophy: update on the sulfur-deficient brittle hair syndromes. *J Am Acad Dermatol,* 2001, vol. 44 (6), 891-920
- **Botta, E. et al.** Analysis of mutations in the XPD gene in Italian patients with trichothiodystrophy: site of mutation correlates with repair deficiency, but gene dosage appears to determine clinical severity. *Am J Hum Genet,* 1998, vol. 63 (4), 1036-48
- **Wakeling, E.L. et al.** Central osteosclerosis with trichothiodystrophy. *Pediatr Radiol,* 2004, vol. 34 (7), 541-6
- **Toelle, S.P. ; E. Valsangiacomo ; E. Boltshauser.** Trichothiodystrophy with severe cardiac and neurological involvement in two sisters. *Eur J Pediatr,* 2001, vol. 160 (12), 728-31
- **Kousseff, B.G. ; N.B. Esterly.** Trichothiodystrophy, IBIDS syndrome or Tay syndrome. *Birth Defects Orig Artic Ser,* 1998, vol. 24 (2), 169-81
- **Przedborski, S. et al.** Trichothiodystrophy, mental retardation, short stature, ataxia, and gonadal dysfunction in three Moroccan siblings. *Am J Med Genet,* 1990, vol. 35 (4), 566-73
- **Civitelli, R. et al.** Central osteosclerosis with ectodermal dysplasia: clinical, laboratory, radiologic, and histopathologic characterization with review of the literature. *J Bone Miner Res,* 1989, vol. 4, 863-75
- **Chapman, S.** The trichothiodystrophy syndrome of Pollitt. *Pediatr Radiol,* 1988, vol. 18 (2), 154-6
- **Price, V.H. et al.** Trichothiodystrophy: sulfur-deficient brittle hair as a marker for a neuroectodermal symptom complex. *Arch Dermatol,* 1980, vol. 116 (12), 1375-84
- **McCuaig, C. et al.** Trichothiodystrophy associated with photosensitivity, gonadal failure, and striking osteosclerosis. *J Am Acad Dermatol,* 1993, vol. 28 (5), 820-6
- **Boer, J. et al.** A mouse model for the basal transcription/DNA repair syndrome trichothiodystrophy. *Mol Cell,* 1998, vol. 1 (7), 981-90
- **Boer, J. et al.** Premature aging in mice deficient in DNA repair and transcription. *Science,* 2002, vol. 296 (5571), 1276-9
- **Castro, C. H. ; J. P. Stains et al.** Development of mice with osteoblast-specific connexin43 gene deletion. *Cell Commun Adhes,* 2003, vol. 10 (4-6), 445-50
- **Chiu, W. S. ; J. F. McManus et al.** Transgenic mice that express Cre recombinase in osteoclasts. *Genesis,* 2004, vol. 39 (3), 178-85
- **Ovchinnikov, D. A. ; J. M. Deng et al.** Co12a1-directed expression of Cre recombinase in differentiating chondrocytes in transgenic mice. *Genesis,* 2000, vol. 26 (2), 145-6
- **Barlow, C. ; M. Schroeder et al.** Targeted expression of Cre recombinase to adipose tissue of transgenic mice directs adipose-specific excision of loxP-flanked gene segments. *Nucleic Acids Res,* 1997, vol. 25 (12), 2543-5
- **R A. Miller.** *Science,* 21 October 2005, vol. 310, 44
- **S. Gupta.** *Semin Cancer Biol,* June 2000, vol. 10, 161
- **Q. Huang et al.** *Toxicol Sci,* 2000, vol. 63, 196